# EUROPÄISCHE PATENTSCHRIFT

(11) **EP 3 131 992 B1**
(45) Veröffentlichungstag und Bekanntmachung des Hinweises auf die Patenterteilung: **17.06.2020**
(21) Anmeldenummer: 15715766.0
(22) Anmeldetag: 16.04.2015
(51) Int. Cl.: C09D 183/10, C07C 279/00, C08G 77/16, C08G 77/42, C09J 183/10

(54) **SCHNELL HÄRTENDE MIGRATIONSFREIE ZUSAMMENSETZUNG AUF BASIS VON SILANGRUPPEN-HALTIGEN POLYMEREN**
FAST CURING MIGRATION-FREE COMPOSITION BASED ON POLYMERS CONTAINING SILANE GROUPS
COMPOSITION SANS MIGRATION À DURCISSEMENT RAPIDE À BASE DE POLYMÈRES CONTENANT DES GROUPES SILANES

(30) Priorität: 16.04.2014 EP 14164923
(43) Veröffentlichungstag der Anmeldung: 22.02.2017
(73) Patentinhaber: Sika Technology AG, 6340 Baar (CH)
(72) Erfinder: BURCKHARDT, Urs, CH-8049 Zürich (CH); CANNAS, Rita, CH-8600 Dübendorf (CH)
(74) Vertreter: Sika Patent Attorneys
(86) Internationale Anmeldenummer: PCT/EP2015/058326
(87) Internationale Veröffentlichungsnummer: WO 2015/158860

(56) Entgegenhaltungen:
- EP-A1- 1 985 666
- EP-A1- 2 100 923
- EP-A1- 2 388 297
- FR-A1- 2 925 496

## Beschreibung

### Technisches Gebiet

Die Erfindung betrifft bei Raumtemperatur härtbare Zusammensetzungen auf Basis von Silangruppen-haltigen Polymeren, welche insbesondere geeignet sind als Klebstoff, Dichtstoff oder Beschichtung.

### Stand der Technik

Bei Raumtemperatur härtbare Zusammensetzungen auf Basis von Silangruppen-haltigen Polymeren sind bekannt. Silangruppen-haltige Polymere sind dabei insbesondere Polyorganosiloxane, welche gemeinhin als "Silicone" bzw. "Siliconkautschuke" bezeichnet werden, und Silangruppen-haltige organische Polymere, welche auch als "silanfunktionelle Polymere", "silanmodifizierte Polymere" (SMP) oder "silanterminierte Polymere" (STP) bezeichnet werden. Solche Zusammensetzungen spielen eine bedeutende Rolle in vielen technischen Anwendungen, beispielsweise als Klebstoffe, Dichtstoffe oder Beschichtungen. Ihre Aushärtung erfolgt über Vernetzungsreaktionen der Silangruppen, welche unter dem Einfluss von Feuchtigkeit hydrolysieren, als Silanolgruppen untereinander kondensieren und dabei Siloxanbindungen bilden. Zur Beschleunigung der Aushärtung werden häufig Katalysatoren eingesetzt. Sehr oft handelt es sich dabei um toxikologisch bedenkliche Stoffe, welche eine potentielle Gefährdung von Verarbeiter und Umwelt darstellen, insbesondere auch nach der Aushärtung, wenn die Katalysatoren oder ihre Abbauprodukte durch Ausgasen, Migration oder Auswaschen freigesetzt werden.
Als Vernetzungskatalysatoren werden klassischerweise Organozinn-Verbindungen, insbesondere Dialkylzinn(IV)-carboxylate, eingesetzt. Diese zeichnen sich durch eine gute Aktivität in Bezug auf die Silanol-Kondensation aus und sind sehr hydrolysebeständig; allerdings sind sie gesundheitsschädlich und stark wassergefährdend. Oft werden sie in Kombination mit weiteren Katalysatoren verwendet, hauptsächlich basischen Verbindungen, insbesondere Aminen, welche vor allem die vorgeschaltete Hydrolyse der Silangruppen beschleunigen.

Aufgrund einer stärkeren Gewichtung von EHS-Aspekten durch Berufsverbände und Verbraucher sowie schärferer staatlicher Regulierung werden seit einiger Zeit vermehrt Anstrengungen unternommen, die Organozinn-Verbindungen durch andere, weniger toxische Katalysatoren auszutauschen. So wurden etwa Organotitanate, -zirkonate und -aluminate als alternative Metall-Katalysatoren beschrieben. Diese haben aber zumeist eine geringere katalytische Aktivität in Bezug auf die Silanol-Kondensation als Organozinn-Verbindungen und bewirken eine deutlich langsamere Vernetzung. Wegen ihrer mangelnden Hydrolysestabilität können sie zudem beim Lagern der Zusammensetzung durch den Restfeuchtegehalt der Inhaltsstoffe einen Grossteil ihrer Aktivität verlieren, wodurch sich die Aushärtung zusätzlich stark verlangsamt oder ganz zum Erliegen kommt.
Eine weitere bekannte Alternative zu Organozinn-Verbindungen stellen stark basische Stickstoff-Verbindungen aus der Klasse der Amidine und Guanidine dar, welche in Kombination mit den erwähnten Metall-Katalysatoren oder auch allein eingesetzt werden können. Viele der gebräuchlichen Amidin- und Guanidin-Katalysatoren, wie insbesondere 1,8-Diazabicyclo[5.4.0]undec-7-en (DBU) und 1,1,3,3-Tetramethylguanidin (TMG), sind allerdings leichtflüchtige und geruchsintensive sowie ebenfalls gesundheitsschädliche und umweltgefährdende Verbindungen. Ausserdem neigen sie dazu, aufgrund geringer Verträglichkeit in der Zusammensetzung zu migrieren und dadurch Separation, Ausschwitzen oder Substratverschmutzung zu verursachen. Die beschriebene Verwendung von aromatischen, bei Raumtemperatur festen Amidinen und Guanidinen schafft hier Abhilfe, erfordert aber den Einsatz von geeigneten Lösemitteln und bringt Einbussen bei der katalytischen Aktivität und damit der Vernetzungsgeschwindigkeit.

Das Dokument FR 2 925 496 betrifft die Verwendung von Guanidinderivaten als Katalysatoren für die Aushärtung von Polysiloxanen, die Silangruppen aufweisen.

### Darstellung der Erfindung

Aufgabe der vorliegenden Erfindung ist es daher, eine bei Raumtemperatur härtbare Zusammensetzung auf Basis von Silangruppen-haltigen Polymeren bereitzustellen, welche eine niedrige Gefahreneinstufung erlaubt, emissions- und geruchsarm ist, eine gute Lagerfähigkeit ohne Separationsneigung aufweist, mittels Feuchtigkeit unter Vernetzung der Silangruppen rasch aushärtet und dabei ein mechanisch hochwertiges und beständiges Material bildet, welches kaum zu migrationsbedingten Fehlern wie Ausschwitzen und Substratverschmutzung neigt.
Diese Aufgabe wird durch eine Zusammensetzung nach Anspruch 1 enthaltend einen Katalysator der Formel (I) gelöst. Diese Eigenschaften werden auch ohne die Verwendung von Metall-haltigen Verbindungen als Co-Katalysatoren erreicht, insbesondere ohne oder mit einer reduzierten Menge an Organozinn-Verbindungen. Der Katalysator der Formel (I) enthält aliphatische Amidin- oder Guanidin-Gruppen und zeigt eine hohe katalytische Aktivität, während aromatische Amidine oder Guanidine kaum oder gar nicht katalytisch aktiv sind. Überraschenderweise weisen erfindungsgemässe Zusammensetzungen eine sehr schnelle, auf die Gegenwart des Katalysators der Formel (I) zurückgehende Aushärtung auf, auch wenn der Katalysator der Formel (I) relativ gross und nicht sehr mobil ist. Weiterhin überraschend ist der Umstand, dass erfindungsgemässe Zusammensetzungen weder vor noch nach der Aushärtung zu migrationsbedingten Fehlern wie Separation, Ausschwitzen und Substratverschmutzung neigen, im Gegensatz zu vielen ähnlichen Zusammensetzungen mit aliphatischen Amidin- und Guanidin-Katalysatoren nach dem Stand der Technik, wo Katalysator-bedingte Migrationseffekte eine starke Rolle spielen. Dies ist überraschend, da der Katalysator der Formel (I) frei von Silangruppen ist und somit bei der Aushärtung nicht ans Polymer angebunden wird.
Weitere Aspekte der Erfindung sind Gegenstand weiterer unabhängiger Ansprüche. Besonders bevorzugte Ausführungsformen der Erfindung sind Gegenstand der abhängigen Ansprüche.

### Wege zur Ausführung der Erfindung

Die vorliegende Erfindung betrifft eine Zusammensetzung umfassend
- mindestens ein Silangruppen-haltiges Polymer; und
- mindestens einen Katalysator der Formel (I),

   A⁅Z]ₙ (I)

   wobei
   n für eine ganze Zahl von 2 bis 20 steht,
   A für einen n-wertigen aliphatischen oder cycloaliphatischen oder arylaliphatischen Kohlenwasserstoff-Rest mit 2 bis 100 C-Atomen, welcher gegebenenfalls ungesättigte Anteile enthält, gegebenenfalls Heteroatome enthält und gegebenenfalls Aminogruppen aufweist, steht, und
   Z für einen Rest der Formel 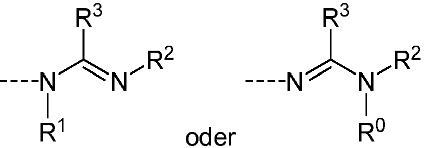 steht,
      wobei
      R¹ und R⁰ unabhängig voneinander jeweils für Wasserstoff oder für einen Alkyl- oder Cycloalkyl- oder Aralkyl-Rest mit 1 bis 8 C-Atomen stehen,
      R² für Wasserstoff oder für einen Alkyl-, Cycloalkyl- oder Aralkyl-Rest mit 1 bis 18 C-Atomen, welcher gegebenenfalls Heteroatome enthält und welcher gegebenenfalls endständige primäre oder sekundäre Aminogruppen aufweist, steht,
      R³ für -NR⁴R⁵ oder für Wasserstoff oder für einen Alkyl-, Cycloalkyl- oder Aralkyl-Rest mit 1 bis 12 C-Atomen steht, R⁴ und R⁵ unabhängig voneinander jeweils für Wasserstoff oder für einen Alkyl-, Cycloalkyl- oder Aralkyl-Rest mit 1 bis 18 C-Atomen, welcher gegebenenfalls Heteroatome enthält, stehen,
         wobei
      R¹ und R² auch zusammen für einen Alkylen-Rest mit 2 bis 6 C-Atomen stehen können,
      R² und R⁰ auch zusammen für einen Alkylen-Rest mit 3 bis 6 C-Atomen, welcher gegebenenfalls Heteroatome enthält, stehen können,
      R² und R³ auch zusammen für einen Alkylen-Rest mit 3 bis 6 C-Atomen stehen können,
      R⁴ und R⁵ auch zusammen für einen Alkylen-Rest mit 4 bis 7 C-Atomen, welcher gegebenenfalls Heteroatome enthält, stehen können, und
      R² und R⁵ auch zusammen für einen Alkylen-Rest mit 2 bis 12 C-Atomen stehen können;
      wobei der Katalysator der Formel (I) kein Stickstoffatom enthält, welches direkt an einen aromatischen Ring gebunden oder Teil eines heteroaromatischen Ringsystems, wie zum Beispiel Imidazol oder Pyrimidin, ist, und wobei er frei von Silangruppen ist.

Im vorliegenden Dokument bezeichnet der Begriff "Silangruppe" eine an einen organischen Rest oder an einen Polyorganosiloxan-Rest gebundene Silylgruppe mit einer bis drei, insbesondere zwei oder drei, hydrolysierbaren Substituenten am Silicium-Atom. Die hydrolysierbaren Substituenten stellen dabei insbesondere Alkoxy-, Acetoxy-, Ketoximato-, Amido- oder Enoxy-Reste mit 1 bis 13 Kohlenstoffatomen dar, bevorzugt Alkoxy- oder Ketoximato-Reste, besonders bevorzugt Alkoxy-Reste; letztere Silangruppen werden im folgenden als "Alkoxysilangruppen" bezeichnet. Unter dem Begriff "Silangruppen" werden weiterhin Silanolgruppen verstanden, welche formal partiell oder vollständig hydrolysierten Silangruppen entsprechen und eine oder mehrere Hydroxyl-Reste am Siliciumatom aufweisen.
Als "Hydroxysilan", "Isocyanatosilan", "Aminosilan" bzw. "Mercaptosilan" werden Organoalkoxysilane bezeichnet, die am organischen Rest zusätzlich zur Silangruppe eine oder mehrere Hydroxyl-, Isocyanato-, Amino- bzw. Mercaptogruppen aufweisen.
Mit "Poly" beginnende Substanznamen wie Polyol oder Polyisocyanat bezeichnen Substanzen, die formal zwei oder mehr der in ihrem Namen vorkommenden funktionellen Gruppen pro Molekül enthalten.
Der Begriff "organisches Polymer" umfasst ein Kollektiv von chemisch einheitlichen, sich aber in Bezug auf Polymerisationsgrad, Molmasse und Kettenlänge unterscheidenden Makromolekülen, das durch eine Polyreaktion (Polymerisation, Polyaddition, Polykondensation) hergestellt wurde und im PolymerRückgrat mehrheitlich Kohlenstoffatome aufweist, sowie Umsetzungsprodukte eines solchen Kollektivs von Makromolekülen. Polymere mit einem Polydiorganosiloxan-Rückgrat (gemeinhin als "Silicone" bezeichnet) stellen keine organischen Polymere im Sinne des vorliegenden Dokuments dar.
Der Begriff "Silangruppen-haltiger Polyether" umfasst auch Silangruppen-haltige organische Polymere, welche zusätzlich zu Polyether-Einheiten auch Urethangruppen, Harnstoffgruppen oder Thiourethangruppen enthalten können.

Solche Silangruppen-haltige Polyether können auch als "Silangruppen-haltige Polyurethane" bezeichnet werden.
Unter "Molekulargewicht" versteht man im vorliegenden Dokument die molare Masse (in Gramm pro Mol) eines Moleküls oder eines Teils eines Moleküls, auch als "Rest" bezeichnet. Als "mittleres Molekulargewicht" wird das Zahlenmittel Mₙ einer oligomeren oder polymeren Mischung von Molekülen oder Resten bezeichnet, welches üblicherweise mittels Gelpermeationschromatographie (GPC) gegen Polystyrol als Standard bestimmt wird.
Als "lagerstabil" oder "lagerfähig" wird eine Substanz oder eine Zusammensetzung bezeichnet, wenn sie bei Raumtemperatur in einem geeigneten Gebinde während längerer Zeit, typischerweise mindestens 3 Monaten bis zu 6 Monaten und mehr, aufbewahrt werden kann, ohne dass sie sich in ihren Anwendungs- oder Gebrauchseigenschaften, insbesondere der Viskosität und der Vernetzungsgeschwindigkeit, durch die Lagerung in einem für ihren Gebrauch relevanten Ausmass verändert.
Eine gestrichelte Linie in den Formeln in diesem Dokument stellt jeweils die Bindung zwischen einem Substituenten und dem zugehörigen Molekülrest dar. Als "Raumtemperatur" wird eine Temperatur von ca. 23 °C bezeichnet.

Der Katalysator der Formel (I) kann auch in tautomerer Form vorliegen. Alle möglichen Tautomerformen des Katalysators der Formel (I) werden im Rahmen der vorliegenden Erfindung als gleichwertig angesehen.
Weiterhin kann der Katalysator der Formel (I) in protonierter Form vorliegen. Ebenfalls kann der Katalysator der Formel (I) in komplexierter Form vorliegen, insbesondere mit Kationen von Zink, Eisen oder Molybdän.

Im Katalysator der Formel (I) steht A bevorzugt für einen n-wertigen aliphatischen oder cycloaliphatischen oder arylaliphatischen Kohlenwasserstoff-Rest mit 2 bis 50, insbesondere 2 bis 20, C-Atomen, welcher gegebenenfalls Ether-Sauerstoff oder Amin-Stickstoff enthält, insbesondere einen bis drei EtherSauerstoffe oder einen bis fünf Amin-Stickstoffe.

Besonders bevorzugt ist A ausgewählt aus der Gruppe bestehend aus 1,2-Ethylen; 1,3-Propylen; 1,2-Propylen; 1,4-Butylen; 1,3-Butylen; 1,2-Butylen; 2,3-Butylen; 1,3-Pentylen; 2-Methyl-1,5-pentylen; 1,6-Hexylen; 2,2(4),4-Trimethyl-1,6-hexamethylen; 1,8-Octylen; 1,10-Decylen; 1,12-Dodecylen; (1,5,5-Trimethylcyclohexan-1-yl)methan-1,3; 1,3-Cyclohexylen-bis(methylen); 1,4-Cyclohexylen-bis(methylen) ; 1,3-Phenylen-bis(methylen); 2- und/oder 4-Methyl-1,3-cyclohexylen; 4-Aza-N-methyl-1,7-heptylen; 4-Aza-N-ethyl-1,7-heptylen; 3-Aza-1,5-pentylen; 3,6-Diaza-1,8-octylen; 3,6,9-Triaza-1,11-undecylen; 4-Aza-1,7-heptylen; 3-Aza-1,6-hexylen; 4,7-Diaza-1,10-decylen; 7-Aza-1,13-tridecylen; Piperazin-1,4-diyl-bis(1,2-ethylen); Piperazin-1,4-diyl-bis(1,3-propylen); 3-Oxa-1,5-pentylen; 3,6-Dioxa-1,8-octylen; 4,7-Dioxa-1,10-decylen und α,ω-Polyoxypropylen mit einem Molekulargewicht im Bereich von etwa 180 bis 500 g/mol.

Davon besonders bevorzugt sind Reste A mit 2 bis 18 C-Atomen. Insbesondere bevorzugt sind Reste A mit 2 bis 12 C-Atomen, welche gegebenenfalls 1 bis 3 Ethersauerstoffe und gegebenenfalls 1 bis 3 sekundäre oder tertiäre Aminogruppen enthalten.

Davon weiterhin besonders bevorzugt sind diejenigen Reste A, welche bei Raumtemperatur flüssige Katalysatoren der Formel (I) ermöglichen. Bei Raumtemperatur flüssige Katalysatoren lassen sich besonders einfach und ohne Lösemittel verarbeiten.

n steht bevorzugt für eine ganze Zahl von 2 bis 10. Besonders bevorzugt steht n für 2 oder 3, insbesondere für 2.
R¹ und R⁰ stehen bevorzugt unabhängig voneinander jeweils für Wasserstoff oder für einen Alkyl-Rest mit 1 bis 4 C-Atomen, insbesondere für Wasserstoff. R² steht bevorzugt für Wasserstoff oder für einen Alkyl-, Cycloalkyl- oder Aralkyl-Rest mit 1 bis 12 C-Atomen, insbesondere 1 bis 8 C-Atomen, welcher gegebenenfalls Heteroatome enthält.
R³ steht bevorzugt für -NR⁴R⁵ oder für Wasserstoff oder für einen Alkyl-, Cycloalkyl- oder Aralkyl-Rest mit 1 bis 8 C-Atomen.
Besonders bevorzugt steht R³ für -NR⁴R⁵.

R⁴ und R⁵ stehen bevorzugt unabhängig voneinander jeweils für Wasserstoff oder für einen Alkyl-, Cycloalkyl- oder Aralkyl-Rest mit 1 bis 12 C-Atomen, welcher gegebenenfalls ein Sauerstoffatom oder ein Stickstoffatom enthält. Weiterhin bevorzugt stehen R⁴ und R⁵ zusammen für einen Alkylen-Rest mit 4 bis 7 C-Atomen, welcher gegebenenfalls ein Sauerstoffatom oder ein Stickstoffatom enthält.
Weiterhin bevorzugt stehen R¹ und R² zusammen für einen Alkylen-Rest mit 2 bis 4 C-Atomen, insbesondere 2 oder 3 C-Atomen.
Weiterhin bevorzugt stehen R² und R⁰ zusammen für einen Alkylen-Rest mit 4 bis 6 C-Atomen, welcher gegebenenfalls Heteroatome enthält.
Weiterhin bevorzugt stehen R² und R³ zusammen für einen Alkylen-Rest mit 3 bis 5 C-Atomen.
Weiterhin bevorzugt stehen R² und R⁵ zusammen für einen Alkylen-Rest mit 2 bis 4 C-Atomen.
Besonders bevorzugt steht R⁴ für Wasserstoff.

Das Gewichtsverhältnis zwischen dem Silangruppen-haltigen Polymer und dem Katalysator der Formel (I) beträgt bevorzugt mindestens 10/1, insbesondere mindestens 20/1, besonders bevorzugt mindestens 40/1.
Das Gewichtsverhältnis zwischen dem Silangruppen-haltigen Polymer und dem Katalysator der Formel (I) beträgt bevorzugt höchstens 10'000/1, insbesondere höchstens 2'000/1, besonders bevorzugt höchstens 1'000/1. Bevorzugt liegt das Gewichtsverhältnis zwischen dem Silangruppen-haltigen organischen Polymer und dem Katalysator der Formel (I) im Bereich von 10/1 bis 2'000/1, besonders bevorzugt 20/1 bis 2'000/1, insbesondere 40/1 bis 1'000/1.
Eine solche Zusammensetzung weist eine gute Lagerfähigkeit und eine schnelle Aushärtung auf.

In einer Ausführungsform der Erfindung steht im Katalysator der Formel (I) R³ für Wasserstoff oder für einen Alkyl-, Cycloalkyl- oder Aralkyl-Rest mit 1 bis 12, bevorzugt 1 bis 8, insbesondere 1 bis 4, C-Atomen, oder R² und R³ stehen zusammen für einen Alkylen-Rest mit 3 bis 6, insbesondere 3 bis 5, C-Atomen. Diese Katalysatoren der Formel (I) weisen Amidingruppen auf.
Bevorzugt steht R³ für Wasserstoff oder für Methyl, insbesondere für Methyl. Bevorzugt steht R¹ für Wasserstoff oder zusammen mit R² für 1,2-Ethylen oder 1,3-Propylen.
Bevorzugt steht R² für Hexyl, Cyclohexyl, Benzyl, 2-Ethylhexyl, Octyl, Decyl, Dodecyl oder 2-Methoxyethyl, oder zusammen mit R¹ für 1,2-Ethylen oder 1,3-Propylen.
Die bevorzugten Ausführungsformen der Amidingruppen aufweisenden Katalysatoren der Formel (I) zeichnen sich durch eine gute katalytische Aktivität und eine ausgezeichnete Verträglichkeit in der Zusammensetzung aus. Gegenüber Guanidingruppen aufweisenden Katalysatoren der Formel (I) weisen sie den Vorteil auf, dass sie eine nicht ganz so hohe katalytische Aktivität besitzten und somit in etwas höherer Menge eingesetzt werden können, wodurch sie weniger anfällig sind für Störungen durch andere Bestandteile der Zusammensetzung, insbesondere den darin enthaltenen Verunreinigungen.

Besonders bevorzugte Amidingruppen aufweisende Katalysatoren der Formel (I) sind ausgewählt aus der Gruppe bestehend aus 1,2-Bis(2-methyl-5,6-dihydropyrimidin-1 (4H)-yl)ethan,1 ,2-Bis(2-methyl-4,5-dihydro-1 H-imidazol-1-yl)-ethan, Bis(2-(2-methyl-4,5-dihydro-1H-imidazol-1-yl)ethyl)amin, N,N"-(2,2(4),4-Trimethylhexan-1,6-diyl)bis(N'-hexylacetimidamid), N,N"-(2,2(4),4-Trimethylhexan-1,6-diyl)bis(N'-(2-methoxyethyl)acetimidamid), N,N"-(2-Methylpentan-1,5-diyl)bis(N'-hexylacetimidamid), N,N"-(Propan-1,3-diyl)bis(N'-hexylacetimidamid), N,N"-(3,6-Dioxaoctan-1,8-diyl)bis(N'-hexylacetimidamid), N,N"-(3,6-Dioxaoctan-1,8-diyl)bis(N'-(2-methoxyethyl)acetimidamid) und N,N"-((Methylazandiyl)bis(propan-3,1-diyl))bis(N'-hexylacetimidamid).
Davon bevorzugt sind Katalysatoren mit cyclischen Amidingruppen, welche einen sechsgliedrigen Ring darstellen. Diese Katalysatoren weisen eine besonders hohe katalytische Aktivität auf.

In einer bevorzugten Ausführungsform der Erfindung steht im Katalysator der Formel (I) R³ für -NR⁴R⁵.

Diese Katalysatoren der Formel (I) weisen Guanidingruppen auf. Sie zeichnen sich durch eine im Vergleich zu den Amidingruppen aufweisenden Katalysatoren noch höheren katalytischen Aktivität und eine ausgezeichnete Verträglichkeit in der Zusammensetzung aus.

In einem Guanidingruppen aufweisenden Katalysator der Formel (I) steht
R⁴ bevorzugt für Wasserstoff;
R² und R⁵ stehen bevorzugt unabhängig voneinander jeweils für einen Alkyl-, Cycloalkyl- oder Aralkyl-Rest mit 1 bis 18 C-Atomen, welcher gegebenenfalls Heteroatome enthält; und
R¹ steht bevorzugt für Wasserstoff.
Diese Katalysatoren sind weitgehend geruchlos und somit besonders vorteilhaft.
Die bevorzugten Ausführungsformen der Guanidingruppen aufweisenden Katalysatoren der Formel (I) weisen die Formel (I a) auf. 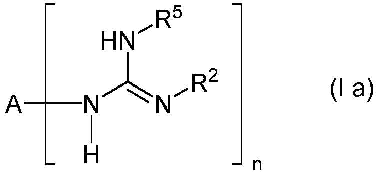 Katalysatoren der Formel (I a) sind besonders gut herstellbar und weisen eine besonders hohe katalytische Aktivität auf.
R² und R⁵ stehen besonders bevorzugt unabhängig voneinander jeweils für Ethyl, Isopropyl, tert.Butyl, 3-(Dimethylamino)propyl oder Cyclohexyl, insbesondere für Isopropyl oder Cyclohexyl, am meisten bevorzugt für Cyclohexyl. Diese Katalysatoren sind besonders gut zugänglich.
A steht bevorzugt für einen n-wertigen aliphatischen oder cycloaliphatischen oder arylaliphatischen Kohlenwasserstoff-Rest mit 2 bis 18 C-Atomen, welcher gegebenenfalls Heteroatome enthält und gegebenenfalls Aminogruppen aufweist.
Besonders bevorzugt steht A für einen n-wertigen aliphatischen oder cycloaliphatischen oder arylaliphatischen Kohlenwasserstoff-Rest mit 2 bis 12 C-Atomen, welcher gegebenenfalls 1 bis 3 Ethersauerstoffe und gegebenenfalls 1 bis 3 sekundäre oder tertiäre Aminogruppen enthält.

Bevorzugt steht n für 2 oder 3, insbesondere für 2.

Besonders bevorzugte Guanidingruppen aufweisende Katalysatoren der Formel (I) sind ausgewählt aus der Gruppe bestehend aus1,1'-(1,2-Ethylen)bis-(2,3-diisopropylguanidin), 1,1'-(1,2-Ethylen)bis(2,3-dicyclohexylguanidin), 1,1'-(1,3-Propylen)bis(2,3-diisopropylguanidin), 1,1'-(1,3-Propylen)bis(2,3-dicyclohexylguanidin), 1,1'-(1,2-Propylen)bis(2,3-diisopropylguanidin), 1,1'-(1,2-Propylen)bis(2,3-dicyclohexylguanidin), 1,1'-(1,4-Butylen)bis(2,3-diisopropyl-guanidin), 1,1'-(1,4-Butylen)bis(2,3-dicyclohexylguanidin), 1,1'-(1,3-Butylen)-bis(2,3-diisopropylguanidin), 1,1'-(1,3-Butylen)bis(2,3-dicyclohexylguanidin), 1,1'-(1,2-Butylen)bis(2,3-diisopropylguanidin), 1,1'-(1,2-Butylen)bis(2,3-dicyclohexylguanidin), 1,1'-(2,3-Butylen)bis(2,3-diisopropylguanidin), 1,1'-(2,3-Butylen)bis(2,3-dicyclohexylguanidin), 1,1'-(1,3-Pentylen)bis(2,3-diisopropylguanidin), 1,1'-(1,3-Pentylen)bis(2,3-dicyclohexylguanidin), 1,1'-(2-Methyl-1,5-pentylen)bis(2,3-diisopropylguanidin), 1,1'-(2-Methyl-1,5-pentylen)bis(2,3-dicyclohexylguanidin), 1,1'-(1,6-Hexylen)bis(2,3-diisopropylguanidin), 1,1'-(1,6-Hexylen)bis(2,3-dicyclohexylguanidin), 1,1'-(2,2(4),4-Trimethyl-1,6-hexamethylen)bis(2,3-diisopropylguanidin), 1,1'-(2,2(4),4-Trimethyl-1,6-hexamethylen)-bis(2,3-dicyclohexylguanidin), 1,1'-(1,8-Octylen)bis(2,3-diisopropylguanidin), 1,1'-(1,8-Octylen)bis(2,3-dicyclohexylguanidin), 1,1'-(1,10-Decylen)bis(2,3-diisopropylguanidin), 1,1'-(1,10-Decylen)bis(2,3-dicyclohexylguanidin), 1,1'-(1,12-Dodecylen)bis(2,3-diisopropylguanidin), 1,1'-(1,12-Dodecylen)bis(2,3-dicyclohexylguanidin), 1-((5-(2,3-Diisopropylguanidino)-1,3,3-trimethylcyclohexyl)methyl)-2,3-diisopropylguanidin, 1-((5-(2,3-Dicyclohexylguanidino)-1,3,3-trimethylcyclohexyl)methyl)-2,3-dicyclohexylguanidin, 1,1'-(1,3-Cyclohexylenbis(methylen))bis(2,3-diisopropylguanidin), 1,1'-(1,3-Cyclohexylen-bis(methylen))bis(2,3-dicyclohexylguanidin), 1,1'-(1,4-Cyclohexylen-bis(methylen))bis-(2,3-diisopropylguanidin), 1,1'-(1,4-Cyclohexylen-bis(methylen))bis(2,3-dicyclohexylguanidin), 1,1'-(1,3-Phenylen-bis(methylen))bis(2,3-diisopropylguanidin), 1,1'-(1,3-Phenylen-bis(methylen))bis(2,3-dicyclohexylguanidin), 1,1'-(2-und/oder 4-Methyl-1,3-cyclohexylen)bis(2,3-diisopropylguanidin), 1,1'-(2-und/oder 4-Methyl-1,3-cyclohexylen)bis(2,3-dicyclohexylguanidin), 1,1'-(4-Aza N-methyl-1,7-heptylen)bis(2,3-diisopropylguanidin), 1,1'-(4-Aza N-methyl-1,7-heptylen)bis(2,3-dicyclohexylguanidin), 1,1'-(4-Aza N-ethyl-1,7-heptylen)bis-(2,3-diisopropylguanidin), 1,1'-(4-Aza N-ethyl-1,7-heptylen)bis(2,3-dicyclohexylguanidin), 1,1'-(3-Aza-1,5-pentylen)bis(2,3-diisopropylguanidin), 1,1'-(3-Aza-1,5-pentylen)bis(2,3-dicyclohexylguanidin), 1,1'-(3,6-Diaza-1,8-octylen)bis(2,3-diisopropylguanidin), 1,1'-(3,6-Diaza-1,8-octylen)bis(2,3-dicyclohexylguanidin), 1,1'-(3,6,9-Triaza-1,11-undecylen)bis(2,3-diisopropylguanidin), 1,1'-(3,6,9-Triaza-1,11-undecylen)bis(2,3-dicyclohexylguanidin), 1,1'-(4-Aza-1,7-heptylen)-bis(2,3-diisopropylguanidin), 1,1'-(4-Aza-1,7-heptylen)bis(2,3-dicyclohexylguanidin), 1,1'-(3-Aza-1,6-hexylen)bis(2,3-diisopropylguanidin), 1,1'-(3-Aza-1,6-hexylen)bis(2,3-dicyclohexylguanidin), 1,1'-(4,7-Diaza-1,10-decylen)bis(2,3-diisopropylguanidin), 1,1'-(4,7-Diaza-1,10-decylen)bis(2,3-dicyclohexylguanidin), 1,1'-(7-Aza-1,13-tridecylen)bis(2,3-diisopropylguanidin), 1,1'-(7-Aza-1,13-tridecylen)bis(2,3-dicyclohexylguanidin), 1,1'-(Piperazin-1,4-diyl-bis(1,2-ethylen))bis(2,3-diisopropylguanidin), 1,1'-(Piperazin-1,4-diyl-bis(1,2-ethylen))bis-(2,3-dicyclohexylguanidin), 1,1'-(Piperazin-1,4-diyl-bis(1,3-propylen))bis(2,3-diisopropylguanidin), 1,1'-(Piperazin-1,4-diyl-bis(1,3-propylen))bis(2,3-dicyclohexylguanidin), 1,1'-(3-Oxa-1,5-pentylen)bis(2,3-diisopropylguanidin), 1,1'-(3-Oxa-1,5-pentylen)bis(2,3-dicyclohexylguanidin), 1,1'-(3,6-Dioxa-1,8-octylen)-bis(2,3-diisopropylguanidin), 1,1'-(3,6-Dioxa-1,8-octylen)bis(2,3-dicyclohexylguanidin), 1,1'-(4,7-Dioxa-1,10-decylen)bis(2,3-diisopropylguanidin), 1,1'-(4,7-Dioxa-1,10-decylen)bis(2,3-dicyclohexylguanidin), 1,1'-(α,ω-Polyoxypropylen)-bis(2,3-diisopropylguanidin) mit einem Molekulargewicht im Bereich von etwa 460 bis 700 g/mol und 1,1'-(α,ω-Polyoxypropylen)bis(2,3-dicyclohexylguanidin) mit einem Molekulargewicht im Bereich von etwa 620 bis 850 g/mol.

Die bevorzugten Ausführungsformen des Katalysators der Formel (I) lassen sich aus handelsüblichen, preisgünstigen Rohstoffen herstellen und haben besonders gute Eigenschaften in Bezug auf katalytische Aktivität und Verträglichkeit des Katalysators.

Der Katalysator der Formel (I) wird insbesondere erhalten durch die Umsetzung von mindestens einem geeigneten Polyamin mit mindestens einem Reagens zur Einführung einer Amidin- oder Guanidin-Gruppe ausgewählt aus der Gruppe bestehend aus Orthoestern, 1,3-Ketoestern, 1,3-Ketoamiden, Nitrilen, Imidsäureestern, Imidsäurechloriden, Amiden, Lactamen, Cyanamid, Carbodiimiden, Harnstoffen, O-Alkylisoharnstoffen, Thioharnstoffen, S-Alkylisothioharnstoffe, Aminoiminomethansulfonsäuren, Guanylpyrazolen und Guanidinen.

Zur Einführung von Amidingruppen sind Orthoester, 1,3-Ketoester, 1,3-Ketoamide, Nitrile, Imidsäureester, Imidsäurechloride, Amide und Lactame geeignet, insbesondere Orthoester, 1,3-Ketoester und Nitrile.
In einem bevorzugten Verfahren zur Herstellung eines Katalysators der Formel (I), bei welchem R³ für Wasserstoff oder für einen Alkyl-, Cycloalkyl- oder Aralkyl-Rest mit 1 bis 12 C-Atomen steht, wird mindestens ein geeignetes Polyamin mit mindestens einem Orthoester oder mindestens einem 1,3-Ketoester oder mindestens einem Nitril umgesetzt. Dabei entsteht ein Amidingruppen aufweisender Katalysator der Formel (I).
Bevorzugte Orthoester sind Orthoformiate, Orthoacetate, Orthopropionate, Orthobutyrate und Orthovalerate, insbesondere Trimethylorthoformiat, Triethylorthoformiat, Trimethylorthoacetat und Triethylorthoacetat.
Bevorzugte 1,3-Ketoester sind Methylacetoacetat, Ethylacetoacetat, Isopropylacetoacetat oder tert.Butylacetoacetat, insbesondere Ethylacetoacetat. Bevorzugte Nitrile sind Acetonitril, Propionitril, Butyronitril, Isobutyronitril, Valeronitril und Capronitril, insbesondere Acetonitril.

Bevorzugt wird ein Amidingruppen aufweisender Katalysator der Formel (I) erhalten durch die Umsetzung von mindestens einem Polyamin mit mindestens einem Orthoester der Formel R³-C(OR^{a})₃ und gegebenenfalls mindestens einem Monoamin der Formel R²-NH-R⁰, insbesondere R²-NH₂, unter Freisetzung von Alkohol R^{a}OH, wobei R^{a} insbesondere für einen Alkylrest mit 1 bis 4 C-Atomen steht. Diese Umsetzung wird bevorzugt bei erhöhter Temperatur, insbesondere bei 40 bis 160 °C und besonders bevorzugt bei 60 bis 140 °C, und erhöhtem Druck in Gegenwart eines Katalysators durchgeführt.
Weiterhin bevorzugt wird ein Amidingruppen aufweisender Katalysator der Formel (I) erhalten durch die Umsetzung von mindestens einem Polyamin mit mindestens einem 1,3-Ketoester der Formel R³-C(O)CH₂C(O)OR^{a}. Diese Umsetzung wird bevorzugt bei einer Temperatur von 20 bis 100 °C, insbesondere 40 bis 80 °C, durchgeführt, wobei der freigesetzte Ester CH₃C(O)OR^{a} bevorzugt destillativ entfernt wird. Bevorzugt wird dabei ein Katalysator eingesetzt, insbesondere eine Säure, bevorzugt eine Sulfonsäure.
Dafür bevorzugte Monoamine sind n-Hexylamin, Cyclohexylamin, Benzylamin, 2-Ethylhexylamin, n-Octylamin, n-Decylamin, Laurylamin und 2-Methoxyethylamin.
Die Umsetzung kann einstufig oder mehrstufig erfolgen.
In einer bevorzugten Ausführungsform dieses Verfahrens wird ein Polyalkylenamin mit zwei primären und mindestens zwei sekundären Aminogruppen, insbesondere TETA, TEPA oder N4-Amin, mit dem Orthoester oder 1,3-Ketoester so umgesetzt, dass mindestens zwei mol Orthoester oder 1,3-Ketoester pro mol Polyalkylenamin-Molekül vorhanden sind. Unter Freisetzung von sechs mol Alkohol R^{a}OH bzw. zwei mol Ester CH₃C(O)OR^{a} entsteht dabei ein Katalysator der Formel (I) mit zwei Amidingruppen, bei welchen R¹ und R² jeweils zusammen für 1,2-Ethylen oder 1,3-Propylen stehen.
In einer weiteren bevorzugten Ausführungsform dieses Verfahrens wird ein Polyamin mit zwei primären Aminogruppen, welches frei ist von sekundären Aminogruppen, mit dem Orthoester oder 1,3-Ketoester so umgesetzt, dass das Polyamin und der Orthoester oder 1,3-Ketoester in einem Molverhältnis im Bereich von 1.2 bis 1.5 vorhanden sind. Dabei entstehen unter Alkohol- oder Esterfreisetzung Gemische, welche neben einem Katalysator der Formel (I) noch oligomere Nebenprodukte mit mehreren Amidingruppen enthalten.
In einer weiteren bevorzgten Ausführungsform dieses Verfahrens werden mindestens ein Polyamin mit zwei primären Aminogruppen, mindestens ein Orthoester oder 1,3-Ketoester und mindestens ein Monoamin der Formel R²-NH-R⁰, insbesondere der Formel R²-NH₂, so umgesetzt, dass pro mol Polyamin mindestens zwei mol Orthoester oder 1,3-Ketoester und mindestens zwei mol Monoamin vorhanden sind. Für den Fall, dass ein Polyamin mit mehr als zwei primäre Aminogruppen verwendet wird, werden bevorzugt soviel Orthoester oder 1,3-Ketoester und soviel Monoamin eingesetzt, dass pro Equivalent primäre Aminogruppe ein mol Orthoester oder 1,3-Ketoester und ein mol Monoamin vorhanden sind. Dabei entstehen Katalysatoren der Formel (I), welche frei sind von primären Aminogruppen. Es ist aber auch möglich, nur ein Teil der primären Aminogruppen mit Orthoester oder 1,3-Ketoester und Monoamin umzusetzen, wobei Katalysatoren der Formel (I) mit primären Aminogruppen entstehen.

Zur Einführung von Guanidingruppen sind Cyanamide, Carbodiimide, Harnstoffe, O-Alkylisoharnstoffe, Thioharnstoffe, S-Alkylisothioharnstoffe, Aminoiminomethansulfonsäuren, Guanylpyrazole und Guanidine geeignet. Bevorzugt sind Cyanamide und Carbodiimide.
Besonders bevorzugt sind Carbodiimide. Auf diesem Weg sind besonders aktive Katalysatoren der Formel (I) auf besonders einfache Weise erhältlich.

In einem weiteren bevorzugten Verfahren zur Herstellung eines Katalysators der Formel (I), bei welchem R³ für -NR⁴R⁵ steht, wird mindestens ein Polyamin der Formel A-(NHR¹)ₙ mit mindestens einem Carbodiimid der Formel R⁵-N=C=N-R² umgesetzt. Dabei entsteht insbesondere ein Guanidingruppen aufweisender Katalysator der Formel (I a). R¹, R², R³, R⁴, R⁵ und n weisen die bereits beschriebenen Bedeutungen auf.

Als Carbodiimid eignen sich typischerweise aliphatische, cycloaliphatische oder arylaliphatische Carbodiimide, insbesondere einfache, im Handel erhältliche aliphatische und cycloaliphatische Carbodiimide, bevorzugt N,N'-Diisopropylcarbodiimid (DIC), N,N'-Di-tert.butylcarbodiimid, N,N'-Dicyclohexylcarbodiimid (DCC) oder N-Ethyl-N'-(3-dimethylaminopropyl)carbodiimid (EDC), besonders bevorzugt N,N'-Diisopropylcarbodiimid (DIC) oder N,N'-Dicyclohexylcarbodiimid (DCC), insbesondere DCC.

Die Umsetzung des Polyamins mit dem Carbodiimid wird bevorzugt bei erhöhter Temperatur, insbesondere bei 40 bis 160 °C und besonders bevorzugt bei 60 bis 140 °C durchgeführt. Die Umsetzung kann ganz ohne die Verwendung von VOC-Lösemitteln erfolgen. Zur Beschleunigung der Umsetzung kann ein Katalysator eingesetzt werden, insbesondere eine Säure wie z.B. eine Carbonsäure oder Kohlensäure, oder eine Lewis-Säure wie z.B. Bortrifluorid-Etherat, Aluminiumchlorid, Aluminiumacetylacetonat, Eisen(III)chlorid, Lithiumperchlorat, Zinkchlorid, Zinkacetat, Zink neodecanoat, Zinkacetylacetonat, Zinktriflat oder Lanthantriflat.
Die Umsetzung kann einstufig oder mehrstufig erfolgen.
Bei der Umsetzung wird bevorzugt höchstens soviel Carbodiimid eingesetzt, dass pro Equivalent Guanidingruppe, die gebildet werden soll, höchstens ein mol Carbodiimid vorhanden ist.
Insbesondere wird das Carbodiimid stöchiometrisch in Bezug auf die primären Aminogruppen des Polyamins eingesetzt. Auf diese Weise ist der erhaltene Katalysator der Formel (I) weitgehend frei von primären Aminogruppen.
Das Reaktionsprodukt aus diesem Verfahren wird bevorzugt ohne Aufarbeitung und/oder Reinigung in der Zusammensetzung verwendet, mit Ausnahme der destillativen Entfernung von flüchtigen Verbindungen, gegebenenfalls unter Vakuum.

Ein für die Herstellung eines Katalysators der Formel (I) geeignetes Polyamin ist ein aliphatisches, cycloaliphatisches oder arylaliphatisches Polyamin ausgewählt aus der folgenden Gruppe:
- aliphatische, cycloaliphatische oder arylaliphatische Polyamine mit zwei primären Aminogruppen und gegebenenfalls sekundären und/oder tertiären Aminogruppen und gegebenenfalls Ethergruppen, insbesondere Ethylendiamin, 1,2- und 1,3-Propandiamin, 2-Methyl-1,2-propandiamin, 2,2-Dimethyl-1,3-propandiamin, die isomeren Butandiamine, 1,3-Pentandiamin (DAMP), 1,5-Pentandiamin, 1,5-Diamino-2-methylpentan (MPMD), 2-Butyl-2-ethyl-1,5-pentandiamin (C11-Neodiamin), 1,6-Hexandiamin, 2,5-Dimethyl-1,6-hexandiamin, 2,2,4- und 2,4,4-Trimethylhexamethylendiamin (TMD), 1,7-Heptandiamin, 1,8-Octandiamin, 1,9-Nonandiamin, 1,10-Decandiamin, 1,11-Undecandiamin, 1,12-Dodecandiamin, 1,2-, 1,3- und 1,4-Diaminocyclohexan, Bis(4-aminocyclohexyl)methan, Bis(4-amino-3-methylcyclohexyl)methan, Bis(4-amino-3-ethylcyclohexyl)methan, Bis(4-amino-3,5-dimethylcyclohexyl)methan, Bis(4-amino-3-ethyl-5-methylcyclohexyl)methan, 1-Amino-3-aminomethyl-3,5,5-trimethylcyclohexan (= Isophorondiamin oder IPD), 2- und 4-Methyl-1,3-diaminocyclohexan und Mischungen davon, 1,3- und 1,4-Bis(aminomethyl)cyclohexan, 2,5(2,6)-Bis(aminomethyl)bicyclo[2.2.1]heptan (NBDA), 3(4),8(9)-Bis(aminomethyl)tricyclo[5.2.1.02,6]decan, 1,4-Diamino-2,2,6-trimethylcyclohexan (TMCDA), 1,8-Menthandiamin, 3,9-Bis(3-aminopropyl)-2,4,8,10-tetraoxaspiro[5.5]undecan, 1,3-Bis(aminomethyl)benzol, 1,4-Bis(aminomethyl)benzol, N,N'-Bis(aminopropyl)piperazin, N,N-Bis(3-aminopropyl)methylamin, N,N-Bis(3-aminopropyl)ethylamin, N,N-Bis(3-aminopropyl)propylamin, N,N-Bis(3-aminopropyl)cyclohexylamin, N,N-Bis(3-aminopropyl)-2-ethyl-hexylamin, Produkte aus der doppelten Cyanoethylierung und nachfolgender Reduktion von Fettaminen, welche abgeleitet sind von natürlichen Fettsäuren, wie N,N-Bis(3-aminopropyl)dodecylamin und N,N-Bis(3-aminopropyl)talgalkylamin, erhältlich als Triameen® Y12D und Triameen® YT (von Akzo Nobel), Diethylentriamin (DETA), Triethylentetramin (TETA), Tetraethylenpentamin (TEPA), Pentaethylenhexamin (PEHA), Polyethylenpolyamin mit 5 bis 7 Ethylenamin-Einheiten (sogenanntes "higher ethylenepolyamine", HEPA), Dipropylentriamin (DPTA), N-(2-Aminoethyl)-1,3-propandiamin (N3-Amin), N,N'-Bis(3-aminopropyl)ethylendiamin (N4-Amin), Bis-hexamethylentriamin (BHMT), 3-(2-Aminoethyl)aminopropylamin, N3-(3-Aminopentyl)-1,3-pentandiamin, N5-(3-Aminopropyl)-2-methyl-1,5-pentandiamin und N5-(3-Amino-1-ethylpropyl)-2-methyl-1,5-pentandiamin, Bis(2-aminoethyl)ether, 3,6-Dioxaoctan-1,8-diamin, 4,7-Dioxadecan-1,10-diamin, 4,7-Dioxadecan-2,9-diamin, 4,9-Dioxadodecan-1,12-diamin, 5,8-Dioxadodecan-3,10-diamin, 4,7,10-Trioxatridecan-1,13-diamin, cycloaliphatische ethergruppenhaltige Diamine aus der Propoxylierung und nachfolgenden Aminierung von 1,4-Dimethylolcyclohexan, erhältlich insbesondere als Jeffamine® RFD-270 (von Huntsman), Polyoxyalkylendiamine mit einem mittleren Molekulargewicht im Bereich von 200 bis 2'000 g/mol, wie sie im Handel beispielsweise unter den Handelsnamen Jeffamine® (von Huntsman), Polyetheramine (von BASF) und PC Amine® (von Nitroil) erhältlich sind, dadurch gekennzeichnet, dass sie 2-Aminopropyl- oder 2-Aminobutyl-Endgruppen tragen, insbesondere Jeffamine® D-230, Jeffamine® D-400, Jeffamine® D-2000, Jeffamine® XTJ-582, Jeffamine® XTJ-578, Jeffamine® HK-511, Jeffamine® ED-600, Jeffamine® ED-900, Jeffamine® ED-2003, Jeffamine® XTJ-569, Jeffamine® XTJ-533, Jeffamine® XTJ-536, Jeffamine® THF-100, Jeffamine® THF-170, Jeffamine® THF-140 und Jeffamine® THF-230 (alle von Huntsman), sowie dazu analoge Typen von BASF und Nitroil, sowie aminopropylierte Polyoxyalkylenamine, wie sie durch Umsetzen von Polyoxyalkylenaminen mit Acrylnitril und nachfolgender Hydrierung erhältlich sind.
- aliphatische, cycloaliphatische oder arylaliphatische Polyamine mit drei primären Aminogruppen und gegebenenfalls sekundären und/oder tertiären Aminogruppen und gegebenenfalls Ethergruppen, insbesondere 4-Aminomethyl-1,8-octandiamin, 1,3,5-Tris-(aminomethyl)-cyclohexan, 1,3,5-Tris-(aminomethyl)-benzol, Tris(2-aminoethyl)amin, Polyoxyalkylentriamine mit einem mittleren Molekulargewicht im Bereich von 200 bis 2'000 g/mol, wie sie im Handel beispielsweise unter den Handelsnamen Jeffamine® (von Huntsman), Polyetheramine (von BASF) und PC Amine® (von Nitroil) erhältlich sind, dadurch gekennzeichnet, dass sie 2-Aminopropyl- oder 2-Aminobutyl-Endgruppen tragen, insbesondere Jeffamine® T-403 und Jeffamine® XTJ-566 (beide von Huntsman), sowie dazu analoge Typen von BASF und Nitroil.
- Polyethylenimine, d.h. Produkte aus der Polymerisation von Ethylenimin mit verzweigter Struktur enthaltend primäre, sekundäre und tertiäre Aminogruppen, wie untenstehend anhand eines einzelnen Moleküls beispielhaft dargestellt. Es handelt sich dabei um Mischungen verschiedener Moleküle mit Molekulargewichtsverteilungen, wie sie typischerweise bei Polymerisationen entstehen. 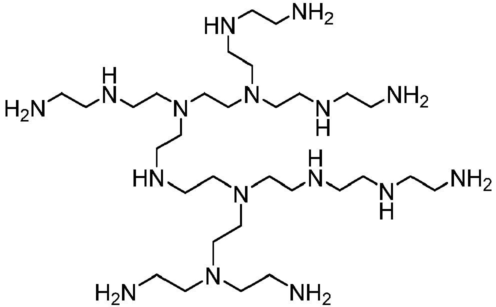 Geeignete Polyethylenimine sind im Handel unter den Markennamen Lupasol® (von BASF) und Epomin® (von Nippon Shokubai) erhältlich. Bevorzugte Typen sind Lupasol® FG und Lupasol® G 20 wasserfrei, Epomin® SP-003, Epomin® SP-006, Epomin® SP-012 und Epomin® SP-018.
- Polyvinylamine, insbesondere die im Handel unter dem Markennamen Lupamin® (von BASF) erhältlichen Typen.
- aliphatische, cycloaliphatische oder arylaliphatische Polyamine mit einer primären, einer sekundären und gegebenenfalls einer tertiären Aminogruppe, insbesondere N-Methyl-1,2-ethandiamin, N-Ethyl-1,2-ethandiamin, N-Butyl-1,2-ethandiamin, N-Hexyl-1,2-ethandiamin, N-(2-Ethylhexyl)-1,2-ethandiamin, N-Cyclohexyl-1,2-ethandiamin, N-Methyl-1,3-propandiamin, N-Ethyl-1,3-propandiamin, N-Butyl-1,3-propandiamin, N-Hexyl-1,3-propandiamin, N-(2-Ethylhexyl)-1,3-propandiamin, N-Dodecyl-1,3-propandiamin, N-Cyclohexyl-1,3-propandiamin, 3-Methylamino-1-pentylamin, 3-Ethylamino-1-pentylamin, 3-Butylamino-1-pentylamin, 3-Hexylamino-1-pentylamin, 3-(2-Ethylhexyl)amino-1-pentylamin, 3-Dodecylamino-1-pentylamin, 3-Cyclohexylamino-1-pentylamin, 4-Aminomethyl-piperidin, 3-(4-Aminobutyl)-piperidin,N-(2-Aminoethyl)piperazin, N-(2-Aminopropyl)piperazin, N¹-((3-Dimethylamino)propyl)-1,3-diaminopropan, sowie 3-aminopropylierte Fettamine wie insbesondere N-Cocoalkyl-1,3-propandiamin, N-Oleyl-1,3-propandiamin, N-Soyaalkyl-1,3-propandiamin, N-Talgalkyl-1,3-propandiamin und N-(C₁₆₋₂₂-Alkyl)-1,3-propandiamin, wie sie beispielsweise unter dem Handelsnamen Duomeen® (von Akzo Nobel) erhältlich sind.
- aliphatische, cycloaliphatische oder arylaliphatische Polyamine mit zwei oder drei sekundären Aminogruppen, insbesondere N,N-Dimethyl-1,2-ethandiamin, N,N-Diethyl-1,2-ethandiamin, N,N-Dimethyl-1,3-propandiamin, N,N-Diethyl-1,3-propandiamin, N¹,N¹-Diethyl-1,4-pentandiamin, sowie sekundäre Polyoxyalkylenamine mit einem mittleren Molekulargewicht im Bereich von 200 bis 2000 g/mol, insbesondere Jeffamine® SD-231, Jeffamine® SD-401, Jeffamine® SD-2001 und Jeffamine® ST-404 (alle von Huntsman).

Davon bevorzugt sind Polyamine mit mindestens zwei primären Aminogruppen.
Davon bevorzugt sind weiterhin Polyamine mit 2 bis 50, insbesondere 2 bis 20, C-Atomen.

Ein besonders bevorzugtes Polyamin ist ausgewählt aus der Gruppe bestehend aus 1,2-Ethandiamin, 1,3-Propandiamin, 1,2-Propandiamin, 1,4-Butandiamin, 1,3-Butandiamin, 1,2-Butandiamin, 2,3-Butandiamin, 1,3-Pentandiamin (DAMP), 1,5-Diamino-2-methylpentan (MPMD), 1,6-Hexandiamin, 2,2,4- und 2,4,4-Trimethylhexamethylendiamin (TMD), 1,8-Octandiamin, 1,10-Decandiamin, 1,12-Dodecandiamin, 1-Amino-3-aminomethyl-3,5,5-trimethylcyclohexan (= Isophorondiamin oder IPD), 1,3-Bis(aminomethyl)cyclohexan, 1,4-Bis(aminomethyl)cyclohexan,1,3-Bis(aminomethyl)benzol, 2- und 4-Methyl-1,3-diaminocyclohexan und Mischungen davon, N,N-Bis(3-aminopropyl)methylamin, N,N-Bis(3-aminopropyl)ethylamin, Diethylentriamin (DETA), Triethylentetramin (TETA), Tetraethylenpentamin (TEPA), Dipropylentriamin (DPTA), N-(2-Aminoethyl)-1,3-propandiamin (N3-Amin), N,N'-Bis(3-aminopropyl)ethylendiamin (N4-Amin), Bis-hexamethylentriamin (BHMT), N,N'-Bis(aminoethyl)piperazin, N,N'-Bis(aminopropyl)piperazin, Bis-(2-aminoethyl)ether, 3,6-Dioxaoctan-1,8-diamin, 4,7-Dioxadecan-1,10-diamin und Polyoxypropylendiamin mit einem Molekulargewicht im Bereich von etwa 210 bis 500 g/mol, insbesondere Jeffamine® D-230 und Jeffamine® D-400.
Davon bevorzugt sind Polyamine mit 2 bis 18 , insbesondere 2 bis 12, C-Atomen.

Ein Verfahren zur Herstellung eines Katalysators der Formel (I) kann insbesondere auch "in situ", d.h. in Gegenwart des Silangruppen-haltigen Polymers, durchgeführt werden. Dazu werden das Polyamin und Reagens zur Einführung einer Amidin- oder Guanidin-Gruppe mit dem Silangruppen-haltigen Polymer vermischt und bei einer Temperatur im Bereich von 40 bis 120 °C umgesetzt. Die in situ-Umsetzung kann insbesondere auch in Gegenwart weiterer Inhaltsstoffe, wie sie für Zusammensetzungen auf Basis Silangruppen-haltiger Polymere typisch sind, durchgeführt werden.
Besonders bevorzugt ist dieses Verfahren für die Herstellung eines Guanidingruppen aufweisenden Katalysators der Formel (I) aus der Umsetzung eines Polyamins mit einem Carbodiimid.
Überraschenderweise kann die Umsetzung zu einem Katalysator der Formel (I) auch in einem voll formulierten Dichtstoff oder Klebstoff oder einer voll formulierten Beschichtung erfolgen, also auch in Gegenwart von beispielsweise Trocknungsmitteln und/oder Weichmachern und/oder Füllstoffen und/oder Rheologie-Modifizierern. Die in-situ Herstellung des Katalysators der Formel (I) in der Zusammensetzung ist besonders vorteilhaft, da der Katalysator somit erst mit einer gewissen zeitlichen Verzögerung entsteht und dadurch den Compoundierprozess wenig stört. Dies kann in der Praxis einen bedeutenden Vorteil darstellen, beispielsweise im Fall von feuchten Bestandteilen wie Füllstoffen, die so in der Zusammensetzung mit Trocknungsmitteln getrocknet werden können und deren Feuchtigkeit somit nicht unter Einbezug des Katalysators mit Silangruppen des Silangruppen-haltigen Polymers reagieren. Dadurch wird die Viskosität der Zusammensetzung nicht unerwünscht erhöht.

Das Silangruppen-haltige Polymer ist in einer Ausführungsform ein Polyorganosiloxan mit endständigen Silangruppen, welches bevorzugt die Formel (III) aufweist, 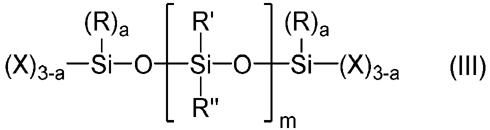 wobei
R, R' und R" unabhängig voneinander jeweils für einen einwertigen Kohlenwasserstoff-Rest mit 1 bis 12 C-Atomen stehen;
X für einen Hydroxyl-Rest oder für einen Alkoxy-, Acetoxy-, Ketoximato-, Amido- oder Enoxy-Rest mit 1 bis 13 C-Atomen steht;
a für 0, 1 oder 2 steht; und
m für eine ganze Zahl im Bereich von 50 bis etwa 2'500 steht.

R steht bevorzugt für Methyl, Vinyl oder Phenyl.
R' und R" stehen bevorzugt unabhängig voneinander jeweils für einen Alkylrest mit 1 bis 5, bevorzugt 1 bis 3 C-Atomen, insbesondere für Methyl.
X steht bevorzugt für einen Hydroxyl-Rest oder für einen Alkoxy- oder Ketoximato-Rest mit 1 bis 6 C-Atomen, insbesondere für einen Hydroxyl-, Methoxy-, Ethoxy-, Methylethylketoximato- oder Methylisobutylketoximato-Rest. Besonders bevorzugt steht X für einen Hydroxylrest.
a steht bevorzugt für 0 oder 1, insbesondere für 0.
Weiterhin ist m bevorzugt so gewählt, dass das Polyorganosiloxan der Formel (III) bei Raumtemperatur eine Viskosität im Bereich von 100 bis 500'000 mPa·s, insbesondere von 1000 bis 100'000 mPa·s, aufweist.

Solche Polyorganosiloxane sind gut handhabbar und vernetzen mit Feuchtigkeit und/oder Silanvernetzern zu festen Silicon-Polymeren mit elastischen Eigenschaften.
Geeignete kommerziell erhältliche Polyorganosiloxane sind beispielsweise erhältlich von Wacker, Momentive Performance Material, GE Advanced Materials, Dow Corning, Bayer oder Shin Etsu.

Bevorzugt enthält die Zusammensetzung zusätzlich zum Polyorganosiloxan mit endständigen Silangruppen einen Silanvernetzer, insbesondere ein Silan der Formel (IV),

(R'")_{q}-Si-(X')_{4-q} (IV)

wobei
R'" für einen einwertigen Kohlenwasserstoff-Rest mit 1 bis 12 C-Atomen steht,
X' für einen Hydroxyl-Rest oder für einen Alkoxy-, Acetoxy-, Ketoximato-, Amido- oder Enoxy-Rest mit 1 bis 13 C-Atomen steht; und
q für einen Wert von 0, 1 oder 2, insbesondere für 0 oder 1, steht.

Besonders geeignete Silane der Formel (IV) sind Methyltrimethoxysilan, Ethyltrimethoxysilan, Propytrimethoxysilan, Vinyltrimethoxysilan, Methyltriethoxysilan, Vinyltriethoxysilan, Phenyltriethoxysilan, Tetramethoxysilan, Tetraethoxysilan, Methyltris(methylethylketoximo)silan, Vinyltris(methylethylketoximo)silan und Methyltris(isobutyl ketoximo)silan.

Das Silangruppen-haltige Polymer ist in einer bevorzugten Ausführungsform der Erfindung ein Silangruppen-haltiges organisches Polymer, insbesondere ein Polyolefin, Polyester, Polyamid, Poly(meth)acrylat oder Polyether oder eine Mischform dieser Polymere, welches jeweils eine oder bevorzugt mehrere Silangruppen trägt. Die Silangruppen können seitlich in der Kette oder endständig stehen und sind über ein C-Atom an das organische Polymer gebunden. Besonders bevorzugt ist das Silangruppen-haltige organische Polymer ein Silangruppen-haltiges Polyolefin oder ein Silangruppen-haltiger Polyester oder ein Silangruppen-haltiges Poly(meth)acrylat oder ein Silangruppen-haltiger Polyether oder eine Mischform dieser Polymere. Am meisten bevorzugt ist ein Silangruppen-haltiger Polyether.

Als Silangruppen am organischen Polymer bevorzugt sind Alkoxysilangruppen, insbesondere Endgruppen der Formel (V), 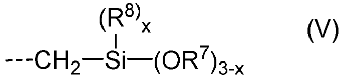 wobei
R⁷ für einen linearen oder verzweigten, einwertigen Kohlenwasserstoffrest mit 1 bis 5 C-Atomen, insbesondere für Methyl oder für Ethyl oder für Isopropyl, steht;
R⁸ für einen linearen oder verzweigten, einwertigen Kohlenwasserstoffrest mit 1 bis 8 C-Atomen, insbesondere für Methyl oder für Ethyl, steht; und
x für einen Wert von 0 oder 1 oder 2, bevorzugt für 0 oder 1, insbesondere für 0, steht.
Besonders bevorzugt steht R⁷ für Methyl oder für Ethyl.

Für bestimmte Anwendungen steht der Rest R⁷ bevorzugt für eine Ethylgruppe, da in diesem Fall bei der Aushärtung der Zusammensetzung ökologisch und toxikologisch harmloses Ethanol freigesetzt wird.
Besonders bevorzugt sind Trimethoxysilangruppen, Dimethoxymethylsilangruppen oder Triethoxysilangruppen.
Dabei weisen Methoxysilangruppen den Vorteil auf, dass sie besonders reaktiv sind, und Ethoxysilangruppen weisen den Vorteil auf, dass sie toxikologisch vorteilhaft und besonders lagerstabil sind.

Das Silangruppen-haltige organische Polymer weist im Mittel bevorzugt 1.3 bis 4, insbesondere 1.5 bis 3, besonders bevorzugt 1.7 bis 2.8 Silangruppen pro Molekül auf. Die Silangruppen sind bevorzugt endständig.
Das Silangruppen-haltige organische Polymer weist bevorzugt ein mittleres Molekulargewicht, bestimmt mittels GPC gegenüber Polystyrol-Standard, im Bereich von 1000 bis 30'000 g/mol, insbesondere von 2000 bis 20'000 g/mol, auf. Das Silangruppen-haltige organische Polymer weist bevorzugt ein Silan-Equivalentgewicht von 300 bis 25'000 g/Eq, insbesondere von 500 bis 15'000 g/Eq, auf.

Das Silangruppen-haltige organische Polymer kann bei Raumtemperatur fest oder flüssig vorliegen. Bevorzugt ist es bei Raumtemperatur flüssig.

Meist bevorzugt handelt es sich beim Silangruppen-haltigen organischen Polymer um einen bei Raumtemperatur flüssigen Silangruppen-haltigen Polyether, wobei die Silangruppen insbesondere Dialkoxysilangruppen und/oder Trialkoxysilangruppen, besonders bevorzugt Trimethoxysilangruppen oder Triethoxysilangruppen, sind.

Verfahren zur Herstellung von Silangruppen-haltigen Polyethern sind dem Fachmann bekannt.
In einem Verfahren sind Silangruppen-haltige Polyether erhältlich aus der Umsetzung von Allylgruppen-haltigen Polyethern mit Hydrosilanen, gegebenenfalls unter Kettenverlängerung mit beispielsweise Diisocyanaten.

In einem weiteren Verfahren sind Silangruppen-haltige Polyether erhältlich aus der Copolymerisation von Alkylenoxiden und Epoxysilanen, gegebenenfalls unter Kettenverlängerung mit beispielsweise Diisocyanaten.
In einem weiteren Verfahren sind Silangruppen-haltige Polyether erhältlich aus der Umsetzung von Polyetherpolyolen mit Isocyanatosilanen, gegebenenfalls unter Kettenverlängerung mit Diisocyanaten.
In einem weiteren Verfahren sind Silangruppen-haltige Polyether erhältlich aus der Umsetzung von Isocyanatgruppen-haltigen Polyethern, insbesondere NCO-terminierten Urethan-Polyethern aus der Umsetzung von Polyetherpolyolen mit einer überstöchiometischen Menge an Polyisocyanaten, mit Aminosilanen, Hydroxysilanen oder Mercaptosilanen. Silangruppen-haltige Polyether aus diesem Verfahren sind besonders bevorzugt. Dieses Verfahren ermöglicht den Einsatz einer Vielzahl von kommerziell gut verfügbaren, kostengünstigen Ausgangsmaterialien, womit unterschiedliche Polymereigenschaften erhalten werden können, beispielsweise eine hohe Dehnbarkeit, eine hohe Festigkeit, ein niedriges Elastizitätsmodul, ein tiefer Glasübergangspunkt oder eine hohe Witterungsbeständigkeit.

Besonders bevorzugt ist der Silangruppen-haltige Polyether erhältlich aus der Umsetzung von NCO-terminierten Urethan-Polyethern mit Aminosilanen oder Hydroxysilanen. Geeignete NCO-terminierte Urethan-Polyether sind erhältlich aus der Umsetzung von Polyetherpolyolen, insbesondere Polyoxyalkylendiolen oder Polyoxyalkylentriolen, bevorzugt Polyoxypropylendiolen oder Polyoxypropylentriolen, mit einer überstöchiometrischen Menge an Polyisocyanaten, insbesondere Diisocyanaten.
Bevorzugt wird die Umsetzung zwischen dem Polyisocyanat und dem Polyetherpolyol unter Feuchtigkeitsausschluss bei einer Temperatur von 50 °C bis 160 °C durchgeführt, gegebenenfalls in Anwesenheit geeigneter Katalysatoren, wobei das Polyisocyanat so dosiert ist, dass dessen Isocyanatgruppen im Verhältnis zu den Hydroxylgruppen des Polyols im stöchiometrischen Überschuss vorhanden sind. Insbesondere wird der Überschuss an Polyisocyanat so gewählt, dass im resultierenden Urethan-Polyether nach der Umsetzung aller Hydroxylgruppen ein Gehalt an freien Isocyanatgruppen von 0.1 bis 5 Gewichts-%, bevorzugt 0.2 bis 4 Gewichts-%, besonders bevorzugt 0.3 bis 3 Gewichts-%, bezogen auf das gesamte Polymer, verbleibt.
Bevorzugte Diisocyanate sind ausgewählt aus der Gruppe bestehend aus 1,6-Hexamethylendiisocyanat (HDI), 1-Isocyanato-3,3,5-trimethyl-5-isocyanatomethyl-cyclohexan (=Isophorondiisocyanat oder IPDI), 2,4- und 2,6-Toluylendiisocyanat und beliebige Gemische dieser Isomeren (TDI) und 4,4'-, 2,4'- und 2,2'-Diphenylmethandiisocyanat und beliebige Gemische dieser Isomeren (MDI). Besonders bevorzugt sind IPDI oder TDI. Meist bevorzugt ist IPDI. Damit werden Silangruppen-haltige Polyether mit besonders guter Lichtechtheit erhalten.

Speziell geeignet als Polyetherpolyole sind Polyoxyalkylendiole oder Polyoxyalkylentriole mit einem Ungesättigtheitsgrad tiefer als 0.02 mEq/g, insbesondere tiefer als 0.01 mEq/g, und einem mittleren Molekulargewicht im Bereich von 400 bis 25'000 g/mol, insbesondere 1000 bis 20'000 g/mol.
Neben Polyetherpolyolen können anteilig auch andere Polyole eingesetzt werden, insbesondere Polyacrylatpolyole, sowie niedrigmolekulare Diole oder Triole.

Geeignete Aminosilane für die Umsetzung mit einem NCO-terminierten Urethan-Polyether sind primäre und sekundäre Aminosilane. Bevorzugt sind 3-Aminopropyltrimethoxysilan, 3-Aminopropyldimethoxymethylsilan, 4-Aminobutyl-trimethoxysilan, 4-Amino-3-methylbutyl-trimethoxysilan, 4-Amino-3,3-dimethylbutyl-trimethoxysilan, N-Butyl-3-aminopropyltrimethoxysilan, N-Phenyl-3-aminopropyltrimethoxysilan, Addukte aus primären Aminosilanen wie 3-Aminopropyltrimethoxysilan, 3-Aminopropyldimethoxymethylsilan oder N-(2-Aminoethyl)-3-aminopropyltrimethoxysilan und Michael-Akzeptoren wie Acrylnitril, (Meth)acrylsäureestern, (Meth)acrylsäureamiden, Maleinsäure- oder Fumarsäurediestern, Citraconsäurediestern oder Itaconsäurediestern, insbesondere N-(3-Trimethoxysilylpropyl)amino-bernsteinsäuredimethyl- oder -diethylester. Ebenfalls geeignet sind Analoga der genannten Aminosilane mit Ethoxy- oder Isopropoxygruppen anstelle der Methoxygruppen am Silicium. Geeignete Hydroxysilane für die Umsetzung mit einem NCO-terminierten Urethan-Polyether sind insbesondere erhältlich aus der Addition von Aminosilanen an Lactone oder an cyclische Carbonate oder an Lactide.
Dafür geeignete Aminosilane sind insbesondere 3-Aminopropyl-trimethoxysilan, 3-Aminopropyl-triethoxysilan, 4-Aminobutyl-trimethoxysilan, 4-Aminobutyl-triethoxysilan, 4-Amino-3-methylbutyl-trimethoxysilan, 4-Amino-3-methylbutyl-triethoxysilan, 4-Amino-3,3-dimethylbutyl-trimethoxysilan, 4-Amino-3,3-dimethylbutyl-triethoxysilan, 2-Aminoethyl-trimethoxysilan oder 2-Aminoethyltriethoxysilan. Besonders bevorzugt sind 3-Aminopropyl-trimethoxysilan, 3-Aminopropyl-triethoxysilan, 4-Amino-3,3-dimethylbutyl-trimethoxysilan oder 4-Amino-3,3-dimethylbutyl-triethoxysilan.
Als Lactone geeignet sind insbesondere γ-Valerolacton, γ-Octalacton, δ-Decalacton, und ε-Decalacton, insbesondere γ-Valerolacton.
Als cyclische Carbonate eignen sich insbesondere 4,5-Dimethyl-1,3-dioxolan-2-on, 4,4-Dimethyl-1,3-dioxolan-2-on, 4-Ethyl-1,3-dioxolan-2-on, 4-Methyl-1,3-dioxolan-2-on oder 4-(Phenoxymethyl)-1,3-dioxolan-2-on.
Als Lactide eignen sich insbesondere 1,4-Dioxan-2,5-dion (Lactid aus 2-Hydroxyessigsäure, auch "Glycolid" genannt), 3,6-Dimethyl-1,4-dioxan-2,5-dion (Lactid aus Milchsäure, auch "Lactid" genannt) und 3,6-Diphenyl-1,4-dioxan-2,5-dion (Lactid aus Mandelsäure).
Bevorzugte Hydroxysilane, welche auf diese Weise erhalten werden, sind N-(3-Triethoxysilylpropyl)-2-hydroxypropanamid, N-(3-Trimethoxysilylpropyl)-2-hydroxypropanamid, N-(3-Triethoxysilylpropyl)-4-hydroxypentanamid, N-(3-Triethoxysilylpropyl)-4-hydroxyoctanamid, N-(3-Triethoxysilylpropyl)-5-hydroxydecanamid und N-(3-Triethoxysilylpropyl)-2-hydroxypropylcarbamat.

Weiterhin sind geeignete Hydroxysilane auch erhältlich aus der Addition von Aminosilanen an Epoxide oder aus der Addition von Aminen an Epoxysilane. Bevorzugte Hydroxysilane, welche auf diese Weise erhalten werden, sind 2-Morpholino-4(5)-(2-trimethoxysilylethyl)cyclohexan-1-ol, 2-Morpholino-4(5)-(2-triethoxysilylethyl)cyclohexan-1-ol oder 1-Morpholino-3-(3-(triethoxysilyl)propoxy)propan-2-ol.

Als Silangruppen-haltige Polyether sind auch kommerziell erhältliche Produkte geeignet, insbesondere die Folgenden: MS Polymer™ (von Kaneka Corp.; insbesondere die Typen S203H, S303H, S227, S810, MA903 und S943); MS Polymer™ bzw. Silyl™ (von Kaneka Corp.; insbesondere die Typen SAT010, SAT030, SAT200, SAX350, SAX400, SAX725, MAX450, MAX951); Excestar® (von Asahi Glass Co. Ltd.; insbesondere die Typen S2410, S2420, S3430, S3630); SPUR+* (von Momentive Performance Materials; insbesondere die Typen 1010LM, 1015LM, 1050MM); Vorasil™ (von Dow Chemical Co.; insbesondere die Typen 602 und 604); Desmoseal® (von Bayer MaterialScience AG; insbesondere die Typen S XP 2458, S XP 2636, S XP 2749, S XP 2774 und S XP 2821), TEGOPAC® (von Evonik Industries AG; insbesondere die Typen Seal 100, Bond 150, Bond 250), Polymer ST (von Hanse Chemie AG/Evonik Industries AG, insbesondere die Typen 47, 48, 61, 61LV, 77, 80, 81); Geniosil® STP (von Wacker Chemie AG; insbesondere die Typen E10, E15, E30, E35).

Besonders bevorzugte Endgruppen der Formel (V) sind Endgruppen der Formel (VI), 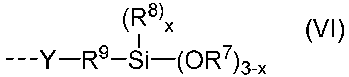 wobei
R⁹ für einen linearen oder verzweigten, zweiwertigen Kohlenwasserstoffrest mit 1 bis 12 C-Atomen, welcher gegebenenfalls cyclische und/oder aromatische Anteile und gegebenenfalls ein oder mehrere Heteroatome, insbesondere ein oder mehrere Stickstoffatome, aufweist, steht;
Y für einen zweiwertigen Rest ausgewählt aus -O-, -S-, -N(R¹⁰)-, -O-CO-N(R¹⁰)-, -N(R¹⁰)-CO-O- und -N(R¹⁰)-CO-N(R¹⁰)- steht,
   wobei R¹⁰ für Wasserstoff oder für einen linearen oder verzweigten Kohlenwasserstoff-Rest mit 1 bis 20 C-Atomen, welcher gegebenenfalls cyclische Anteile aufweist, und welcher gegebenenfalls eine Alkoxysilyl-, Ether- oder Carbonsäureestergruppe aufweist, steht; und
R⁷, R⁸ und x die bereits genannten Bedeutungen aufweisen.
Bevorzugt steht R⁹ für 1,3-Propylen oder für 1,4-Butylen, wobei Butylen mit einer oder zwei Methylgruppen substituiert sein kann.

Besonders bevorzugt steht R⁹ für 1,3-Propylen.

Zusätzlich zum Katalysator der Formel (I) kann die erfindungsgemässe Zusammensetzung weitere Katalysatoren für die Vernetzung der Silangruppen enthalten. Als weitere Katalysatoren geeignet sind insbesondere Metall-Katalysatoren und/oder basische Stickstoff- oder Phosphorverbindungen.

Mögliche Metall-Katalysatoren sind insbesondere Verbindungen von Zinn, Titan, Zirkonium, Aluminium oder Zink, insbesondere Diorganozinn(IV)-Verbindungen wie insbesondere Dibutylzinn(IV)-diacetat, Dibutylzinn(IV)-dilaurat, Dibutylzinn(IV)-dineodecanoat oder Dibutylzinn(IV)-bis(acetylacetonat) und Dioctylzinn(IV)-dilaurat, sowie Titan(IV)- oder Zirkonium(IV)- oder Aluminium(III)- oder Zink(II)-Komplexe mit insbesondere Alkoxy-, Carboxylat-, 1,3-Diketonat-, 1,3-Ketoesterat- oder 1,3-Ketoamidat-Liganden.

Mögliche basische Stickstoff- oder Phosphorverbindungen sind insbesondere Imidazole, Pyridine, Phosphazen-Basen oder bevorzugt Amine, Hexahydrotriazine oder Biguanide sowie Amidine oder Guanidine, welche nicht der Formel (I) entsprechen.

Geeignete Amine sind insbesondere Alkyl-, Cycloalkyl- oder Aralkylamine wie Triethylamin, Triisopropylamin, 1-Butylamin, 2-Butylamin, tert.Butylamin, 3-Methyl-1-butylamin, 3-Methyl-2-butylamin, Dibutylamin, Tributylamin, Hexylamin, Dihexylamin, Cyclohexylamin, Dicyclohexylamin, Dimethylcyclohexylamin, Benzylamin, Dibenzylamin, Dimethylbenzylamin, Octylamin, 2-Ethylhexylamin, Di-(2-ethylhexyl)amin, Laurylamin, N,N-Dimethyl-laurylamin, Stearylamin, N,N-Dimethyl-stearylamin; von natürlichen Fettsäuregemischen abgeleitete Fettamine, wie insbesondere Cocoalkylamin, N,N-Dimethyl-cocoalkylamin, C₁₆₋₂₂-Alkylamin, N,N-Dimethyl-C₁₆₋₂₂-alkylamin, Soyaalkylamin, N,N-Dimethyl-soyaalkylamin, Oleylamin, N,N-Dimethyl-oleylamin, Talgalkylamin oder N,N-Dimethyl-talgalkylamin, erhältlich beispielsweise unter den Handelsnamen Armeen® (von Akzo Nobel) oder Rofamin® (von Ecogreen Oleochemicals); aliphatische, cycloaliphatische oder araliphatische Diamine wie Ethylendiamin, Butandiamin, Hexamethylendiamin, Dodecandiamin, Neopentandiamin, 2-Methyl-pentamethylendiamin (MPMD), 2,2(4),4-Trimethylhexamethylendiamin (TMD), Isophorondiamin (IPD), 2,5(2,6)-Bis-(aminomethyl)-bicyclo[2.2.1]heptan (NBDA), 1,3-Xylylendiamin (MXDA), N,N'-Di(tert.butyl)ethylendiamin, N,N,N',N'-Tetramethyl-ethylendiamin, N,N,N',N'-Tetramethyl-propylendiamin, N,N,N',N'-Tetramethyl-hexamethylendiamin, 3-Dimethylaminopropylamin, 3-(Methylamino)propylamin, 3-(Cyclohexylamino)propylamin, Piperazin, N-Methylpiperazin, N,N'-Dimethylpiperazin, 1,4-Diazabicyclo[2.2.2]octan, Fettpolyamine wie N-Cocoalkyl-1,3-propandiamin, N-Oleyl-1,3-propandiamin, N-Soyaalkyl-1,3-propandiamin, N-Talgalkyl-1,3-propandiamin oder N-(C₁₆₋₂₂-Alkyl)-1,3-propandiamin, erhältlich beispielsweise unter dem Handelsnamen Duomeen® (von Akzo Nobel); Polyalkylenamine wie Diethylentriamin, Dipropylentriamin, Triethylentetramin (TETA), Tetraethylenpentamin (TEPA), Pentamethylenhexamin (PEHA), 3-(2-Aminoethyl)aminopropylamin, N,N'-Bis(3-aminopropyl)ethylendiamin, N-(3-Aminopropyl)-N-methylpropandiamin, Bis(3-dimethylaminopropyl)amin, N-(3-Dimethylaminopropyl)-1,3-propylendiamin, N-(2-Aminoethyl)piperazin (N-AEP), N-(2-Aminopropyl)piperazin, N,N'-Di-(2-aminoethyl)piperazin, 1-Methyl-4-(2-dimethylaminoethyl)piperazin, N,N,N',N",N"-Pentamethyldiethylentriamin, N,N,N',N",N"-Pentamethyldipropylentriamin, Polyethylenimine, erhältlich beispielsweise unter den Handelsnamen Lupasol® (von BASF) und Epomin® (von Nippon Shokubai); Etheramine, wie insbesondere 2-Methoxyethylamin, 2-Ethoxyethylamin, 3-Methoxypropylamin, 3-Ethoxypropylamin, 3-(2-Ethylhexyloxy)propylamin, 3-(2-Methoxyethoxy)propylamin, 2(4)-Methoxyphenylethylamin, Morpholin, N-Methylmorpholin, N-Ethylmorpholin, 2-Aminoethylmorpholin, Bis(2-aminoethyl)ether, Bis(dimethylaminoethyl)-ether, Bis(dimorpholinoethyl)ether, N,N,N'-Trimethyl-N'-hydroxyethylbis(2-aminoethyl)ether, 3,6-Dioxaoctan-1,8-diamin, 4,7-Dioxadecan-1,10-diamin, 4,7-Dioxadecan-2,9-diamin, 4,9-Dioxadodecan-1,12-diamin, 5,8-Dioxadodecan-3,10-diamin, 4,7,10-Trioxatridecan-1,13-diamin oder 2-Aminopropyl-terminierte Glykole, wie sie beispielsweise unter dem Handelsnamen Jeffamin® (von Huntsman) erhältlich sind; Aminoalkohole, wie insbesondere Ethanolamin, Isopropanolamin, Diethanolamin, Diisopopanolamin, Triethanolamin, Triisopropanolamin, N-Butylethanolamin, Diglykolamin, N,N-Diethylethanolamin, N-Methyldiethanolamin, N-Methyldiisopropylamin, N,N,N'-Trimethylaminoethyl-ethanolamin, N-(3-Dimethylaminopropyl)-N,N-diisopropanolamin, N,N-Bis(3-dimethylaminopropyl)-N-isopropanolamin, 2-(2-Dimethylaminoethoxy)ethanolamin oder Addukte aus Mono- und Polyaminen mit Epoxiden oder Diepoxiden; Phenolgruppen-haltige Amine, wie insbesondere Kondensationsprodukte aus Phenolen, Aldehyden und Aminen (sogenannte Mannich-Basen und Phenalkamine) wie insbesondere 2-(Dimethylaminomethyl)phenol, 2,4,6-Tris(dimethylaminomethyl)phenol oder Polymere aus Phenol, Formaldehyd und N,N-Dimethyl-1,3-propandiamin sowie im Handel unter den Markennamen Cardolite® (von Cardolite), Aradur® (von Huntsman) und Beckopox® (von Cytec) erhältliche Phenalkamine; Amidgruppen-haltige Polyamine, sogenannte Polyamidoamine, wie sie im Handel erhältlich sind, beispielsweise unter den Markennamen Versamid® (von Cognis), Aradur® (von Huntsman), Euretek® (von Huntsman) oder Beckopox® (von Cytec); oder Aminosilane, wie insbesondere 3-Aminopropyltrimethoxysilan, 3-Aminopropyldimethoxymethylsilan, N-(2-Aminoethyl)-3-aminopropyltrimethoxysilan, N-(2-Aminoethyl)-3-aminopropyl-methyldimethoxysilan, N-(2-Aminoethyl)-N'-[3-(trimethoxysilyl)propyl]ethylendiamin oder deren Analoga mit Ethoxy- anstelle der Methoxygruppen am Silicium.
Geeignete Triazine sind insbesondere 1,3,5-Hexahydrotriazin oder 1,3,5-Tris(3-(dimethylamino)propyl)-hexahydrotriazin.
Geeignete Biguanide sind insbesondere Biguanid, 1-Butylbiguanid, 1,1-Dimethylbiguanid, 1-Butylbiguanid, 1-Phenylbiguanid oder 1-(o-Tolyl)biguanid (OTBG).
Geeignete Amidine, welche nicht der Formel (I) entsprechen, sind insbesondere 1,8-Diazabicyclo[5.4.0]undec-7-en (DBU), 1,5-Diazabicyclo[4.3.0]non-5-en (DBN), 6-Dibutylamino-1,8-diazabicyclo[5.4.0]undec-7-en, 6-Dibutylamino-1,8-diazabicyclo[5.4.0]undec-7-en, N,N'-Di-n-hexylacetamidin (DHA), 2-Methyl-1,4,5,6-tetrahydropyrimidin, 1,2-Dimethyl-1,4,5,6-tetrahydropyrimidin, 2,5,5-Trimethyl-1,4,5,6-tetrahydropyrimidin, N-(3-Trimethoxysilylpropyl)-4,5-dihydroimidazol oder N-(3-Triethoxysilylpropyl)-4,5-dihydroimidazol.

Geeignete Guanidine, welche nicht der Formel (I) entsprechen, sind insbesondere 1-Butylguanidin, 1,1-Dimethylguanidin, 1,3-Dimethylguanidin, 1,1,3,3-Tetramethylguanidin (TMG), 2-(3-(Trimethoxysilyl)propyl)-1,1,3,3-tetramethylguanidin, 2-(3-(Methyldimethoxysilyl)propyl)-1,1,3,3-tetramethylguanidin, 2-(3-(Triethoxysilyl)propyl)-1,1,3,3-tetramethylguanidin, 1,5,7-Triazabicyclo[4.4.0]-dec-5-en (TBD), 7-Methyl-1,5,7-triazabicyclo[4.4.0]dec-5-en, 7-Cyclohexyl-1,5,7-triazabicyclo[4.4.0]dec-5-en, 1-Phenylguanidin, 1-(o-Tolyl)guanidin (OTG), 1,3-Diphenylguanidin, 1,3-Di(o-tolyl)guanidin oder 2-Guanidinobenzimidazol.

Weiterhin kann die erfindungsgemässe Zusammensetzung als Co-Katalysator eine Säure enthalten, insbesondere eine Carbonsäure. Bevorzugt sind aliphatische Carbonsäuren wie Ameisensäure, Laurinsäure, Stearinsäure, Isostearinsäure, Ölsäure, 2-Ethyl-2,5-dimethylcapronsäure, 2-Ethylhexansäure, Neodecansäure, Fettsäuregemische aus der Verseifung von natürlichen Fetten und Ölen oder Di- und Polycarbonsäuren, insbesondere Poly(meth)acrylsäuren.

Die erfindungsgemässe Zusammensetzung ist in einer bevorzugten Ausführungsform im Wesentlichen frei von Organozinn-Verbindungen. Organozinnfreie Zusammensetzungen sind hinsichtlich Gesundheitsschutz und Umweltschutz vorteilhaft. Insbesondere beträgt der Zinngehalt der Zusammensetzung weniger als 0.1 Gew.-%, insbesondere weniger als 0.05 Gew.-%.

In einer Ausführungsform der Erfindung enthält die Zusammensetzung neben dem Silangruppen-haltigen organischen Polymer und einem Katalysator der Formel (I) zusätzlich mindestens ein Organotitanat. Eine Kombination aus Katalysator der Formel (I) und Organotitanat weist eine besonders hohe katalytische Aktivität auf. Dadurch wird eine schnelle Aushärtung der Zusammensetzung bei verhältnismässig geringer Einsatzmenge des Katalysators der Formel (I) ermöglicht.
Als Organotitanat geeignet sind insbesondere Titan(IV)-Komplexverbindungen. Bevorzugte Organoititanate sind insbesondere ausgewählt aus
- Titan(IV)-Komplexverbindungen mit zwei 1,3-Diketonat-Liganden, insbesondere 2,4-Pentandionat (=Acetylacetonat), und zwei Alkoholat-Liganden;
- Titan(IV)-Komplexverbindungen mit zwei 1,3-Ketoesterat-Liganden, insbesondere Ethylacetoacetat, und zwei Alkoholat-Liganden;
- Titan(IV)-Komplexverbindungen mit einem oder mehreren Aminoalkoholat-Liganden, insbesondere Triethanolamin oder 2-((2-Aminoethyl)amino)ethanol, und einem oder mehreren Alkoholat-Liganden;
- Titan(IV)-Komplexverbindungen mit vier Alkoholat-Liganden;
- sowie höherkondensierte Organotitanate, insbesondere oligomeres Titan(IV)-tetrabutanolat, auch als Polybutyltitanat bezeichnet;
wobei als Alkoholat-Liganden insbesondere Isobutoxy, n-Butoxy, Isopropoxy, Ethoxy und 2-Ethylhexoxy geeignet sind.

Insbesondere geeignet sind die kommerziell erhältlichen Typen Tyzor® AA, GBA, GBO, AA-75, AA-65, AA-105, DC, BEAT, BTP, TE, TnBT, KTM, TOT, TPT oder IBAY (alle von Dorf Ketal); Tytan PBT, TET, X85, TAA, ET, S2, S4 oder S6 (alle von Borica Company Ltd.) und Ken-React® KR® TTS, 7, 9QS, 12, 26S, 33DS, 38S, 39DS, 44, 134S, 138S, 133DS, 158FS oder LICA® 44 (alle von Kenrich Petrochemicals).

Ganz besonders geeignete Organotitanate sind ausgewählt aus Bis(ethylacetoacetato)diisobutoxy-titan(IV) (kommerziell erhältlich beispielsweise als Tyzor® IBAY von Dorf Ketal), Bis(ethylacetoacetato)diisopropoxy-titan(IV) (kommerziell erhältlich beispielsweise als Tyzor® DC von Dorf Ketal), Bis(acetylacetonato)diisopropoxy-titan(IV), Bis(acetylacetonato)diisobutoxy-titan(IV), Tris(oxyethyl)-amin-isopropoxy-titan(IV), Bis[tris(oxyethyl)amin]diisopropoxy-titan(IV), Bis(2-ethylhexan-1,3-dioxy)-titan(IV), Tris[2-((2-Aminoethyl)amino)ethoxy]ethoxy-titan(IV), Bis(neopentyl(diallyl)oxy)-diethoxy-titan(IV), Titan(IV)-tetrabutanolat, Tetra(2-ethylhexyloxy)titanat, Tetra(isopropoxy)titanat und Polybutyltitanat. Am meisten bevorzugt sind Bis(ethylacetoacetato)diisobutoxy-titan(IV) oder Bis(ethylacetoacetato)diisopropoxy-titan(IV).

In einer Organotitanat-haltigen Zusammensetzung liegt das Gewichtsverhältnis zwischen dem Silangruppen-haltigen Polymer und dem Katalysator der Formel (I) bevorzugt im Bereich von 40/1 bis 2000/1.
Das Gewichtsverhältnis zwischen dem Organotitanat und dem Katalysator der Formel (I) liegt bevorzugt im Bereich von 10/1 bis 1/10, besonders bevorzugt 5/1 bis 1/5, insbesondere 5/1 bis 1/3.

Die erfindungsgemässe Zusammensetzung kann weitere Bestandteile enthalten, insbesondere die folgenden Hilfs- und Zusatzmittel:
- Haftvermittler und/oder Vernetzer, insbesondere Aminosilane wie insbesondere 3-Aminopropyl-trimethoxysilan, 3-Aminopropyl-dimethoxymethylsilan, N-(2-Aminoethyl)-3-aminopropyl-trimethoxysilan, N-(2-Aminoethyl)-3-aminopropyl-dimethoxymethylsilan, N-(2-Aminoethyl)-N'-[3-(trimethoxysilyl)propyl]ethylendiamin oder deren Analoga mit Ethoxy- anstelle von Methoxygruppen, weiterhin N-Phenyl-, N-Cyclohexyl- oder N-Alkylaminosilane, Mercaptosilane, Epoxysilane, (Meth)acrylosilane, Anhydridosilane, Carbamatosilane, Alkylsilane oder Iminosilane, oligomere Formen dieser Silane, Addukte aus primären Aminosilanen mit Epoxysilanen oder (Meth)acrylosilanen oder Anhydridosilanen, aminofunktionelle Alkylsilsesquioxane, insbesondere aminofunktionelles Methylsilsesquioxan oder aminofunktionelles Propylsilsesquioxan. Insbesondere geeignet sind 3-Aminopropyltrimethoxysilan, 3-Aminopropyltriethoxysilan, N-(2-Aminoethyl)-3-aminopropyltrimethoxysilan, N-(2-Aminoethyl)-3-aminopropyl-triethoxysilan, 3-Glycidoxypropyltrimethoxysilan, 3-Glycidoxypropyltriethoxysilan oder 3-Ureidopropyltrimethoxysilan, oder oligomere Formen dieser Silane;
- Trocknungsmittel, insbesondere Tetraethoxysilan, Vinyltrimethoxysilan, Vinyltriethoxysilan oder Organoalkoxysilane, welche in α-Stellung zur Silangruppe eine funktionelle Gruppe aufweisen, insbesondere N-(Methyldimethoxysilylmethyl)-O-methyl-carbamat, (Methacryloxymethyl)silane, Methoxymethylsilane, Orthoameisensäureester, Calciumoxid oder Molekularsiebe, insbesondere Vinyltrimethoxysilan oder Vinyltriethoxysilan;
- Weichmacher, insbesondere trialkylsilylterminierte Polydialkylsiloxane wie insbesondere trimethylsilylterminierte Polydimethylsiloxane, insbesondere mit Viskositäten im Bereich von 10 bis 1'000 mPa·s, oder entsprechende Verbindungen, bei denen einige der Methylgruppen ersetzt sind durch andere organische Gruppen, insbesondere Phenyl-, Vinyl- oder Trifluorpropylgruppen, sogenannte Reaktivweichmacher in Form von monofunktionellen, also einseitig reaktiven, Polysiloxanen, Carbonsäureester wie Phthalate, insbesondere Dioctylphthalat, Bis(2-ethylhexyl)phthalat, Bis(3-propylheptyl)phthalat, Diisononylphthalat oder Diisodecylphthalat, Diester von ortho-Cyclohexandicarbonsäure, insbesondere Diisononyl-1,2-cyclohexandicarboxylat, Adipate, insbesondere Dioctyladipat, Bis(2-Ethylhexyl)adipat, Azelate, insbesondere Bis(2-ethylhexyl)acelat, Sebacate, insbesondere Bis(2-ethylhexyl)sebacat oder Diisononylsebacat, Polyole, insbesondere Polyoxyalkylenpolyole oder Polyesterpolyole, Glykolether, Glykolester, organische Phosphor- oder Sulfonsäureester, Sulfonsäureamide, Polybutene oder von natürlichen Fetten oder Ölen abgeleitete Fettsäuremethyl- oder -ethylester, auch "Biodiesel" genannt, wobei Siloxangruppen-haltige Weichmacher besonders geeignet sind für Silangruppen-haltige Polymere in Form von Polyorganosiloxanen;
- Lösemittel;
- anorganische oder organische Füllstoffe, insbesondere natürliche, gemahlene oder gefällte Calciumcarbonate, welche gegebenenfalls mit Fettsäuren, insbesondere Stearinsäure, beschichtet sind, Baryt (Schwerspat), Talke, Quarzmehle, Quarzsand, Dolomite, Wollastonite, Kaoline, calcinierte Kaoline, Mica (Kalium-Aluminium-Silikat), Molekularsiebe, Aluminiumoxide, Aluminiumhydroxide, Magnesiumhydroxid, Kieselsäuren inklusive hochdisperse Kieselsäuren aus Pyrolyseprozessen, industriell hergestellte Russe, Graphit, Metall-Pulver wie Aluminium, Kupfer, Eisen, Silber oder Stahl, PVC-Pulver oder Hohlkugeln;
- Fasern, insbesondere Glasfasern, Kohlefasern, Metallfasern, Keramikfasern oder Kunststofffasern wie Polyamidfasern oder Polyethylenfasern;
- Farbstoffe;
- Pigmente, insbesondere Titandioxid oder Eisenoxide;
- Rheologie-Modifizierer, insbesondere Verdickungsmittel, insbesondere Schichtsilikate wie Bentonite, Derivate von Rizinusöl, hydriertes Rizinusöl, Polyamide, Polyurethane, Harnstoffverbindungen, pyrogene Kieselsäuren, Celluloseether oder hydrophob modifizierte Polyoxyethylene;
- Stabilisatoren gegen Oxidation, Wärme, Licht und UV-Strahlung;
- natürliche Harze, Fette oder Öle wie Kolophonium, Schellack, Leinöl, Rizinusöl oder Sojaöl;
- nicht-reaktive Polymere, wie insbesondere Homo- oder Copolymere von ungesättigten Monomeren, insbesondere aus der Gruppe umfassend Ethylen, Propylen, Butylen, Isobutylen, Isopren, Vinylacetat oder Alkyl(meth)acrylate, insbesondere Polyethylene (PE), Polypropylene (PP), Polyisobutylene, Ethylenvinylacetat-Copolymere (EVA) oder ataktische Poly-α-Olefine (APAO);
- flammhemmende Substanzen, insbesondere die bereits genannten Füllstoffe Aluminiumhydroxid und Magnesiumhydroxid, oder insbesondere organische Phosphorsäureester wie insbesondere Triethylphosphat, Trikresylphosphat, Triphenylphosphat, Diphenylkresylphosphat, Isodecyldiphenylphosphat, Tris(1,3-dichlor-2-propyl)phosphat, Tris(2-chlorethyl)phosphat, Tris(2-ethylhexyl)phosphat, Tris(chlorisopropyl)phosphat, Tris(chlorpropyl)-phosphat, isopropyliertes Triphenylphosphat, Mono-, Bis- oder Tris(isopropylphenyl)phosphate unterschiedlichen Isopropylierungsgrades, Resorcinol-bis(diphenylphosphat), Bisphenol-A-bis(diphenylphosphat) oder Ammoniumpolyphosphate;
- oberflächenaktive Substanzen, insbesondere Netzmittel, Verlaufsmittel, Entlüftungsmittel oder Entschäumer;
- Biozide, insbesondere Algizide, Fungizide oder das Pilzwachstum hemmende Substanzen;
sowie weitere üblicherweise in feuchtigkeitshärtenden Zusammensetzungen eingesetzte Substanzen.Es kann sinnvoll sein, gewisse Bestandteile vor dem Einmischen in die Zusammensetzung chemisch oder physikalisch zu trocknen.

In einer bevorzugten Ausführungsform enthält die Zusammensetzung mindestens ein Trocknungsmittel und mindestens einen Haftvermittler und/oder Vernetzer.
In einer bevorzugten Ausführungsform enthält die Zusammensetzung keine Phthalate als Weichmacher. Solche Zusammensetzungen sind toxikologisch vorteilhaft und weisen weniger Probleme mit Migrationseffekten auf.

Die erfindungsgemässe Zusammensetzung wird vorzugsweise unter Ausschluss von Feuchtigkeit hergestellt und aufbewahrt. Typischerweise ist die Zusammensetzung in einer geeigneten Verpackung oder Anordnung, wie insbesondere einer Flasche, einer Büchse, einem Beutel, einem Eimer, einem Fass oder einer Kartusche, unter Ausschluss von Feuchtigkeit lagerstabil.

Die erfindungsgemässe Zusammensetzung kann in Form einer einkomponentigen oder in Form einer mehrkomponentigen, insbesondere zweikomponentigen, Zusammensetzung vorliegen.
Als "einkomponentig" wird im vorliegenden Dokument eine Zusammensetzung bezeichnet, bei welcher alle Bestandteile der Zusammensetzung vermischt im gleichen Gebinde gelagert werden und welche mit Feuchtigkeit härtbar ist. Als "zweikomponentig" wird im vorliegenden Dokument eine Zusammensetzung bezeichnet, bei welcher die Bestandteile der Zusammensetzung in zwei verschiedenen Komponenten vorliegen, welche in voneinander getrennten Gebinden gelagert werden. Erst kurz vor oder während der Applikation der Zusammensetzung werden die beiden Komponenten miteinander vermischt, worauf die vermischte Zusammensetzung aushärtet, gegebenenfalls unter Einwirkung von Feuchtigkeit.

Für den Fall, dass die Zusammensetzung als Silangruppen-haltiges Polymer ein Polyorganosiloxan mit endständigen Silangruppen enthält, ist sowohl eine einkomponentige Zusammensetzung, auch als RTV-1 bezeichnet, als auch eine zweikomponentige Zusammensetzung, auch als RTV-2 bezeichnet, bevorzugt. Im Fall einer RTV-2 Zusammensetzung ist das Polyorganosiloxan mit endständigen Silangruppen bevorzugt ein Bestandteil der ersten Komponente und der Silanvernetzer, insbesondere ein Silan der Formel (IV), bevorzugt ein Bestandteil der zweiten Komponente, und der Katalysator der Formel (I) kann entweder in der ersten und/oder in der zweiten Komponente enthalten sein.

Für den Fall, dass die Zusammensetzung als Silangruppen-haltiges Polymer ein Silangruppen-haltiges organisches Polymer enthält, ist die Zusammensetzung bevorzugt einkomponentig.

Die erfindungsgemässe Zusammensetzung wird insbesondere in einem Temperaturbereich zwischen 5 und 45 °C, bevorzugt bei Umgebungstemperatur, appliziert und härtet auch bei diesen Bedingungen aus.

Bei der Applikation der beschriebenen Zusammensetzung auf mindestens einen Festkörper oder Artikel kommen die in der Zusammensetzung enthaltenen Silangruppen in Kontakt mit Feuchtigkeit. Bei Kontakt mit Feuchtigkeit hydrolysieren die Silangruppen. Dabei bilden sich Silanolgruppen (Si-OH-Gruppen) und durch nachfolgende Kondensationsreaktionen Siloxangruppen (Si-O-Si-Gruppen). Gegebenenfalls vorhandene weitere feuchtigkeitsreaktive Gruppen reagieren ebenfalls mit vorhandener Feuchtigkeit. Als Ergebnis dieser Reaktionen härtet die Zusammensetzung aus. Der Katalysator der Formel (I) beschleunigt diese Aushärtung.
Falls für die Aushärtung Wasser benötigte wird, kann dieses entweder aus der Luft stammen (Luftfeuchtigkeit), oder aber die vorgängig beschriebene Zusammensetzung kann mit einer Wasser enthaltenden Komponente in Kontakt gebracht werden, zum Beispiel durch Bestreichen, beispielsweise mit einem Abglättmittel, oder durch Besprühen, oder es kann der Zusammensetzung bei der Applikation Wasser oder eine Wasser enthaltende Komponente zugesetzt werden, zum Beispiel in Form einer wasserhaltigen oder Wasser freisetzenden Flüssigkeit oder Paste. Eine Paste ist insbesondere geeignet für den Fall, dass die Zusammensetzung selber in Form einer Paste vorliegt. Eine derartige Wasser enthaltende Komponente wird der erfindungsgemässen Zusammensetzung typischerweise in einem Gewichtsverhältnis von erfindungsgemässer Zusammensetzung zu Wasser enthaltenden Komponente im Bereich von 5:1 bis 200:1, bevorzugt 10:1 bis 100:1, insbesondere 10:1 bis 50:1, zugesetzt.
Eine zweite oder gegebenenfalls weitere Komponenten wird bzw. werden mit der ersten Komponente vermischt, insbesondere über einen Statikmischer oder über einen dynamischen Mischer.
Bei einer Aushärtung mittels Luftfeuchtigkeit härtet die Zusammensetzung von aussen nach innen durch, wobei zunächst eine Haut an der Oberfläche der Zusammensetzung gebildet wird. Die sogenannte Hautbildungszeit stellt ein Mass für die Aushärtungsgeschwindigkeit der Zusammensetzung dar. Die Geschwindigkeit der Aushärtung wird dabei im Allgemeinen von verschiedenen Faktoren, wie beispielsweise der Verfügbarkeit von Wasser, der Temperatur usw., bestimmt.

Die erfindungsgemässe Zusammensetzung verfügt über eine gute Lagerfähigkeit ohne Separationsneigung, erlaubt aufgrund der geringen Toxizität und geringen Flüchtigkeit des Katalysators der Forrmel (I) eine niedrige Gefahreneinstufung und ermöglicht emissions- und geruchsarme Zusammensetzungen, welche rasch aushärten und dabei ein mechanisch hochwertiges und beständiges Material bilden. Besonders vorteilhaft ist dabei der Umstand, dass dieses Material kaum zu migrationsbedingten Fehlern wie Ausschwitzen oder Substratverschmutzung neigt, im Gegensatz zu Zusammensetzungen enthaltend Katalysatoren nach dem Stand der Technik, wie beispielsweise DBU, TMG, DHA oder Titan(IV)-Komplexe. Zusammensetzungen enthaltend solche Katalysatoren neigen zu Migrationseffekten, was sich vor der Aushärtung durch Separation und nach der Aushärtung durch klebrige und/oder schmierige Oberflächen und/oder Substratverschmutzungen äussern kann. Letztere Effekte sind äusserst unerwünscht, da klebrige und schmierige Oberflächen schnell verschmutzen und schlecht überstreichbar sind, und Substratverunreinigungen zu bleibenden Verfärbungen führen können.

Die erfindungsgemässe Zusammensetzung eignet sich für eine Vielzahl von Verwendungen, insbesondere als Anstrich, Lack oder Primer, als Harz zur Herstellung von Faserverbundwerkstoff (Composite), als Vergussmasse, Dichtstoff, Klebstoff, Belag, Beschichtung oder Anstrich für Bau- und Industrieanwendungen, beispielsweise als Nahtabdichtung, Hohlraumversiegelung, Elektroisolationsmasse, Spachtelmasse, Fugendichtstoff, Schweiss- oder Bördelnahtdichtstoff, Montageklebstoff, Karrosserieklebstoff, Scheibenklebstoff, Sandwichelementklebstoff, Kaschierklebstoff, Laminatklebstoff, Verpackungsklebstoff, Holzklebstoff, Parkettklebstoff, Verankerungsklebstoff, Bodenbelag, Bodenbeschichtung, Balkonbeschichtung, Dachbeschichtung, Betonschutzbeschichtung, Parkhausbeschichtung, Versiegelung, Rohrbeschichtung, Korrosionsschutzbeschichtung, Textilbeschichtung, Dämpfungselement, Dichtungselement oder Spachtelmasse.

Bei der Applikation wird die erfindungsgemässe Zusammensetzung auf mindestens ein Substrat appliziert.
Geeignete Substrate sind insbesondere
- Glas, Glaskeramik, Beton, Mörtel, Backstein, Ziegel, Gips oder Natursteine wie Kalkstein, Granit oder Marmor;
- Metalle und Legierungen, wie Aluminium, Eisen, Stahl oder Buntmetalle, sowie oberflächenveredelte Metalle oder Legierungen, wie verzinkte oder verchromte Metalle;
- Leder, Textilien, Papier, Holz, mit Harzen, beispielsweise Phenol-, Melamin- oder Epoxidharzen, gebundene Holzwerkstoffe, Harz-Textil-Verbundwerkstoffe und weitere sogenannte Polymer-Composites;
- Kunststoffe, wie Polyvinylchlorid (Hart- und Weich-PVC), Acrylonitril-Butadien-Styrol-Copolymere (ABS), Polycarbonat (PC), Polyamid (PA), Polyester, Poly(methylmethacrylat) (PMMA), Epoxidharze, Polyurethane (PUR), Polyoxymethylen (POM), Polyolefine (PO), Polyethylen (PE) oder Polypropylen (PP), Ethylen/Propylen-Copolymere (EPM) oder Ethylen/Propylen/Dien-Terpolymere (EPDM), oder faserverstärkte Kunststoffe wie Kohlefaserverstärkte Kunststoffe (CFK), Glasfaser-verstärkte Kunststoffe (GFK) oder Sheet Moulding Compounds (SMC), wobei die Kunststoffe bevorzugt mittels Plasma, Corona oder Flammen oberflächenbehandelt sein können;
- beschichtete Substrate, wie pulverbeschichtete Metalle oder Legierungen;
- Farben oder Lacke, insbesondere Automobildecklacke.

Die Substrate können bei Bedarf vor dem Applizieren der Zusammensetzung vorbehandelt werden, insbesondere durch physikalische und/oder chemische Reinigungsverfahren oder durch das Aufbringen eines Haftvermittlers, einer Haftvermittlerlösung oder eines Primers.
Besonders geeignet ist die erfindungsgemässe Zusammensetzung für den Kontakt mit Substraten, die besonders empfindlich sind auf Störungen durch migrierende Substanzen, insbesondere durch das Ausbilden von Verfärbungen oder Flecken. Dies sind insbesondere feinporige Substrate wie Marmor, Kalkstein oder andere Natursteine, Gips, Zementmörtel oder Beton, aber auch Kunststoffe. Insbesondere auf PVC werden bei der Anwesenheit von Katalysatoren wie beispielsweise DBU oder TMG starke Verfärbungen beobachtet, die sich durch Reinigung nicht entfernen lassen. Solche Effekte werden mit den Katalysatoren der Formel (I) nicht beobachtet.

Besonders geeignet ist die Zusammensetzung als Klebstoff und/oder Dichtstoff, insbesondere für die Fugenabdichtung und für elastische Klebeverbindungen in Bau- und Industrieanwendungen, sowie als elastische Beschichtung mit rissüberbrückenden Eigenschaften, insbesondere zum Schützen und/oder Abdichten von beispielsweise Dächern, Böden, Balkonen, Parkdecks oder Betonröhren.

Insbesondere betrifft die vorliegende Erfindung somit auch die Verwendung einer vorhergehend beschriebenen Zusammensetzung als Klebstoff, Dichtstoff oder Beschichtung.
Eine solche Zusammensetzung enthält typischerweise Weichmacher, Füllstoffe, Haftvermittler und/oder Vernetzer und Trocknungsmittel und gegebenenfalls weitere Hilfs- und Zusatzstoffe.
Üblicherweise beträgt dabei der Anteil an Silangruppen-haltigem Polymer in der erfindungsgemässen Zusammensetzung 10 bis 80 Gew.-%, insbesondere 15 bis 60 Gew.-%, bevorzugt 15 bis 50 Gew.-%, bezogen auf das Gesamtgewicht der Zusammensetzung.

Für eine Anwendung als Klebstoff oder Dichtstoff weist die Zusammensetzung bevorzugt eine pastöse Konsistenz mit strukturviskosen Eigenschaften auf. Ein solcher pastöser Dichtstoff oder Klebstoff wird insbesondere aus handelsüblichen Kartuschen, welche manuell, mittels Druckluft oder Batterie betrieben werden, oder aus einem Fass oder Hobbock mittels einer Förderpumpe oder eines Extruders, gegebenenfalls mittels eines Applikationsroboters, auf ein Substrat aufgetragen.
Für eine Anwendung als Beschichtung weist die Zusammensetzung bevorzugt eine bei Raumtemperatur flüssige Konsistenz mit selbstverlaufenden Eigenschaften auf. Gegebenenfalls ist sie leicht thixotrop, so dass die Beschichtung an abschüssigen bis senkrechten Flächen applizierbar ist, ohne sofort wegzufliessen. Sie wird insbesondere appliziert mittels Roller oder Pinsel oder durch Ausgiessen und Verteilen mittels beispielsweise einem Roller, einem Schaber oder einer Zahntraufel.

Verklebt oder abgedichtet werden können zwei gleichartige oder zwei verschiedene Substrate, insbesondere die vorgängig genannten Substrate.

Weiterhin betrifft die Erfindung eine ausgehärtete Zusammensetzung, welche erhältlich ist aus einer Zusammensetzung, wie sie vorhergehend beschrieben worden ist, nach deren Härtung. Die Aushärtung erfolgt mit Wasser, insbesondere in Form von Luftfeuchtigkeit, und/oder mit einem geeigneten Vernetzer. Die ausgehärtete Zusammensetzung zeichnet sich dadurch aus, dass sie kaum oder gar nicht zu migrationsbedingten Fehlern wie Ausschwitzen oder Substratverschmutzung neigt. Dies im Gegensatz zu ausgehärteten Zusammensetzungen auf Basis von Silangruppen-haltigen Polymeren enthaltend Amidin- oder Guanidin-Katalysatoren, wie sie aus dem Stand der Technik bekannt sind, wo katalysatorbedingte Migrationseffekte bekannt sind.

Aus der Verwendung als Klebstoff, Dichtstoff oder Beschichtung entsteht ein Artikel, welcher mit der beschriebenen Zusammensetzung verklebt, abgedichtet oder beschichtet wurde. Beim Artikel handelt es sich insbesondere um ein Bauwerk, insbesondere ein Bauwerk des Hoch- oder Tiefbaus, um ein industriell gefertigtes Gut oder um ein Konsumgut, insbesondere ein Fenster, eine Haushaltmaschine oder ein Transportmittel wie insbesondere ein Automobil, ein Bus, ein Lastkraftwagen, ein Schienenfahrzeug, ein Schiff, ein Flugzeug oder ein Helikopter; oder der Artikel kann ein Anbauteil davon sein.

Weiterhin betrifft die vorliegende Erfindung die Verwendung eines Katalysators der Formel (I), wie er vorhergehend beschrieben worden ist, als Vernetzungskatalysator für härtbare Zusammensetzungen, insbesondere für Silangruppen-haltige härtbare Zusammensetzungen, insbesondere wie sie vorhergehend beschrieben worden sind.
In einer bevorzugten Verwendung des Katalysators der Formel (I) ist die feuchtigkeitshärtende Zusmmensetzung im Wesentlichen frei von Organozinn-Verbindungen. Insbesondere beträgt der Zinngehalt der Zusammensetzung weniger als 0.06 Gew.-%, insbesondere weniger als 0.01 Gew.-%.

Ein weiterer Gegenstand der vorliegenden Erfindung ist ein Katalysator der Formel (I a'), 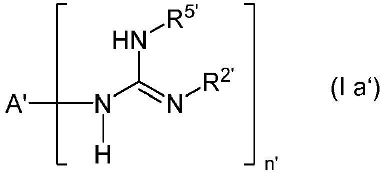 wobei
n' für 2 oder 3, insbesondere für 2, steht;
A' für einen n'-wertigen aliphatischen oder cycloaliphatischen oder arylaliphatischen Kohlenwasserstoff-Rest mit 2 bis 18 C-Atomen, welcher gegebenenfalls Heteroatome enthält und gegebenenfalls Aminogruppen aufweist, steht; und
R^{2'} und R^{5'} unabhängig voneinander jeweils für einen Alkyl-, Cycloalkyl- oder Aralkyl-Rest mit 1 bis 18 C-Atomen, welcher gegebenenfalls Heteroatome enthält, stehen.
Der Katalysator der Formel (I a') entspricht dabei dem bereits beschriebenen Katalysator der Formel (I a) mit bevorzugten Definitionen für n, A, R² und R⁵.

A steht bevorzugt für einen n-wertigen aliphatischen oder cycloaliphatischen oder arylaliphatischen Kohlenwasserstoff-Rest mit 2 bis 12 C-Atomen, welcher gegebenenfalls 1 bis 3 Ethersauerstoffe und gegebenenfalls 1 bis 3 sekundäre oder tertiäre Aminogruppen enthält.
R^{2'} und R^{5'} stehen bevorzugt unabhängig voneinander jeweils für einen Alkyl-, Cycloalkyl- oder Aralkyl-Rest mit 1 bis 12 C-Atomen, insbesondere 1 bis 8 C-Atomen, welcher gegebenenfalls Heteroatome, insbesondere ein Stickstoffatom, enthält, wobei der Katalysator frei von Silangruppen ist.
Besonders bevorzugt stehen R^{2'} und R^{5'} unabhängig voneinander jeweils für Ethyl, Isopropyl, tert.Butyl, 3-(Dimethylamino)propyl oder Cyclohexyl, insbesondere jeweils für Isopropyl oder Cyclohexyl, am meisten bevorzugt jeweils für Cyclohexyl.

Ein Katalysator der Formel (I a') ist geruchlos und somit besonders vorteilhaft. Er ist besonders gut herstellbar und weist eine besonders hohe katalytische Aktivität und gute Selektivität in Bezug auf die Hydrolyse und Kondensationsreaktion von Silangruppen auf. Dadurch ermöglicht er silanvernetzende Zusammensetzungen mit einer guten Lagerfähigkeit vor und einer raschen Aushärtung unter Ausbildung mechanisch hochwertiger und beständiger Materialien nach der Applikation, und er neigt als Bestandteil einer härtbaren Zusammensetzung weder vor noch nach der Aushärtung zu migrationsbedingten Fehlern wie Separation, Ausschwitzen und Substratverschmutzung.
Die bevorzugten Ausführungsformen sind besonders gut herstellbar und katalytisch besonders aktiv.
Der Katalysators der Formel (I a') kann vorteilhaft verwendet werden in einer härtbaren Zusammensetzung, insbesondere in einer Silangruppen-haltigen Zusammensetzung.

### Beispiele

Im Folgenden sind Ausführungsbeispiele aufgeführt, welche die beschriebene Erfindung näher erläutern sollen. Selbstverständlich ist die Erfindung nicht auf diese beschriebenen Ausführungsbeispiele beschränkt.

Als "Normklima" wird eine Temperatur von 23±1 °C und eine relative Luftfeuchtigkeit von 50±5% bezeichnet.

**¹H-NMR-Spektren** wurden auf einem Spektrometer des Typs Bruker Ascend 400 bei 400.14 MHz gemessen; die chemischen Verschiebungen δ sind angegeben in ppm relativ zu Tetramethylsilan (TMS). Echte und Pseudo-Kopplungsmuster wurden nicht unterschieden.
**Infrarotspektren** (FT-IR) wurden auf einem mit horizontaler ATR-Messeinheit mit Diamant-Kristall ausgestatteten FT-IR Gerät Nicolet iS5 von Thermo Scientific gemessen. Flüssige Proben wurden unverdünnt als Filme aufgetragen, feste Proben wurden in CH₂Cl₂ gelöst. Die Absorptionsbanden sind in Wellenzahlen (cm⁻¹) angegeben (Messfenster: 4000-650 cm⁻¹). **Viskositäten** wurden auf einem thermostatisierten Kegel-Platten-Viskosimeter Rheotec RC30 (Kegeldurchmesser 50 mm, Kegelwinkel 1°, Kegelspitze-Platten-Abstand 0.05 mm, Scherrate 10 S⁻¹) gemessen.
Die **Hautbildungszeit** (HBZ) wurde dadurch bestimmt, dass einige Gramm der Zusammensetzung in einer Schichtdicke von ca. 2 mm auf Pappkarton aufgetragen wurden und im Normklima die Zeitdauer gemessen wurde, bis beim leichten Antippen der Oberfläche der Zusammensetzung mittels einer Pipette aus LDPE erstmals keine Rückstände auf der Pipette mehr zurückblieben.
Die Beschaffenheit der **Oberfläche** wurde haptisch geprüft.
Die mechanischen Eigenschaften **Zugfestigkeit, Bruchdehnung** und **E-Modul** (bei 0-5% und bei 0-50% Dehnung) wurden gemäss DIN EN 53504 bei einer Zuggeschwindigkeit von 200 mm/min. gemessen.
Die **Shore A-Härte** wurde bestimmt nach DIN 53505 an während 7 Tagen im Normklima ausgehärteten Prüfkörpern.

### Herstellung von Katalysatoren der Formel (I):

### Katalysator K-1: 1,1'-(2,2(4),4-Trimethyl-1,6-hexamethylen)bis(2,3-diisopropylguanidin)

In einem Rundkolben wurden 3.17 g Vestamine® TMD (von Evonik) und 5.05 g N,N'-Diisopropylcarbodiimid vermischt und die Mischung unter Rühren auf 120 °C erwärmt. In regelmässigen Abständen wurde das Reaktionsgemisch mittels FT-IR-Spektroskopie untersucht. Nach 24 Stunden war die Carbodiimid-Bande bei ca. 2120 cm⁻¹ vollständig verschwunden. Darauf wurde das Reaktionsgemisch im Vakuum von den flüchtigen Bestandteilen befreit. Man erhielt 8.21 g eines hellgelben, geruchlosen Öls.
FT-IR: 3305 (N-H), 2959, 2927, 2867, 1632 (C=N), 1504, 1465, 1361, 1327, 1172, 1125, 860, 713.

### Katalysator K-2: 1,1'-(4-Aza-N-methyl-1,7-heptylen)bis(2,3-diisopropylguanidin)

In einem Rundkolben wurden 2.92 g N,N-Bis(3-aminopropyl)methylamin und 4.91 g N,N'-Diisopropylcarbodiimid vermischt und die Mischung unter Rühren auf 120 °C erwärmt. In regelmässigen Abständen wurde das Reaktionsgemisch mittels FT-IR-Spektroskopie untersucht. Nach 19 Stunden war die Carbodiimid-Bande bei ca. 2120 cm⁻¹ vollständig verschwunden. Darauf wurde das Reaktionsgemisch im Vakuum von den flüchtigen Bestandteilen befreit. Man erhielt 7.74 g eines hellgelben, geruchlosen Öls.
FT-IR: 3271 (N-H), 2960, 2925, 2866, 2796, 1632 (C=N), 1505, 1464, 1361, 1355, 1173, 1124, 860, 714.

### Katalysator K-3: 1,1'-(2,2(4),4-Trimethyl-1,6-hexamethylen)bis(2,3-dicyclohexylguanidin)

In einem Rundkolben wurden 2.25 g Vestamine® TMD (von Evonik) und 5.85 g N,N'-Dicyclohexylcarbodiimid vermischt und die Mischung unter Rühren auf 120 °C erwärmt. In regelmässigen Abständen wurde das Reaktionsgemisch mittels FT-IR-Spektroskopie untersucht. Nach 26 Stunden war die Carbodiimid-Bande bei ca. 2120 cm⁻¹ vollständig verschwunden. Dann wurden 8.86 g Acclaim® 4200 (Polyoxypropylendiol, von Bayer) dazugegeben und 10 Minuten gerührt. Darauf wurde das Reaktionsgemisch im Vakuum von den flüchtigen Bestandteilen befreit. Man erhielt 16.97 g eines hellgelben, geruchlosen Öls. FT-IR: 3436, 2968, 2925, 2852, 1641 (C=N), 1497, 1448, 1372, 1342, 1255, 1114, 1013, 926, 889, 713.

### Katalysator K-4: 1,1'-(2-Methyl-1,5-pentylen)bis(2,3-dicyclohexylguanidin)

In einem Rundkolben wurden 1.67 g 1,5-Diamino-2-methylpentan (Dytek® A, von Invista) und 5.30 g N,N'-Dicyclohexylcarbodiimid vermischt und die Mischung unter Rühren auf 120 °C erwärmt. In regelmässigen Abständen wurde das Reaktionsgemisch mittels FT-IR-Spektroskopie untersucht. Nach 24 Stunden war die Carbodiimid-Bande bei ca. 2120 cm⁻¹ vollständig verschwunden. Dann wurden 6.93 g Acclaim® 4200 (Polyoxypropylendiol, von Bayer) dazugegeben und 10 Minuten gerührt. Darauf wurde das Reaktionsgemisch im Vakuum von den flüchtigen Bestandteilen befreit. Man erhielt 13.89 g eines hellgelben, geruchlosen Öls.
FT-IR: 2924, 2851, 1641 (C=N), 1497, 1448, 1371, 1343, 1255, 1100, 1013, 926, 888, 712.

### Katalysator K-5: 1,1'-(3,6-Dioxa-1,8-octylen)bis(2,3-dicyclohexylguanidin)

In einem Rundkolben wurden 2.13 g 3,6-Dioxaoctan-1,8-diamin (Jeffamine® EDR-148 von Huntsman) und 5.85 g N,N'-Dicyclohexylcarbodiimid vermischt und die Mischung unter Rühren auf 120 °C erwärmt. In regelmässigen Abständen wurde das Reaktionsgemisch mittels FT-IR-Spektroskopie untersucht. Nach 24 Stunden war die Carbodiimid-Bande bei ca. 2120 cm⁻¹ vollständig verschwunden. Dann wurden 6.62 g Acclaim® 4200 (Polyoxypropylendiol, von Bayer) dazugegeben und 10 Minuten gerührt. Darauf wurde das Reaktionsgemisch im Vakuum von den flüchtigen Bestandteilen befreit. Man erhielt 14.60 g eines hellgelben, geruchlosen Öls.
FT-IR: 3362, 2921, 2849, 1627 (C=N), 1505, 1447, 1337, 1237, 1113, 888, 715.

### Katalysator K-6: 1,1'-(α,ω)-Polyoxypropylen)bis(2,3-dicyclohexylguanidin) mit einem Molekulargewicht von etwa 840 g/mol

In einem Rundkolben wurden 10.54 g Polyoxypropylendiamin mit einem mittleren Molekukargewicht von ca. 430 g/mol (Jeffamine® D-400 von Huntsman, Amingehalt ca. 4.4 meq/g) und 8.23 g N,N'-Dicyclohexylcarbodiimid vermischt und die Mischung unter Rühren auf 120 °C erwärmt. In regelmässigen Abständen wurde das Reaktionsgemisch mittels FT-IR-Spektroskopie untersucht.

Nach 24 Stunden war die Carbodiimid-Bande bei ca. 2120 cm⁻¹ vollständig verschwunden. Darauf wurde das Reaktionsgemisch im Vakuum von den flüchtigen Bestandteilen befreit. Man erhielt 18.77 g eines hellgelben, geruchlosen Öls mit einer Viskosität bei 25 °C von 12.3 Pa·s.
FT-IR: 3357 (N-H), 2968, 2924, 2951, 1637 (C=N), 1498, 1448, 1372, 1340, 1256, 1237, 1100, 1018, 926, 889, 845, 715.

### Katalysator K-7: 1,1'-(α,ω)-Polyoxypropylen)bis(2,3-diisopropylguanidin) mit einem Molekulargewicht von etwa 680 g/mol

In einem Rundkolben wurden 10.54 g Polyoxypropylendiamin mit einem mittleren Molekukargewicht von ca. 430 g/mol (Jeffamine® D-400 von Huntsman, Amingehalt ca. 4.4 meq/g) und 2.95 g N,N'-Diisopropylcarbodiimid vermischt und die Mischung unter Rühren auf 120 °C erwärmt. In regelmässigen Abständen wurde das Reaktionsgemisch mittels FT-IR-Spektroskopie untersucht. Nach 18 Stunden war die Carbodiimid-Bande bei ca. 2120 cm⁻¹ vollständig verschwunden. Darauf wurde das Reaktionsgemisch im Vakuum von den flüchtigen Bestandteilen befreit. Man erhielt 8.50 g eines hellgelben, geruchlosen Öls mit einer Viskosität bei 25 °C von 0.4 Pa·s.
FT-IR: 3365 (N-H), 2955, 2929, 2967, 1640 (C=N), 1452, 1372, 1338, 1295, 1099, 1018, 923, 859, 845, 713.

### Katalysator K-8: 1,1'-(4-Aza-N-methyl-1,7-heptylen)bis(2,3-dicyclohexylguanidin)

In einem Rundkolben wurden 1.52 g N,N-Bis(3-aminopropyl)methylamin und 4.26 g N,N'-Dicyclohexylcarbodiimid vermischt und die Mischung unter Rühren auf 120 °C erwärmt. In regelmässigen Abständen wurde das Reaktionsgemisch mittels FT-IR-Spektroskopie untersucht. Nach 24 Stunden war die Carbodiimid-Bande bei ca. 2120 cm⁻¹ vollständig verschwunden. Dann wurden 4.50 g Acclaim® 4200 (Polyoxypropylendiol, von Bayer) dazugegeben und 10 Minuten gerührt. Darauf wurde das Reaktionsgemisch im Vakuum von den flüchtigen Bestandteilen befreit. Man erhielt 10.28 g eines hellgelben, geruchlosen Öls.
FT-IR: 3270, 2921, 2849, 1633 (C=N), 1495, 1447, 1360, 1325, 1255, 1236, 1145, 1112, 888, 714.

### Katalysator K-9: 1,1'-(3,6-Diaza-1,8-octylen)bis(2,3-dicyclohexylguanidin)

In einem Rundkolben wurden 1.86 g Triethylentetramin (techn., Amingehalt ca. 25.7 mmol N/g) und 5.20 g N,N'-Dicyclohexylcarbodiimid vermischt und die Mischung unter Rühren auf 120 °C erwärmt. In regelmässigen Abständen wurde das Reaktionsgemisch mittels FT-IR-Spektroskopie untersucht. Nach 6 Stunden war die Carbodiimid-Bande bei ca. 2120 cm⁻¹ vollständig verschwunden. Dann wurden 7.66 g Acclaim® 4200 (Polyoxypropylendiol, von Bayer) dazugegeben und 10 Minuten gerührt. Darauf wurde das Reaktionsgemisch im Vakuum von den flüchtigen Bestandteilen befreit. Man erhielt 14.72 g eines hellgelben, geruchlosen Öls.
FT-IR: 3270, 2921, 2849, 1632 (C=N), 1506, 1447, 1398, 1359, 1255, 1236, 1146, 888, 717.

### Katalysator K-10: 1,1'-(3,6,9-Triaza-1,11-undecylen)bis(2,3-dicyclohexylguanidin)

In einem Rundkolben wurden 2.88 g Tetraethylenpentamin (Epikure™ Curing Agent 927, von Momentive) und 6.20 g N,N'-Dicyclohexylcarbodiimid vermischt und die Mischung unter Rühren auf 120 °C erwärmt. In regelmässigen Abständen wurde das Reaktionsgemisch mittels FT-IR-Spektroskopie untersucht. Nach 4 Stunden war die Carbodiimid-Bande bei ca. 2120 cm⁻¹ vollständig verschwunden. Darauf wurde das Reaktionsgemisch im Vakuum von den flüchtigen Bestandteilen befreit. Man erhielt 9.07 g eines hellgelben, glasartigen Feststoffs.
FT-IR: 3270 (N-H), 2921, 2948, 1632 (C=N), 1497, 1446, 1335, 1254, 1237, 1145, 888, 718.

### Katalysator K-11:

In einem Rundkolben wurden 3.70 g Polyethylenimin (Lupasol® FG, von BASF; mittleres Molekulargewicht ca. 800 g/mol) und 7.27 g N,N'-Dicyclohexylcarbodiimid vermischt und die Mischung unter Rühren auf 120 °C erwärmt. In regelmässigen Abständen wurde das Reaktionsgemisch mittels FT-IR-Spektroskopie untersucht. Nach 3 Stunden war die Carbodiimid-Bande bei ca. 2120 cm⁻¹ vollständig verschwunden. Dann wurden 10.31 g Acclaim® 4200 (Polyoxypropylendiol, von Bayer) dazugegeben und 10 Minuten gerührt. Darauf wurde das Reaktionsgemisch im Vakuum von den flüchtigen Bestandteilen befreit. Man erhielt 21.28 g eines hellgelben, geruchlosen Wachses.
FT-IR: 3281 (N-H), 2923, 2849, 1634 (C=N), 1496, 1447, 1371, 1337, 1255, 1102, 1014, 908, 888, 715.

### Katalysator K-12:

In einem Rundkolben wurden 6.62 g Polyethylenimin (Lupasol® FG, von BASF; mittleres Molekulargewicht ca. 800 g/mol) und 3.36 g N,N'-Dicyclohexylcarbodiimid vermischt und die Mischung unter Rühren auf 120 °C erwärmt. In regelmässigen Abständen wurde das Reaktionsgemisch mittels FT-IR-Spektroskopie untersucht. Nach 90 Minuten war die Carbodiimid-Bande bei ca. 2120 cm⁻¹ vollständig verschwunden. Darauf wurde das Reaktionsgemisch im Vakuum von den flüchtigen Bestandteilen befreit. Man erhielt 9.99 g eines hellgelben, geruchlosen Öls.
FT-IR: 3277 (N-H), 2922, 2848, 2811, 1633 (C=N), 1447, 1358, 1331, 1270, 1257, 1114, 1048, 888, 844, 761.

### Katalysator K-13: 1,2-Bis(2-methyl-5,6-dihydropyrimidin-1(4H)-yl)ethan

In einem Reagensglas wurden 2.01 g N,N'-Bis(3-aminopropyl)ethylendiamin (N4-Amin, von BASF), 3.29 g Trimethyl-orthoacetat und 0.07 g Lanthan(III)-trifluormethansulfonat vermischt und in einem Mikrowellengerät bei einem Maximaldruck von 150 Pa unter Rühren während 30 Minuten auf 180 °C erwärmt. Darauf wurde das Reaktionsgemisch in einen Rundkolben transferiert und im Vakuum von den flüchtigen Bestandteilen befreit. Man erhielt 2.89 g eines transparenten, geruchsarmen Öls.
¹H-NMR (CDCl₃): δ 1.8-1.9 (m, 4 H, =NCH₂C*H*₂CH₂N-), 2.0 (s, 6 H, Me), 3.23 (*t*, 4 H, =NCH₂CH₂C*H*₂N-), 3.27 (s, 4 H, NCH₂CH₂N), 3.31 (*t*, 4 H, CH₂N=). FT-IR: 3270 (N-H), 2924, 2824, 1605 (C=N), 1475, 1422, 1379, 1311, 1237, 1218, 1111, 1040, 943, 882, 713.

### Katalysator K-14: Bis(2-(2-methyl-4,5-dihydro-1H-imidazol-1-yl)ethyl)amin

In einem Rundkolben wurden 5.68 g Tetraethylenpentamin (Epikure™ Curing Agent 927, von Momentive), 10.73 g Trimethyl-orthoacetat und 0.19 g Lanthan(III)-trifluormethansulfonat vermischt und unter Rühren während 44 Stunden unter Rückfluss erwärmt. Darauf wurde das Reaktionsgemisch im Vakuum von den flüchtigen Bestandteilen befreit. Man erhielt 7.67 g eines hellgelben, geruchsarmen Öls.
FT-IR: 3256 (N-H), 2925, 2813, 1665, 1609 (C=N), 1557, 1418, 1364, 1261, 1144, 1031, 1009, 934, 767.

### Katalysator K-15: N,N"-(2,2(4),4-Trimethylhexan-1,6-diyl)bis(N'-hexylacetimid-amid)

In einem Reagensglas wurden 1.90 g Vestamine® TMD (von Evonik), 2.44 g n-Hexylamin, 3.03 g Trimethyl-orthoacetat und 0.07 g Lanthan(III)-trifluormethansulfonat vermischt und in einem Mikrowellengerät bei einem Maximaldruck von 150 Pa unter Rühren während 30 Minuten auf 160 °C erwärmt. Darauf wurde das Reaktionsgemisch in einen Rundkolben transferiert und im Vakuum von den flüchtigen Bestandteilen befreit. Man erhielt 4.62 g eines leicht trüben, geruchsarmen Öls.
¹H-NMR (CDCl₃): δ 0.8-1.0 (mehrere s, 15 H, CH₃), 1.25-1.38 (m, 12 H, CH₂), 1.4-1.55 (m, 3 H), 1.78-1.85 (m, 6 H, CH₃C=N), 3.05-3.20 (m, 4 H, CH₂N). FT-IR: 3270 (N-H), 2954, 2923, 2855, 1633 (C=N), 1537, 1466, 1376, 1281, 1201, 1095, 1031, 970, 725.

### Katalysator K-16: N,N"-(2,2(4),4-Trimethylhexan-1,6-diyl)bis(N'-(2-methoxyethyl)acetimidamid)

In einem Reagensglas wurden 1.79 g Vestamine® TMD (von Evonik), 1.71 g 2-Methoxyethylamin, 2.86 g Trimethyl-orthoacetat und 0.07 g Lanthan(III)-trifluormethansulfonat vermischt und in einem Mikrowellengerät bei einem Maximaldruck von 150 Pa unter Rühren während 30 Minuten auf 180 °C erwärmt. Darauf wurde das Reaktionsgemisch in einen Rundkolben transferiert und im Vakuum von den flüchtigen Bestandteilen befreit. Man erhielt 3.92 g eines leicht trüben, geruchsarmen Öls.
¹H-NMR (CDCl₃): δ 0.8-1.0 (*m,* 9 H, CH₃), 1.1-1.55 (*m,* 5 H), 1.78-1.85 (*m,* 6 H, CH₃C=N), 2.8-3.2 (*m,* 8 H, CH₂N und CH₂O), 3.3-3.4 (breites s, 6 H, CH₃O), 3.5-3.6 *(m,* 4 H, CH₂N).
FT-IR: 3381 (N-H), 2952, 2891, 2869, 1639 (C=N), 1525, 1455, 1373, 1255, 1196, 1119, 1030, 965, 839.

### Katalysator K-17: N,N"-(2-Methylpentan-1,5-diyl)bis(N'-hexylacetimidamid)

In einem Reagensglas wurden 1.34 g 1,5-Diamino-2-methylpentan (Dytek® A, von Invista), 2.38 g n-Hexylamin, 2.96 g Trimethyl-orthoacetat und 0.06 g Lanthan(III)-trifluormethansulfonat vermischt und in einem Mikrowellengerät bei einem Maximaldruck von 150 Pa unter Rühren während 30 Minuten auf 160 °C erwärmt. Darauf wurde das Reaktionsgemisch in einen Rundkolben transferiert und im Vakuum von den flüchtigen Bestandteilen befreit. Man erhielt 3.95 g eines leicht trüben, geruchsarmen Öls.
¹H-NMR (CDCl₃): δ 0.85-0.95 (*m*, 9 H, CH₃), 1.15-1.2 (*m*, 2 H, CH₂), 1.25-1.38 (*m*, 12 H, CH₂), 1.4-1.7 (*m*, 7 H), 1.85 (s, 6 H, CH₃C=N), 2.85-3.20 (*m*, 8 H, CH₂N).
FT-IR: 3270 (N-H), 2954, 2923, 2853, 1633 (C=N), 1532, 1456, 1376, 1278, 1158, 1090, 1031, 983, 725.

### Katalysator K-18: N,N"-(Propan-1,3-diyl)bis(N'-hexylacetimidamid)

In einem Reagensglas wurden 0.85 g 1,3-Diaminopropan, 2.37 g n-Hexylamin, 2.82 g Trimethyl-orthoacetat und 0.07 g Lanthan(III)-trifluormethansulfonat vermischt und in einem Mikrowellengerät bei einem Maximaldruck von 150 Pa unter Rühren während 30 Minuten auf 160 °C erwärmt. Darauf wurde das Reaktionsgemisch in einen Rundkolben transferiert und im Vakuum von den flüchtigen Bestandteilen befreit. Man erhielt 2.69 g eines leicht trüben, geruchsarmen Öls.
¹H-NMR (CDCl₃): δ 0.85-0.95 (*m*, 6 H, CH₃), 1.25-1.38 (*m*, 12 H, CH₂), 1.52 (*q*, 4 H, C*H₂*CH₂N), 1.76 (*m,* 2 H, NCH₂C*H*₂CH₂N), 1.85 (s, 6 H, CH₃C=N), 3.10 (*m*, 4 H, CH₂N), 3.27-3.35 (*q*, 4 H, NC*H*₂CH₂C*H*₂N).
FT-IR: 3231 (N-H), 2955, 2924, 2854, 1634 (C=N), 1539, 1439, 1381, 1362, 1321, 1294, 1159, 1140, 1096, 1031, 977, 839, 724.

### Katalysator K-19:

In einem Reagensglas wurden 3.52 g 3,6-Dioxaoctan-1,8-diamin (Jeffamine® EDR-148 von Huntsman), 3.42 g Trimethyl-orthoacetat und 0.11 g Lanthan(III)-trifluormethansulfonat vermischt und in einem Mikrowellengerät bei einem Maximaldruck von 150 Pa unter Rühren während 30 Minuten auf 160 °C erwärmt. Darauf wurde das Reaktionsgemisch in einen Rundkolben transferiert und im Vakuum von den flüchtigen Bestandteilen befreit. Man erhielt 4.85 g eines hellgelben, geruchlosen Öls.
¹H-NMR (CDCl₃): δ 1.87 und 1.88 (beide s, 3 H, CH₃), 3.32-3.45 (*m*, 4 H, CH₂O), 3.57-3.65 (*m*, 8 H, CH₂N und CH₂O).
FT-IR: 3367 (N-H), 2863, 1682, 1638 (C=N), 1529, 1440, 1372, 1349, 1257, 1115, 1031, 932, 820, 779.

### Katalysator K-20: N,N"-(3,6-Dioxaoctan-1,8-diyl)bis(N'-hexylacetimidamid)

In einem Reagensglas wurden 1.63 g 3,6-Dioxaoctan-1,8-diamin (Jeffamine® EDR-148 von Huntsman), 2.06 g n-Hexylamin, 3.15 g Trimethyl-orthoacetat und 0.10 g Lanthan(III)-trifluormethansulfonat vermischt und in einem Mikrowellengerät bei einem Maximaldruck von 150 Pa unter Rühren während 30 Minuten auf 160 °C erwärmt. Darauf wurde das Reaktionsgemisch in einen Rundkolben transferiert und im Vakuum von den flüchtigen Bestandteilen befreit. Man erhielt 4.26 g eines leicht trüben, geruchsarmen Öls.
¹H-NMR (CDCl₃): δ 0.89 (*t*, 6 H, C*H*₃CH₂), 1.25-1.38 (*m*, 12 H, CH₂), 1.50 (*q*, 4 H, NCH₂C*H*₂), 1.85-1.88 (*m*, 3 H, CH₃C=N), 3.12 (*t*, 4 H, CH₂N), 3.35 (*q*, 4 H, CH₂O), 3.58-3.68 (*m*, 8 H, CH₂N und CH₂O).
FT-IR: 3373 (N-H), 2954, 2923, 2856, 1685, 1638 (C=N), 1530, 1456, 1440, 1375, 1350, 1257, 1119, 1030, 931, 725.

### Katalysator K-21: N,N"-(2,2(4),4-Trimethylhexan-1,6-diyl)bis(N'-(2-methoxyethyl)acetimidamid)

In einem Reagensglas wurden 1.68 g 3,6-Dioxaoctan-1,8-diamin (Jeffamine® EDR-148 von Huntsman), 1.70 g 2-Methoxyethylamin, 2.86 g Trimethyl-orthoacetat und 0.07 g Lanthan(III)-trifluormethansulfonat vermischt und in einem Mikrowellengerät bei einem Maximaldruck von 150 Pa unter Rühren während 30 Minuten auf 160 °C erwärmt. Darauf wurde das Reaktionsgemisch in einen Rundkolben transferiert und im Vakuum von den flüchtigen Bestandteilen befreit. Man erhielt 3.77 g eines leicht trüben, geruchsarmen Öls.
¹H-NMR (CDCl₃): δ 1.85 (s, 6 H, CH₃C=N), 3.20-3.40 (*m*, 14 H, CH₃O, CH₂N und CH₂O), 3.51 (*m*, 4 H, CH₂N), 3.55-3.65 (*m*, 8 H, CH₂N und CH₂O).
FT-IR: 3374 (N-H), 2866, 1638 (C=N), 1525, 1455, 1374, 1350, 1285, 1196, 1114, 1030, 965, 926, 837.

### Katalysator K-22: N,N"-((Methylazandiyl)bis(propan-3,1-diyl))bis(N'-hexyl-acetimidamid)

In einem Reagensglas wurden 1.80 g N,N-Bis(3-aminopropyl)methylamin, 2.37 g n-Hexylamin, 2.82 g Trimethyl-orthoacetat und 0.07 g Lanthan(III)-trifluormethansulfonat vermischt und in einem Mikrowellengerät bei einem Maximaldruck von 150 Pa unter Rühren während 30 Minuten auf 160 °C erwärmt. Darauf wurde das Reaktionsgemisch in einen Rundkolben transferiert und im Vakuum von den flüchtigen Bestandteilen befreit. Man erhielt 4.36 g eines leicht trüben, geruchsarmen Öls.
¹H-NMR (CDCl₃): δ 0.85-0.95 (*m*, 6 H, CH₃), 1.10-1.38 (*m*, 14 H, CH₂), 1.4-1.7 (*m*, 3H), 1.85 (*s*, 6H, CH₃C=N), 2.85-3.2 (*m*, 8 H, NCH₂), 3.05-3.2 (*m*, 4 H, CH₂N).
FT-IR: 3270 (N-H), 2924, 2853, 2793, 1633 (C=N), 1532, 1456, 1376, 1353, 1294, 1261, 1154, 1051, 1031, 837, 725.

### Herstellung von Silangruppen-haltigen Polymeren:

### Polymer STP-1:

Unter Feuchtigkeitsausschluss wurden 1000 g Polyol Acclaim® 12200 (Polyoxypropylendiol mit niedrigem Ungesättigtheitsgrad, von Bayer; OH-Zahl 11.0 mg KOH/g), 43.6 g Isophorondiisocyanat (IPDI; Vestanat® IPDI, von Evonik), 126.4 g Diisodecylphthalat (DIDP) und 0.1 g Bismuttris(neodecanoat) (10 Gew.-% in DIDP) unter stetigem Rühren auf 90 °C aufgeheizt und auf dieser Temperatur belassen, bis der titrimetrisch bestimmte Gehalt an freien Isocyanatgruppen einen stabilen Wert von 0.63 Gew.-% erreicht hatte. Anschliessend wurden 63.0 g N-(3-Trimethoxysilylpropyl)-amino-bernsteinsäurediethylester (Addukt aus 3-Aminopropyltrimethoxysilan und Maleinsäurediethylester; hergestellt nach den Angaben in US 5,364,955) eingemischt und die Mischung bei 90 °C solange gerührt, bis mittels FT-IR-Spektroskopie kein freies Isocyanat mehr nachgewiesen wurde. Der so erhaltene Trimethoxysilangruppen-haltige Polyether mit einem Silan-Equivalentgewicht von ca. 6880 g/Eq (aus den Einsatzmengen berechnet) wurde auf Raumtemperatur abgekühlt und unter Ausschluss von Feuchtigkeit aufbewahrt.

### Polymer STP-2:

Unter Feuchtigkeitsausschluss wurden 1000 g Polyol Acclaim® 12200 (Polyoxypropylendiol mit niedrigem Ungesättigtheitsgrad, von Bayer; OH-Zahl 11.0 mg KOH/g), 43.6 g Isophorondiisocyanat (IPDI; Vestanat® IPDI, von Evonik), 126.4 g Diisodecylphthalat (DIDP) und 0.1 g Bismuttris(neodecanoat) (10 Gew.-% in DIDP) unter stetigem Rühren auf 90 °C aufgeheizt und auf dieser Temperatur belassen, bis der titrimetrisch bestimmte Gehalt an freien Isocyanatgruppen einen stabilen Wert von 0.64 Gew.-% erreicht hatte. Anschliessend wurden 70.6 g N-(3-Triethoxysilylpropyl)-amino-bernsteinsäurediethylester (Addukt aus 3-Aminopropyltriethoxysilan und Maleinsäurediethylester) eingemischt und die Mischung bei 90 °C solange gerührt, bis mittels FT-IR-Spektroskopie kein freies Isocyanat mehr nachgewiesen wurde. Der so erhaltene Triethoxysilangruppen-haltige Polyether mit einem Silan-Equivalentgewicht von ca. 6920 g/Eq (aus den Einsatzmengen berechnet) wurde auf Raumtemperatur abgekühlt und unter Ausschluss von Feuchtigkeit aufbewahrt.

### Verwendete kommerzielle Katalysatoren und Abkürzungen dafür:

- DBU: 1,8-Diazabicyclo[5.4.0]undec-7-en
- TMG: 1,1,3,3-Tetramethylguanidin
- TMTHP: 2,5,5-Trimethyl-1,4,5,6-tetrahydropyrimidin
- Si-MDHI: 1-(3-(Trimethoxysilyl)propyl)-2-methyl-4,5-dihydro-1*H*-imidazol
- IBAY: Tyzor® IBAY, Bis(ethylacetoacetato)diisobutoxy-titan(IV), von Dorf Ketal

### Zusammensetzungen auf Basis von Silangruppen-haltigen Polymeren:

Die Zusammensetzungen Z1 bis Z63 sind erfindungsgemässe Beispiele, die Zusammensetzungen V1 bis V16 sind Vergleichsbeispiele.

### Zusammensetzungen Z1 bis Z11 und Vergleiche V1 bis V2:

Eine Zusammensetzung aus 96.5 g Polymer STP-1, 0.5 g Vinyltrimethoxysilan und 3.0 g 3-Aminopropyltrimethoxysilan wurde mit verschiedenen Katalysatoren in der angegebenen Menge gemäss Tabelle 1 vermengt und die Mischung auf Viskosität und Hautbildungszeit (HBZ) im Normklima, vor und nach Lagerung, geprüft. Die Hautbildungszeit dient dabei als Mass für die Aktivität des Katalysators in Bezug auf die Vernetzungsreaktion der Silangruppen, d.h. für die Vernetzungsgeschwindigkeit; die Veränderung der Viskosität und der Hautbildungszeit nach Lagerung sind ein Mass für die Lagerstabilität. Weiterhin wurde die applizierte Mischung nach 24 Stunden im Normklima daraufhin geprüft, ob die Oberfläche wie gewünscht trocken war oder sich ein schmieriger Film gebildet hatte, was ein Anzeichen für das Ausschwitzen des Katalysators aufgrund schlechter Verträglichkeit mit dem ausgehärteten Kunststoff ist, und/oder ob die Oberfläche klebrig war, was ein Anzeichen für eine unvollständige Aushärtung ist. Weiterhin wurde von der Mischung ein Film von 2 mm Dicke hergestellt, während 7 Tagen im Normklima aushärten gelassen und auf mechanische Eigenschaften geprüft. Die Ergebnisse sind in Tabelle 1 und 2 wiedergegeben. "Zus." steht für "Zusammensetzung".

**Tabelle 1:**

| **Zus.** | **Katalysator** | **Menge** | **Konzentration¹** | **Viskosität [Pa·s]** | | | **HBZ** | |
|---|---|---|---|---|---|---|---|---|
| | | | | **frisch** | **gelagert²** | **Zunahme** | **frisch** | **gelagert²** |
| **V1** | DBU | 0.28 g | 1.9 | 27.2 | 36.9 | 36% | 25' | 29' |
| **V2** | TMG | 0.21 g | 1.9 | 22.3 | 24.6 | 1 0% | 65' | 75' |
| **Z1** | K-1 | 0.38 g | 1.9 | 25.4 | 33.1 | 30% | 10' | 16' |
| **Z2** | K-2 | 0.36 g | 1.9 | 25.7 | 36.6 | 42% | 7' | 10' |
| **Z3** | K-3 | 1.10 g | 1.9 | 22.2 | 26.1 | 18% | 17' | 17' |
| **Z4** | K-4 | 0.99 g | 1.9 | 22.1 | 27.5 | 24% | 11' | 13' |
| **Z5** | K-5 | 0.94 g | 1.9 | 22.5 | 27.7 | 23% | 14' | 14' |
| **Z6** | K-6 | 0.86 g | 1.9 | 27.8 | 37.4 | 34% | 13' | 12' |
| **Z7** | K-7 | 0.67 g | 1.9 | 25.7 | 33.0 | 28% | 12' | 17' |
| **Z8** | K-8 | 0.91 g | 1.9 | 23.3 | 26.1 | 12% | 9' | 10' |
| **Z9** | K-10 | 0.55 g | 1.9 | 27.8 | 33.1 | 19% | 9' | 16' |
| **Z10** | K-11 | 1.05 g | 1.8 | 20.6 | 22.8 | 11% | 26' | 20' |
| **Z11** | K-12 | 1.12 g | 1.9 | 26.7 | 38.3 | 43% | 17' | 18' |

| | | | | | | | | |
|---|---|---|---|---|---|---|---|---|
| ¹ mmol Amidin- oder Guanidin-Gruppen auf 100 g Silangruppen-haltiges Polymer. ² während 7 Tagen bei 60 °C in geschlossenem Gebinde. | | | | | | | | |

**Tabelle 2:**

| **Zus.** | **Oberfläche nach 24h** | **Zugfestigkeit** | **Bruchdehnung** | **E-Modul** | |
|---|---|---|---|---|---|
| | | | | **0-5%** | **0-50%** |
| **V1** | schmierig | 0.58 MPa | 72% | 1.16 MPa | 0.77 MPa |
| **V2** | klebrig | 0.62 MPa | 90% | 1.19 MPa | 0.75 MPa |
| **Z1** | trocken | 0.61 MPa | 84% | 1.05 MPa | 0.76 MPa |
| **Z2** | trocken | 0.65 MPa | 97% | 1.05 MPa | 0.74 MPa |
| **Z3** | trocken | 0.62 MPa | 79% | 1.11 MPa | 0.77 MPa |
| **Z4** | trocken | 0.66 MPa | 89% | 1.08 MPa | 0.78 MPa |
| **Z5** | trocken | 0.66 MPa | 86% | 1.09 MPa | 0.79 MPa |
| **Z6** | trocken | 0.62 MPa | 89% | 1.11 MPa | 0.75 MPa |
| **Z7** | trocken | 0.61 MPa | 84% | 1.09 MPa | 0.76 MPa |
| **Z8** | trocken | 0.67 MPa | 89% | 1.14 MPa | 0.77 MPa |
| **Z9** | trocken | 0.67 MPa | 100% | 0.99 MPa | 0.73 MPa |
| **Z10** | trocken | 0.66 MPa | 95% | 1.07 MPa | 0.74 MPa |
| **Z11** | trocken | 0.75 MPa | 119% | 1.10 MPa | 0.75 MPa |

### Zusammensetzungen Z12 bis Z21 und Vergleiche V3 bis V4:

Eine Zusammensetzung aus 96.5 g Polymer STP-1, 0.5 g Vinyltrimethoxysilan und 3.0 g 3-Aminopropyltrimethoxysilan wurde mit verschiedenen Katalysatoren in der angegebenen Menge gemäss Tabelle 3 vermengt und die Mischung wie für Zusammensetzung Z1 beschrieben auf Viskosität, Hautbildungszeit (HBZ), Oberflächenbeschaffenheit und mechanische Eigenschaften geprüft. Die Ergebnisse sind in Tabelle 3 und 4 wiedergegeben. "Zus." steht für "Zusammensetzung".

**Tabelle 3:**

| **Zus.** | **Katalysator** | **Menge** | **Konzentration¹** | **Viskosität [Pa·s]** | | | **HBZ** | |
|---|---|---|---|---|---|---|---|---|
| | | | | **frisch** | **gelagert²** | **Zunahme** | **frisch** | **gelagert²** |
| **V3** | TMTHP³ | 0.23 g | 1.9 | 24.4 | 28.7 | 18% | 23' | 23' |
| **V4** | Si-MDHI | 0.45 g | 1.9 | 22.8 | 25.6 | 12% | 90' | 85' |
| **Z12** | K-13³ | 0.20 g | 1.9 | 28.3 | 31.8 | 12% | 37' | 44' |
| **Z13** | K-14 | 0.22 g | 1.9 | 26.6 | 28.9 | 9% | 82' | 105' |
| **Z14** | K-15 | 0.37 g | 1.9 | 27.8 | 33.7 | 21% | 47' | 47' |
| **Z15** | K-16 | 0.33 g | 1.9 | 27.7 | 37.4 | 35% | 49' | 36' |
| **Z16** | K-17 | 0.37 g | 1.9 | 28.4 | 38.5 | 36% | 43' | 35' |
| **Z17** | K-18 | 0.30 g | 1.9 | 26.2 | 31.9 | 22% | 21' | 28' |
| **Z18** | K-19 | 0.31 g | 1.9 | 27.9 | 43.1 | 54% | 105' | 50' |
| **Z19** | K-20 | 0.37 g | 1.9 | 27.0 | 33.8 | 25% | 50' | 49' |
| **Z20** | K-21 | 0.32 g | 1.9 | 28.2 | 37.0 | 31% | 55' | 43' |
| **Z21** | K-22 | 0.36 g | 1.9 | 26.8 | 31.4 | 17% | 35' | 41' |

| | | | | | | | | |
|---|---|---|---|---|---|---|---|---|
| ¹ mmol Amidin- oder Guanidin-Gruppen auf 100 g Silangruppen-haltiges Polymer. ² während 7 Tagen bei 60 °C in geschlossenem Gebinde. ³ als Lösung (25 Gew.-%) in N-Ethylpyrrolidon. | | | | | | | | |

**Tabelle 4:**

| **Zus.** | **Oberfläche nach 24h** | **Zugfestigkeit** | **Bruchdehnung** | **E-Modul** | |
|---|---|---|---|---|---|
| | | | | **0-5%** | **0-50%** |
| **V3** | schmierig | 0.63 MPa | 86% | 1.05 MPa | 0.74 MPa |
| **V4** | leicht klebrig | 0.48 MPa | 49% | 1.13 MPa | 0.78 MPa |
| **Z12** | trocken | 0.72 MPa | 111% | 1.19 MPa | 0.77 MPa |
| **Z13** | trocken | 0.74 MPa | 120% | 0.98 MPa | 0.73 MPa |
| **Z14** | trocken | 0.67 MPa | 88% | 1.08 MPa | 0.78 MPa |
| **Z15** | trocken | 0.66 MPa | 90% | 1.23 MPa | 0.80 MPa |
| **Z16** | trocken | 0.68 MPa | 93% | 1.29 MPa | 0.80 MPa |
| **Z17** | fast trocken | 0.63 MPa | 82% | 1.14 MPa | 0.77 MPa |
| **Z18** | trocken | 0.78 MPa | 123% | 1.09 MPa | 0.77 MPa |
| **Z19** | fast trocken | 0.65 MPa | 85% | 1.08 MPa | 0.78 MPa |
| **Z20** | trocken | 0.72 MPa | 106% | 1.19 MPa | 0.80 MPa |
| **Z21** | fast trocken | 0.68 MPa | 101% | 1.08 MPa | 0.74 MPa |

### Zusammensetzungen Z22 bis Z31 und Vergleiche V5 bis V6:

Eine Zusammensetzung aus 95.9 g Polymer STP-2, 0.4 g Vinyltriethoxysilan und 3.7 g 3-Aminopropyltriethoxysilan wurde mit verschiedenen Katalysatoren in der angegebenen Menge gemäss Tabelle 5 vermengt und die Mischung wie für Zusammensetzung Z1 beschrieben auf Viskosität, Hautbildungszeit (HBZ), Oberflächenbeschaffenheit und mechanische Eigenschaften geprüft. Die Ergebnisse sind in Tabelle 5 und 6 wiedergegeben. "Zus." steht für "Zusammensetzung".

**Tabelle 5:**

| **Zus.** | **Katalysator** | **Menge** | **Konzentration¹** | **Viskosität [Pa·s]** | | | **HBZ** | |
|---|---|---|---|---|---|---|---|---|
| | | | | **frisch** | **gelagert²** | **Zunahme** | **frisch** | **gelagert²** |
| **V5** | DBU | 0.55 g | 3.8 | 34.4 | 37.7 | 10% | 185' | 150' |
| **V6** | TMG | 0.42 g | 3.8 | 34.5 | 42.4 | 23% | >12h | >12h |
| **Z22** | K-1 | 0.75 g | 3.8 | 34.8 | 42.1 | 21% | 70' | 80' |
| **Z23** | K-2 | 0.72 g | 3.8 | 35.0 | 41.1 | 17% | 53' | 47' |
| **Z24** | K-3 | 2.12 g | 3.7 | 35.2 | 35.8 | 2% | 90' | 95' |
| **Z25** | K-4 | 1.92 g | 3.7 | 37.0 | 37.8 | 2% | 75' | 70' |
| **Z26** | K-5 | 1.88 g | 3.8 | 35.3 | 37.2 | 5% | 60' | 75' |
| **Z27** | K-6 | 1.71 g | 3.8 | 38.8 | 39.5 | 2% | 65' | 80' |
| **Z28** | K-7 | 1.29 g | 3.7 | 35.1 | 41.2 | 17% | 105' | 82' |
| **Z29** | K-8 | 1.86 g | 3.9 | 36.3 | 37.4 | 3% | 50' | 72' |
| **Z30** | K-10³ | 1.07 g | 3.7 | 26.8 | 29.4 | 10% | 50' | 72' |
| **Z31** | K-6 | 0.68 g | 1.5 | 43.8 | 44.0 | 1% | 125' | 135' |
| | IBAY | 0.82 g | 1.9 | | | | | |

| | | | | | | | | |
|---|---|---|---|---|---|---|---|---|
| ¹ mmol Amidin- oder Guanidin-Gruppen oder Metallatome auf 100 g Silangruppen-haltiges Polymer. ² während 7 Tagen bei 60 °C in geschlossenem Gebinde. ³ als Lösung (25 Gew.-%) in N-Ethylpyrrolidon. | | | | | | | | |

**Tabelle 6:**

| **Zus.** | **Oberfläche nach 24h** | **Zugfestigkeit** | **Bruchdehnung** | **E-Modul** | |
|---|---|---|---|---|---|
| | | | | **0-5%** | **0-50%** |
| **V5** | schmierig, klebrig | 0.65 MPa | 124% | 0.85 MPa | 0.62 MPa |
| **V6** | sehr stark klebrig | n.b. | n.b. | n.b. | n.b. |
| **Z22** | trocken | 0.69 MPa | 141% | 0.87 MPa | 0.60 MPa |
| **Z23** | trocken | 0.63 MPa | 133% | 0.74 MPa | 0.56 MPa |
| **Z24** | trocken | 0.64 MPa | 120% | 0.83 MPa | 0.61 MPa |
| **Z25** | trocken | 0.63 MPa | 114% | 0.83 MPa | 0.62 MPa |
| **Z26** | trocken | 0.72 MPa | 143% | 0.83 MPa | 0.62 MPa |
| **Z27** | trocken | 0.51 MPa | 77% | 0.87 MPa | 0.62 MPa |
| **Z28** | trocken | 0.59 MPa | 104% | 0.92 MPa | 0.60 MPa |
| **Z29** | trocken | 0.73 MPa | 143% | 0.89 MPa | 0.64 MPa |
| **Z30** | trocken | 0.70 MPa | 139% | 0.80 MPa | 0.61 MPa |
| **Z31** | trocken | 0.65 MPa | 137% | 0.84 MPa | 0.57 MPa |

| | | | | | |
|---|---|---|---|---|---|
| "n.b. steht für "nicht bestimmt" (nicht messbar) | | | | | |

### Zusammensetzungen Z32 bis Z46 und Vergleich V7:

In einem Planetenmischer wurden 36.2 g Polymer STP-1, 60.2 g gemahlene Kreide (Omyacarb® 5 GU, von Omya), 1.2 g Thixotropierpaste, deren Herstellung nachfolgend beschrieben ist, 1.2 g Vinyltrimethoxysilan, 1.2 g 3-Aminopropyltrimethoxysilan sowie verschiedene Katalysatoren in der angegebenen Menge gemäss Tabelle 7 vermengt und die wie für Zusammensetzung Z1 beschrieben auf Hautbildungszeit (HBZ), Oberflächenbeschaffenheit und mechanische Eigenschaften geprüft. Die Ergebnisse sind in Tabelle 7 wiedergegeben. "Zus." steht für "Zusammensetzung".
Die Thixotropierpaste wurde hergestellt, indem in einem Vakuummischer 300 g Diisodecylphthalat (Palatinol® Z, von BASF) und 48 g 4,4'-Methylendiphenyldiisocyanat (Desmodur® 44 MC L, von Bayer) vorgelegt und leicht aufgewärmt und anschliessend unter starkem Rühren 27 g n-Butylamin langsam zugetropft wurden. Die entstehende Paste wurde unter Vakuum und Kühlung eine Stunde weitergerührt.

**Tabelle 7:**

| **Zus.** | **Katalysator** | **Menge** | **Konzentration** | **HBZ** | **Oberfläche nach 24h** | **Zugfestigkeit** | **Bruchdehnung** | **E-Modul [MPa] 0-5% 0-100%** | |
|---|---|---|---|---|---|---|---|---|---|
| **V7** | DBU | 0.12 g | 0.8 | 25' | leicht schmierig | 2.5MPa | 103% | 6.1 | 2.8 |
| **Z32** | K-1 | 0.16 g | 0.8 | 15' | trocken | 3.0MPa | 124% | 5.9 | 2.7 |
| **Z33** | K-3 | 0.48 g | 0.8 | 14' | trocken | 2.8MPa | 118% | 5.9 | 2.6 |
| **Z34** | K-5 | 0.41 g | 0.8 | 10' | trocken | 3.0MPa | 105% | 6.0 | 2.9 |
| **Z35** | K-6 | 0.38 g | 0.8 | 9' | trocken | 3.1 MPa | 125% | 5.9 | 2.9 |
| **Z36** | K-7 | 0.29 g | 0.8 | 12' | trocken | 2.9MPa | 131% | 5.8 | 2.7 |
| **Z37** | K-8 | 0.40 g | 0.8 | 8' | trocken | 2.8MPa | 109% | 5.8 | 2.7 |
| **Z38** | K-9 | 0.47 g | 0.8 | 8' | trocken | 2.9MPa | 111% | 6.0 | 2.8 |
| **Z39** | K-11 | 0.48 g | 0.8 | 20' | trocken | 3.0MPa | 134% | 5.8 | 2.7 |
| **Z40** | K-12 | 0.49 g | 0.8 | 16' | trocken | 3.0MPa | 131% | 5.6 | 2.6 |
| **Z41** | K-13 | 0.09 g | 0.8 | 35' | trocken | 2.8MPa | 139% | 5.4 | 2.6 |
| **Z42** | K-16 | 0.14 g | 0.8 | 60' | trocken | 3.0MPa | 130% | 6.1 | 2.7 |
| **Z43** | K-17 | 0.16 g | 0.8 | 40' | trocken | 2.9MPa | 128% | 5.7 | 2.6 |
| **Z44** | K-19 | 0.14 g | 0.8 | 60' | trocken | 2.5MPa | 166% | 5.6 | 2.2 |
| **Z45** | K-20 | 0.16 g | 0.8 | 50' | trocken | 2.7MPa | 146% | 5.9 | 2.4 |
| **Z46** | K-21 | 0.14 g | 0.8 | 70' | trocken | 2.8MPa | 137% | 6.0 | 2.5 |

| | | | | | | | | | |
|---|---|---|---|---|---|---|---|---|---|
| ¹ mmol Amidin- oder Guanidin-Gruppen auf 100 g Zusammensetzung. | | | | | | | | | |

### Zusammensetzungen Z47 bis Z55 und Vergleich V8:

In einem Planetenmischer wurden 36.2 g Polymer STP-2, 60.2 g gemahlene Kreide (Omyacarb® 5 GU, von Omya), 1.2 g Thixotropierpaste, hergestellt wie für Zusammensetzung Z31 beschrieben, 1.2 g Vinyltriethoxysilan, 1.2 g 3-Aminopropyltriethoxysilan sowie verschiedene Katalysatoren in der angegebenen Menge gemäss Tabelle 8 vermengt und die Mischung wie beschrieben auf Hautbildungszeit (HBZ), Oberflächenbeschaffenheit und mechanische Eigenschaften geprüft. Die Ergebnisse sind in Tabelle 8 wiedergegeben. "Zus." steht für "Zusammensetzung".

**Tabelle 8:**

| **Zus.** | **Katalysator** | **Menge** | **Konzentration¹** | **HBZ** | **Oberfläche nach 24h** | **Zugfestigkeit** | **Bruchdehnung** | **E-Modul [MPa] 0-5% 0-100%** | |
|---|---|---|---|---|---|---|---|---|---|
| **V8** | DBU | 0.40 g | 2.6 | 83' | leicht schmierig | 2.5MPa | 155% | 4.0 | 2.0 |
| **Z47** | K-2 | 0.52 g | 2.6 | 45' | trocken | 3.4MPa | 139% | 5.8 | 2.7 |
| **Z48** | K-3 | 1.56 g | 2.6 | 78' | trocken | 3.3MPa | 154% | 4.4 | 2.5 |
| **Z49** | K-5 | 1.34 g | 2.6 | 64' | trocken | 3.8MPa | 155% | 4.9 | 2.7 |
| **Z50** | K-6 | 1.22 g | 2.6 | 57' | trocken | 3.1 MPa | 167% | 5.0 | 2.5 |
| **Z51** | K-7 | 0.95 g | 2.6 | 60' | trocken | 3.0MPa | 148% | 5.1 | 2.5 |
| **Z52** | K-8 | 1.29 g | 2.6 | 45' | trocken | 3.5MPa | 138% | 5.0 | 2.7 |
| **Z53** | K-9 | 1.52 g | 2.6 | 75' | trocken | 3.2MPa | 145% | 4.6 | 2.5 |
| **Z54** | K-12 | 1.60 g | 2.6 | 59' | trocken | 2.0MPa | 161% | 4.4 | 2.3 |
| **Z55** | K-6 | 0.61 g | 1.3 | 57' | trocken | 2.2MPa | 196% | 4.7 | 1.9 |
| | IBAY | 0.59 g | 1.3 | | | | | | |

| | | | | | | | | | |
|---|---|---|---|---|---|---|---|---|---|
| ¹ mmol Amidin- oder Guanidin-Gruppen oder Metallatome auf 100 g Zusammensetzung. | | | | | | | | | |

### Zusammensetzungen Z56 bis Z57 und Vergleich V9 bis V12:

Eine Zusammensetzung aus 96.5 g Silangruppen-haltiger Polyether TEGOPAC® Bond 150 (von Evonik), 0.5 g Vinyltriethoxysilan und 3.0 g 3-Aminopropyltriethoxysilan wurde mit verschiedenen Katalysatoren in der angegebenen Menge gemäss Tabelle 9 vermengt und die Mischung wie beschrieben auf Hautbildungszeit (HBZ) und Oberflächenbeschaffenheit nach 7 Tagen im Normklima geprüft. Die Ergebnisse sind in Tabelle 9 wiedergegeben. "Zus." steht für "Zusammensetzung".

**Tabelle 9:**

| **Zus.** | **Katalysator** | **Menge** | **Konzentration¹** | **HBZ** | **Oberfläche nach 7d** |
|---|---|---|---|---|---|
| **V9** | DBU | 2.28 g | 15 | 4h 20' | schmierig |
| **V10** | DBTDL | 0.79 g | 1.25 | 1h 45' | trocken |
| **V11** | IBAY | 7.25 g | 15 | 2h | weich und klebrig |
| **V12** | DBU | 4.93 g | 5 | 3h | klebrig |
| | IBAY | 2.40 g | 5 | | |
| **Z56** | K-6 | 6.25 g | 14.7 | 1h | fast trocken |
| **Z57** | K-6 | 2.10 g | 5 | 2h 50' | trocken |
| | IBAY | 2.40 g | 5 | | |

| | | | | | |
|---|---|---|---|---|---|
| ¹ mmol Amidin- oder Guanidin-Gruppen bzw. Metallatome auf 100 g Silangruppen-haltiger Polyether. | | | | | |

### Zusammensetzungen Z58 bis Z59 und Vergleiche V13 bis V14:

In einem Kunststoffbecher wurden 20.2 g einer ersten Komponente bestehend aus 99 Gewichtsteilen (GT) eines OH-terminierten linearen Polydimethylsiloxans mit einer Viskosität von ca. 50'000 mPas bei 23 °C (Wacker® Silicone Rubber Polymer FD 50, von Wacker) und 1 GT Wacker® E 2 Siliconöl-Emulsion (nichtionisch in Wasser emulgiertes mittelviskoses OH-terminiertes lineares Polydimethylsiloxan, von Wacker; Feststoffgehalt 37-40%) mit einer zweiten Komponente bestehend aus 0.80 g Vinyltrimethoxysilan (VTMO) und einem Katalysator in der in der Tabelle 10 angegebenen Art und Menge innig vermischt und und die Mischung wie beschrieben auf Hautbildungszeit (HBZ) und Oberflächenbeschaffenheit geprüft. Weiterhin wurde die applizierte Mischung nach 7 Tagen im Normklima auf Shore A-Härte geprüft. Die Ergebnisse sind in Tabelle 10 wiedergegeben. "Zus." steht für "Zusammensetzung".

**Tabelle 10:**

| **Zus.** | **Katalysator** | **Menge** | **Konzentration¹** | **HBZ** | **Oberfläche nach 24h** | **Shore A nach 7 d** |
|---|---|---|---|---|---|---|
| **V13** | DBU | 0.06 g | 2.0 | 55' | trocken | 3 |
| **V14** | IBAY | 0.48 g | 5.0 | 40' | trocken | <2 |
| **Z58** | K-2 | 0.08 g | 2.0 | 30' | trocken | 6 |
| **Z59** | K-9 | 0.23 g | 2.0 | 26' | trocken | 7 |

| | | | | | | |
|---|---|---|---|---|---|---|
| ¹ mmol Amidin- oder Guanidin-Gruppen oder Metallatome auf 100 g OH-terminiertes Polydimethylsiloxan. | | | | | | |

### Zusammensetzung Z60 (in situ-Herstellung des Katalysators):

Eine Zusammensetzung aus 30.0 g Polymer STP-1, 0.15 g Vinyltrimethoxysilan und 1.2 g 3-Glycidoxypropyltrimethoxysilan wurde unter Feuchtigkeitsausschluss mit 0.8 g Polyoxypropylendiamin mit einem mittleren Molekukargewicht von ca. 430 g/mol (Jeffamine® D-400 von Huntsman, Amingehalt ca. 4.4 meq/g) und 0.4 g N,N'-Diisopropylcarbodiimid vermengt, die Mischung in eine innenlackierte Aluminiumtube abgefüllt und im Ofen auf 80 °C erwärmt. Nach den in der Tabelle 11 angegebenen Zeitabständen wurde die Mischung auf Hautbildungszeit (HBZ) im Normklima sowie auf den Umsatz des Carbodiimids (via Abnahme der Intensität der Carbodiimid-Bande bei ca. 2120 cm⁻¹ im FT-IR, Intensität zu Beginn = 0% Umsatz, keine Bande mehr nachweisbar = 100% Umsatz) geprüft. Die Ergebnisse sind in Tabelle 11 wiedergegeben.

**Tabelle 11:**

| **Zeit** | **HBZ** | **Carbodiimid-Umsatz** |
|---|---|---|
| 0h | > 9h | 0% |
| 2h | 3h 05' | 21% |
| 4h | 1h 05' | 39% |
| 6h | 52' | 64% |
| 26h | 43' | 100% |

### Zusammensetzung Z61 (in situ-Herstellung des Katalysators):

Die in situ-Herstellung des Katalysators aus Zusammensetzung Z60 wurde wiederholt, wobei aber das 3-Glycidoxypropyltrimethoxysilan weggelassen wurde . Die Ergebnisse sind in Tabelle 12 wiedergegeben.

**Tabelle 12:**

| **Zeit** | **HBZ** | **Carbodiimid-Umsatz** |
|---|---|---|
| 0h | 7h | 0% |
| 2h | 2h 20' | 20% |
| 4h | 50' | 51% |
| 6h | 32' | 69% |
| 26h | 26' | 100% |

### Zusammensetzungen Z62 bis Z63 und Vergleiche V15 und V16:

Eine Zusammensetzung aus 96.0 g Silangruppen-haltigem Polyether, entweder GENIOSIL® STP-E 15 (von Wacker) oder Desmoseal® S XP 2821 (von Bayer), 0.35 g Vinyltrimethoxysilan und 3.72 g 3-Aminopropyltrimethoxysilan wurde mit verschiedenen Katalysatoren in der angegebenen Menge gemäss Tabelle 13 vermengt und die Mischung wie für Zusammensetzung Z1 beschrieben geprüft. Die Ergebnisse sind in Tabelle 13 wiedergegeben. "Zus." steht für "Zusammensetzung".

**Tabelle 13:**

| **Zus.** | **Polymer** | **Katalysator, Menge** | **Konzentration¹** | **HBZ** | **Oberfläche nach 24h** | **Zugfestigkeit** | **Bruchdehnung** | **E-Modul 0-5%** |
|---|---|---|---|---|---|---|---|---|
| **V15** | E-15² | DBU, 0.28 g | 1.8 | 60' | schmierig | 0.72 MPa | 48% | 1.84 MPa |
| **Z62** | E-15² | K-6, 0.78 g | 1.7 | 13' | trocken | 0.79 MPa | 54% | 1.83 MPa |
| **V16** | 2821³ | DBU, 0.28 g | 1.8 | 55' | trocken | 0.85 MPa | 28% | 3.2 MPa |
| **Z63** | 2821³ | K-6, 0.78 g | 1.7 | 40' | trocken | 0.92 MPa | 29% | 3.2 MPa |

| | | | | | | | | |
|---|---|---|---|---|---|---|---|---|
| ¹ mmol Amidin- oder Guanidin-Gruppen auf 100 g Silangruppen-haltiger Polyether. ² GENIOSIL® STP-E 15 (von Wacker); ³ Desmoseal® S XP 2821 (von Bayer) | | | | | | | | |

## Patentansprüche

1. Zusammensetzung umfassend
- mindestens ein Silangruppen-haltiges Polymer; und
- mindestens einen Katalysator der Formel (I)
A⁅Z]ₙ (I)
wobei
n für eine ganze Zahl von 2 bis 20 steht
A für einen n-wertigen aliphatischen oder cycloaliphatischen oder arylaliphatischen Kohlenwasserstoff-Rest mit 2 bis 100 C-Atomen, welcher gegebenenfalls ungesättigte Anteile enthält, gegebenenfalls Heteroatome enthält und gegebenenfalls Aminogruppen aufweist, steht, und
Z für einen Rest der Formel 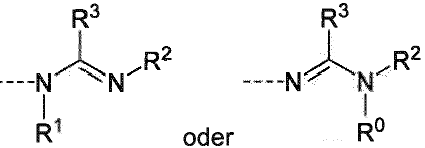 steht,
wobei
R¹ und R⁰ unabhängig voneinander jeweils für Wasserstoff oder für einen Alkyl- oder Cycloalkyl- oder Aralkyl-Rest mit 1 bis 8 C-Atomen stehen,
R² für Wasserstoff oder für einen Alkyl-, Cycloalkyl- oder Aralkyl-Rest mit 1 bis 18 C-Atomen, welcher gegebenenfalls Heteroatome enthält und welcher gegebenenfalls endständige primäre oder sekundäre Aminogruppen aufweist, steht,
R³ für -NR⁴R⁵ oder für Wasserstoff oder für einen Alkyl-, Cycloalkyl- oder Aralkyl-Rest mit 1 bis 12 C-Atomen steht,
R⁴ und R⁵ unabhängig voneinander jeweils für Wasserstoff oder für einen Alkyl-, Cycloalkyl- oder Aralkyl-Rest mit 1 bis 18 C-Atomen, welcher gegebenenfalls Heteroatome enthält, stehen,
wobei
R¹ und R² auch zusammen für einen Alkylen-Rest mit 2 bis 6 C-Atomen stehen können,
R² und R⁰ auch zusammen für einen Alkylen-Rest mit 3 bis 6 C-Atomen, welcher gegebenenfalls Heteroatome enthält, stehen können,
R² und R³ auch zusammen für einen Alkylen-Rest mit 3 bis 6 C-Atomen stehen können,
R⁴ und R⁵ auch zusammen für einen Alkylen-Rest mit 4 bis 7 C-Atomen, welcher gegebenenfalls Heteroatome enthält, stehen können, und
R² und R⁵ auch zusammen für einen Alkylen-Rest mit 2 bis 12 C-Atomen stehen können;
wobei der Katalysator der Formel (I) kein Stickstoffatom enthält, welches direkt an einen aromatischen Ring gebunden oder Teil eines heteroaromatischen Ringsystems ist und wobei der Katalysator der Formel (I) frei von Silangruppen ist.

2. Zusammensetzung nach Anspruch 1, **dadurch gekennzeichnet, dass** A für einen n-wertigen aliphatischen oder cycloaliphatischen oder arylaliphatischen Kohlenwasserstoff-Rest mit 2 bis 50 C-Atomen, welcher gegebenenfalls Ether-Sauerstoff oder Amin-Stickstoff enthält, steht.

3. Zusammensetzung nach einem der vorhergehenden Ansprüche, **dadurch gekennzeichnet, dass** A ausgewählt ist aus der Gruppe bestehend aus 1,2-Ethylen; 1,3-Propylen; 1,2-Propylen; 1,4-Butylen; 1,3-Butylen; 1,2-Butylen; 2,3-Butylen; 1,3-Pentylen; 2-Methyl-1,5-pentylen; 1,6-Hexylen; 2,2(4),4-Trimethyl-1,6-hexamethylen; 1,8-Octylen; 1,10-Decylen; 1,12-Dodecylen; (1,5,5-Trimethylcyclohexan-1-yl)methan-1,3; 1,3-Cyclohexylen-bis(methylen); 1,4-Cyclohexylen-bis(methylen); 1,3-Phenylen-bis(methylen); 2- und/oder 4-Methyl-1,3-cyclohexylen; 4-Aza-N-methyl-1,7-heptylen; 4-Aza-N-ethyl-1,7-heptylen; 3-Aza-1,5-pentylen; 3,6-Diaza-1,8-octylen; 3,6,9-Triaza-1,11-undecylen; 4-Aza-1,7-heptylen; 3-Aza-1,6-hexylen; 4,7-Diaza-1,10-decylen; 7-Aza-1,13-tridecylen; Piperazin-1,4-diyl-bis(1,2-ethylen); Piperazin-1,4-diyl-bis(1,3-propylen); 3-Oxa-1,5-pentylen; 3,6-Dioxa-1,8-octylen; 4,7-Dioxa-1,10-decylen und α,ω-Polyoxypropylen mit einem Molekulargewicht im Bereich von 180 bis 500 g/mol, bestimmt durch GPC gegen polystyrol als Standard.

4. Zusammensetzung nach einem der vorhergehenden Ansprüche, **dadurch gekennzeichnet, dass** im Katalysator der Formel (I) R³ für -NR⁴R⁵ steht,
R⁴ für Wasserstoff steht,
R² und R⁵ unabhängig voneinander jeweils für einen Alkyl-, Cycloalkyl- oder Aralkyl-Rest mit 1 bis 18 C-Atomen, welcher gegebenenfalls Heteroatome enthält, stehen, und
R¹ für Wasserstoff steht.

5. Zusammensetzung nach Anspruch 4, **dadurch gekennzeichnet, dass** im Katalysator der Formel (I) R² und R⁵ unabhängig voneinander jeweils für Ethyl, Isopropyl, tert.Butyl, 3-(Dimethylamino)propyl oder Cyclohexyl stehen.

6. Zusammensetzung nach einem der vorhergehenden Ansprüche, **dadurch gekennzeichnet, dass** das Silangruppen-haltige Polymer ein Polyorganosiloxan mit endständigen Silangruppen ist.

7. Zusammensetzung nach einem der Ansprüche 1 bis 5, **dadurch gekennzeichnet, dass** das Silangruppen-haltige Polymer ein Silangruppen-haltiges Polyolefin oder ein Silangruppen-haltiger Polyester oder ein Silangruppen-haltiges Poly(meth)acrylat oder ein Silangruppen-haltiger Polyether oder eine Mischform dieser Polymere ist.

8. Zusammensetzung nach einem der vorhergehenden Ansprüche, **dadurch gekennzeichnet, dass** zusätzlich mindestens ein Organotitanat enthalten ist.

9. Verfahren zur Herstellung einer Zusammensetzung nach einem der Ansprüche 1 bis 8, umfassend einen Schritt zur Herstellung eines Katalysators der Formel (I), bei welchem R³ für Wasserstoff oder für einen Alkyl-, Cycloalkyl- oder Aralkyl-Rest mit 1 bis 12 C-Atomen steht, **dadurch gekennzeichnet, dass** mindestens ein Polyamin mit mindestens einem Orthoester oder mindestens einem 1,3-Ketoester oder mindestens einem Nitril umgesetzt wird.

10. Verfahren zur Herstellung einer Zusammensetzung nach einem der Ansprüche 1 bis 8, umfassend einen Schritt zur Herstellung eines Katalysators der Formel (I), bei welchem R³ für -NR⁴R⁵ steht, **dadurch gekennzeichnet, dass** mindestens ein Polyamin der Formel A-(NHR¹)ₙ mit mindestens einem Carbodiimid der Formel R⁵-N=C=N-R² umgesetzt wird.

11. Verfahren nach einem der Ansprüche 9 oder 10, **dadurch gekennzeichnet, dass** das Polyamin ausgewählt ist aus der Gruppe bestehend aus 1,2-Ethandiamin, 1,3-Propandiamin, 1,2-Propandiamin, 1,4-Butandiamin, 1,3-Butandiamin, 1,2-Butandiamin, 2,3-Butandiamin, 1,3-Pentandiamin, 1,5-Diamino-2-methylpentan, 1,6-Hexandiamin, 2,2,4- und 2,4,4-Trimethylhexamethylendiamin, 1,8-Octandiamin, 1,10-Decandiamin, 1,12-Dodecandiamin, 1-Amino-3-aminomethyl-3,5,5-trimethylcyclohexan, 1,3-Bis(aminomethyl)cyclohexan, 1,4-Bis(aminomethyl)cyclohexan,1,3-Bis(aminomethyl)benzol, 2- und 4-Methyl-1,3-diaminocyclohexan und Mischungen davon, N,N-Bis(3-aminopropyl)methylamin, N,N-Bis(3-aminopropyl)ethylamin, Diethylentriamin, Triethylentetramin, Tetraethylenpentamin, Dipropylentriamin, N-(2-Aminoethyl)-1,3-propandiamin, N,N'-Bis(3-amino-propyl)ethylendiamin, Bis-hexamethylentriamin, N,N'-Bis(aminoethyl)piperazin, N,N'-Bis(aminopropyl)piperazin, Bis(2-aminoethyl)ether, 3,6-Dioxaoctan-1,8-diamin, 4,7-Dioxadecan-1,10-diamin und Polyoxypropylendiamin mit einem Molekulargewicht im Bereich von 210 bis 500 g/mol, bestimmt durch GPC gegen polystyrol als Standard.

12. Verfahren nach einem der Ansprüche 9 bis 11, **dadurch gekennzeichnet, dass** die Umsetzung in Gegenwart des Silangruppen-haltigen Polymers erfolgt.

13. Verwendung einer Zusammensetzung nach einem der Ansprüche 1 bis 8 als Klebstoff, Dichtstoff oder Beschichtung.

14. Ausgehärtete Zusammensetzung, erhalten aus einer Zusammensetzung nach einem der Ansprüche 1 bis 8 nach deren Härtung.

15. Verwendung eines Katalysators der Formel (I), wie er in Anspruch 1 beschrieben ist, als Vernetzungskatalysator für härtbare Zusammensetzungen.

16. Katalysator der Formel (Ia'), 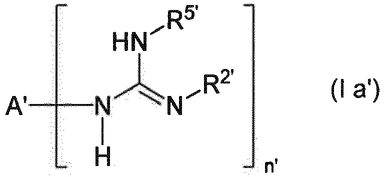 wobei
n' für 2 oder 3, insbesondere für 2, steht;
A' für einen n'-wertigen aliphatischen oder cycloaliphatischen oder arylaliphatischen Kohlenwasserstoff-Rest mit 2 bis 18 C-Atomen, welcher gegebenenfalls Heteroatome enthält und gegebenenfalls Aminogruppen aufweist, steht; und
R^{2'} und R^{5'} unabhängig voneinander jeweils für einen Alkyl-, Cycloalkyl- oder Aralkyl-Rest mit 1 bis 18 C-Atomen, welcher gegebenenfalls Heteroatome enthält, stehen; wobei der Katalysator der Formel (Ia') frei von Silangruppen ist.

## Claims

1. Composition comprising
- at least one polymer containing silane groups; and
- at least one catalyst of the formula (I)
A⁅Z]ₙ (I)
wherein
n is an integer from 2 to 20
A is an n-valent aliphatic or cycloaliphatic or arylaliphatic hydrocarbon radical having 2 to 100 carbon atoms, optionally comprising unsaturated moieties, optionally comprising heteroatoms and optionally comprising amino groups, and
Z is a radical of the formula 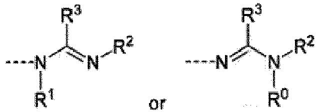 wherein
R¹ and R⁰ are each independently hydrogen or an alkyl or cycloalkyl or aralkyl radical having 1 to 8 carbon atoms,
R² is hydrogen or an alkyl, cycloalkyl or aralkyl radical having 1 to 18 carbon atoms, optionally comprising heteroatoms and optionally comprising terminal primary or secondary amino groups,
R³ is -NR⁴R⁵ or hydrogen or an alkyl, cycloalkyl or aralkyl radical having 1 to 12 carbon atoms,
R⁴ and R⁵ are each independently hydrogen or an alkyl, cycloalkyl or aralkyl radical having 1 to 18 carbon atoms, optionally comprising heteroatoms, wherein
R¹ and R² together may also be an alkylene radical having 2 to 6 carbon atoms,
R² and R⁰ together may also be an alkylene radical having 3 to 6 carbon atoms, optionally comprising heteroatoms,
R² and R³ together may also be an alkylene radical having 3 to 6 carbon atoms,
R⁴ and R⁵ together may also be an alkylene radical having 4 to 7 carbon atoms, optionally comprising heteroatoms,
and
R² and R⁵ together may also be an alkylene radical having 2 to 12 carbon atoms;
wherein the catalyst of the formula (I) does not comprise a nitrogen atom which is bonded directly to an aromatic ring or is part of a heteroaromatic ring system and wherein the catalyst of the formula (I) is free of silane groups.

2. Composition according to Claim 1, **characterized in that** A is an n-valent aliphatic or cycloaliphatic or arylaliphatic hydrocarbon radical having 2 to 50 carbon atoms, optionally comprising ether oxygen or amine nitrogen.

3. Composition according to either of the preceding claims, **characterized in that** A is selected from the group consisting of 1,2-ethylene; 1,3-propylene; 1,2-propylene, 1,4-butylene; 1,3-butylene; 1,2-butylene; 2,3-butylene; 1,3-pentylene; 2-methyl-1,5-pentylene; 1,6-hexylene; 2,2(4),4-trimethyl-1,6-hexamethylene; 1,8-octylene; 1,10-decylene; 1,12-dodecylene; (1,5,5-trimethylcyclohexan-1-yl)methane-1,3; 1,3-cyclohexylenebis(methylene); 1-4-cyclohexylenebis(methylene); 1,3-phenylenebis(methylene); 2- and/or 4-methyl-1,3-cyclohexylene; 4-aza-N-methyl-1,7-heptylene; 4-aza-N-ethyl-1,7-heptylene; 3-aza-1,5-pentylene; 3,6-diaza-1,8-octylene; 3,6,9-triaza-1,11-undecylene; 4-aza-1,7-heptylene; 3-aza-1,6-hexylene; 4,7-diaza-1,10-decylene; 7-aza-1,13-tridecylene; piperazine-1,4-diylbis(1,2-ethylene); piperazine-1,4-diylbis(1,3-propylene); 3-oxa-1,5-pentylene; 3,6-dioxa-1,8-octylene; 4,7-dioxa-1,10-decylene and α,ω-polyoxypropylene having a molecular weight in the range of 180 to 500 g/mol, determined by GPC with polystyrene as standard.

4. Composition according to any of the preceding claims, **characterized in that** in the catalyst of the formula (I), R³ is -NR⁴R⁵,
R⁴ is hydrogen,
R² and R⁵ are each independently an alkyl, cycloalkyl or aralkyl radical having 1 to 18 carbon atoms, optionally comprising heteroatoms, and
R¹ is hydrogen.

5. Composition according to Claim 4, **characterized in that** in the catalyst of the formula (I), R² and R⁵ are each independently ethyl, isopropyl, tert-butyl, 3-(dimethylamino)propyl or cyclohexyl.

6. Composition according to any of the preceding claims, **characterized in that** the polymer containing silane groups is a polyorganosiloxane with terminal silane groups.

7. Composition according to any of Claims 1 to 5, **characterized in that** the polymer containing silane groups is a polyolefin containing silane groups or a polyester containing silane groups or a poly(meth)acrylate containing silane groups or a polyether containing silane groups or a mixed form of these polymers.

8. Composition according to any of the preceding claims, **characterized in that** at least one organotitanate is additionally present.

9. Method for preparing a composition according to any of Claims 1 to 8, comprising a step for preparing a catalyst of the formula (I), in which R³ is hydrogen or an alkyl, cycloalkyl or aralkyl radical having 1 to 12 carbon atoms, **characterized in that** at least one polyamine is reacted with at least one orthoester or at least one 1,3-ketoester or at least one nitrile.

10. Method for preparing a composition according to any of Claims 1 to 8, comprising a step for preparing a catalyst of the formula (I) in which R³ is -NR⁴R⁵, **characterized in that** at least one polyamine of the formula A-(NHR¹)ₙ is reacted with at least one carbodiimide of the formula R⁵-N=C=N-R².

11. Method according to either of Claims 9 and 10, **characterized in that** the polyamine is selected from the group consisting of 1,2-ethanediamine, 1,3-propanediamine, 1,2-propanediamine, 1,4-butanediamine, 1,3-butanediamine, 1,2-butanediamine, 2,3-butanediamine, 1,3-pentanediamine, 1,5-diamino-2-methylpentane, 1,6-hexanediamine, 2,2,4- and 2,4,4-trimethylhexamethylenediamine, 1,8-octanediamine, 1,10-decanediamine, 1,12-dodecanediamine, 1-amino-3-aminomethyl-3,5,5-trimethylcyclohexane, 1,3-bis(aminomethyl)cyclohexane, 1,4-bis(aminomethyl)cyclohexane, 1,3-bis(aminomethyl)benzene, 2- and 4-methyl-1,3-diaminocyclohexane and mixtures thereof, N,N-bis(3-aminopropyl)methylamine, N,N-bis(3-aminopropyl)ethylamine, diethylenetriamine, triethylenetetramine, tetraethylenepentamine, dipropylenetriamine, N-(2-aminoethyl)-1,3-propanediamine, N,N'-bis(3-aminopropyl)ethylenediamine, bis(hexamethylene)triamine, N,N'-bis(aminoethyl)piperazine, N,N'-bis(aminopropyl)piperazine, bis(2-aminoethyl) ether, 3,6-dioxaoctane-1,8-diamine, 4,7-dioxadecane-1,10-diamine and polyoxypropylenediamine having a molecular weight in the range of 210 to 500 g/mol, determined by GPC with polystyrene as standard.

12. Method according to any of Claims 9 to 11, **characterized in that** the reaction is carried out in the presence of the polymer containing silane groups.

13. Use of a composition according to any of Claims 1 to 8 as adhesive, sealant or coating.

14. Cured composition, obtained from a composition according to any of Claims 1 to 8 after curing thereof.

15. Use of a catalyst of the formula (I), as described in Claim 1, as crosslinking catalyst for curable compositions.

16. Catalyst of the formula (I a'), 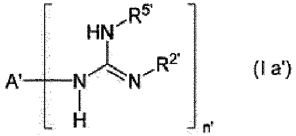 wherein
n' is 2 or 3, especially 2;
A' is an n-valent aliphatic or cycloaliphatic or arylaliphatic hydrocarbon radical having 2 to 18 carbon atoms, optionally comprising heteroatoms and optionally comprising amino groups; and
R^{2'} and R^{5'} are each independently an alkyl, cycloalkyl or aralkyl radical having 1 to 18 carbon atoms, optionally comprising heteroatoms; wherein the catalyst of the formula (I a') is free of silane groups.

## Revendications

1. Composition comprenant
- au moins un polymère contenant des groupes silane ; et
- au moins un catalyseur de formule (I)
A⁅Z]ₙ (I)
n représentant un nombre entier de 2 à 20
A représentant un radical hydrocarboné n-valent aliphatique ou cycloaliphatique ou arylaliphatique comportant 2 à 100 atomes de C, qui contient éventuellement des parties insaturées, contient éventuellement des hétéroatomes et présente éventuellement des groupes amino, et
Z représentant un radical de formule 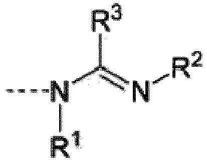 ou 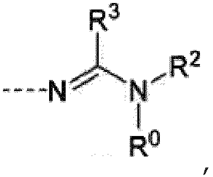
R¹ et R⁰, indépendamment l'un de l'autre, à chaque fois représentant hydrogène ou un radical alkyle ou cycloalkyle ou aralkyle comportant 1 à 8 atome(s) de C,
R² représentant hydrogène ou un radical alkyle ou cycloalkyle ou aralkyle comportant 1 à 18 atome(s) de C, qui contient éventuellement des hétéroatomes et qui présente éventuellement des groupes terminaux amino primaire ou secondaire, R³ représentant -NR⁴R⁵ ou hydrogène ou un radical alkyle, cycloalkyle ou aralkyle comportant 1 à 12 atome(s) de C,
R⁴ et R⁵ représentant indépendamment l'un de l'autre à chaque fois hydrogène ou un radical alkyle, cycloalkyle ou aralkyle comportant 1 à 18 atome(s) de C, qui contient éventuellement des hétéroatomes,
R¹ et R² ensemble pouvant également représenter un radical alkylène comportant 2 à 6 atomes de C,
R² et R⁰ ensemble pouvant également représenter un radical alkylène comportant 3 à 6 atomes de C, qui contient éventuellement des hétéroatomes,
R² et R³ ensemble pouvant également représenter un radical alkylène comportant 3 à 6 atomes de C,
R⁴ et R⁵ ensemble pouvant également représenter un radical alkylène comportant 4 à 7 atomes de C, qui contient éventuellement des hétéroatomes,
R² et R⁵ ensemble pouvant également représenter un radical alkylène comportant 2 à 12 atomes de C ;
le catalyseur de formule (I) ne contenant aucun atome d'azote, qui est directement lié à un cycle aromatique ou qui fait partie d'un système cyclique hétéroaromatique et le catalyseur de formule (I) étant exempt de groupes silane.

2. Composition selon la revendication 1, **caractérisée en ce que** A représente un radical hydrocarboné n-valent aliphatique ou cycloaliphatique ou arylaliphatique comportant 2 à 50 atomes de C, qui contient éventuellement de l'oxygène d'éther ou de l'azote d'amine.

3. Composition selon l'une quelconque des revendications précédentes, **caractérisée en ce que** A est choisi dans le groupe constitué par 1,2-éthylène ; 1,3-propylène ; 1,2-propylène ; 1,4-butylène ; 1,3-butylène ; 1,2-butylène ; 2,3-butylène ; 1,3-pentylène ; 2-méthyl-1,5-pentylène ; 1,6-hexylène ; 2,2(4),4-triméthyl-1,6-hexaméthylène ; 1,8-octylène ; 1,10-décylène ; 1,12-dodécylène ; (1,5,5-triméthylcyclohexane-1-yl)méthane-1,3 ; 1,3-cyclohexylène-bis(méthylène) ; 1,4-cyclohexylène-bis(méthylène); 1,3-phénylène-bis(méthylène) ; 2-et/ou 4-méthyl-1,3-cyclohexylène ; 4-aza-N-méthyl-1,7-heptylène ; 4-aza-N-éthyl-1,7-heptylène ; 3-aza-1,5-pentylène ; 3,6-diaza-1,8-octylène ; 3,6,9-triaza-1,11-undécylène ; 4-aza-1,7-heptylène ; 3-aza-1,6-hexylène ; 4,7-diaza-1,10-décylène ; 7-aza-1,13-tridécylène ; pipérazine-1,4-diyl-bis(1,2-éthylène) ; pipérazine-1,4-diyl-bis(1,3-propylène) ; 3-oxa-1,5-pentylène ; 3,6-dioxa-1,8-octylène ; 4,7-dioxa-1,10-décylène et α,ω- polyoxypropylène comportant un poids moléculaire dans la plage de 180 à 500 g/mole, déterminé par CPG avec du polystyrène comme référence.

4. Composition selon l'une quelconque des revendications précédentes, **caractérisée en ce que** dans le catalyseur de formule (I), R³ représente - NR⁴R⁵,
R⁴ représente hydrogène,
R² et R⁵ indépendamment l'un de l'autre représente à chaque fois un radical alkyle, cycloalkyle ou aralkyle comportant 1 à 18 atome(s) de C, qui contient éventuellement des hétéroatomes, et
R¹ représente hydrogène.

5. Composition selon la revendication 4, **caractérisée en ce que** dans le catalyseur de formule (I) R² et R⁵ indépendamment l'un de l'autre représente à chaque fois éthyle, isopropyle, tert.butyle, 3-(diméthylamino)propyle ou cyclohexyle.

6. Composition selon l'une quelconque des revendications précédentes, **caractérisée en ce que** le polymère contenant des groupes silane est un polyorganosiloxane comportant des groupes silane terminaux.

7. Composition selon l'une quelconque des revendications 1 à 5, **caractérisée en ce que** le polymère contenant des groupes silane est une polyoléfine contenant des groupes silane ou un polyester contenant des groupes silane ou un poly(méth)acrylate contenant des groupes silane ou un polyéther contenant des groupes silane ou un mélange de ces polymères.

8. Composition selon l'une quelconque des revendications précédentes, **caractérisée en ce que** de plus au moins un organotitanate est contenu.

9. Procédé pour la préparation d'une composition selon l'une quelconque des revendications 1 à 8, comprenant une étape pour la préparation d'un catalyseur de formule (I), dans laquelle R³ représente hydrogène ou un radical alkyle, cycloalkyle ou aralkyle comportant 1 à 12 atome(s) de C, **caractérisé en ce qu'**au moins une polyamine comportant au moins un orthoester ou au moins un 1,3-cétoester ou au moins un nitrile est transformée.

10. Procédé pour la préparation d'une composition selon l'une quelconque des revendications 1 à 8, comprenant une étape pour la préparation d'un catalyseur de formule (I), dans laquelle R³ représente -NR⁴R⁵, **caractérisé en ce qu'**au moins une polyamine de formule A-(NHR¹)ₙ est transformée avec au moins un carbodiimide de formule R⁵-N=C=N-R².

11. Procédé selon l'une quelconque des revendications 9 et 10, **caractérisé en ce que** la polyamine est choisie dans le groupe constitué par la 1,2-éthanediamine, la 1,3-propanediamine, la 1,2-propanediamine, la 1,4-butanediamine, la 1,3-butanediamine, la 1,2-butanediamine, la 2,3-butanediamine, la 1,3-pentanediamine, le 1,5-diamino-2-méthylpentane, la 1,6-hexanediamine, la 2,2,4- et la 2,4,4-triméthylhexaméthylènediamine, la 1,8-octanediamine, la 1,10-décanediamine, la 1,12-dodécanediamine, le 1-amino-3-aminométhyl-3,5,5-triméthylcyclohexane, le 1,3-bis(aminométhyl)cyclohexane, le 1,4-bis(aminométhyl)cyclohexane, le 1,3-bis(aminométhyl)benzène, le 2- et 4-méthyl-1,3-diaminocyclohexane et des mélanges correspondants, la N,N-bis(3-aminopropyl)méthylamine, la N,N-bis(3-aminopropyl)-éthylamine, la diéthylènetriamine, la triéthylènetétramine, la tétraéthylènepentamine, la dipropylènetriamine, la N-(2-aminoéthyl)-1,3-propanediamine, la N,N'-bis(3-aminopropyl)éthylènediamine, la bis-hexaméthylènetriamine, la N,N'-bis(aminoéthyl)pipérazine, la N,N'-bis(aminopropyl)pipérazine, le bis(2-aminoéthyl)éther, la 3,6-dioxaoctan-1,8-diamine, la 4,7-dioxadécan-1,10-diamine et la polyoxypropylènediamine comportant un poids moléculaire dans la plage de 210 à 500 g/mole, déterminé par CPG avec du polystyrène comme référence.

12. Procédé selon l'une quelconque des revendications 9 à 11, **caractérisé en ce que** la transformation est réalisée en présence du polymère contenant des groupes silane.

13. Utilisation d'une composition selon l'une quelconque des revendications 1 à 8 en tant qu'adhésif, agent d'étanchéité ou revêtement.

14. Composition durcie, obtenue à partir d'une composition selon l'une quelconque des revendications 1 à 8 après son durcissement.

15. Utilisation d'un catalyseur de formule (I), tel que décrit dans la revendication 1, en tant que catalyseur de réticulation pour des compositions durcissables.

16. Catalyseur de formule (I a'), 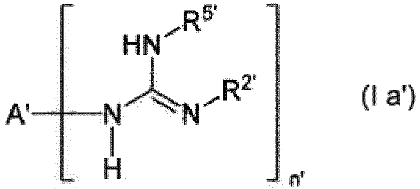
n' représentant 2 ou 3, en particulier 2 ;
A' représentant un radical hydrocarboné n'-valent aliphatique ou cycloaliphatique ou arylaliphatique comportant 2 à 18 atomes de C, qui contient éventuellement des hétéroatomes et présente éventuellement des groupes amino ; et
R^{2'} et R^{5'} représentant indépendamment l'un de l'autre à chaque fois un radical alkyle, cycloalkyle ou aralkyle comportant 1 à 18 atome(s) de C, qui contient éventuellement des hétéroatomes ; le catalyseur de formule (I a') étant exempt de groupes silane.
